(19) Europäisches Patentamt
European Patent Office
Office européen des brevets

(11) EP 3 020 360 B1

(12) **EUROPÄISCHE PATENTSCHRIFT**

(45) Veröffentlichungstag und Bekanntmachung des Hinweises auf die Patenterteilung:
**08.05.2019 Patentblatt 2019/19**

(51) Int Cl.:
***A61K 6/093*** *(2006.01)* ***A61K 6/083*** *(2006.01)*

(21) Anmeldenummer: **15193617.6**

(22) Anmeldetag: **09.11.2015**

(54) **RADIKALISCH HÄRTBARE DENTALE ZUSAMMENSETZUNGEN**

RADICALLY CURABLE DENTAL COMPOSITIONS

COMPOSITIONS DENTAIRES À DURCISSEMENT RADICALAIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **11.11.2014 DE 102014116389**

(43) Veröffentlichungstag der Anmeldung:
**18.05.2016 Patentblatt 2016/20**

(60) Teilanmeldung:
**19164843.5**

(73) Patentinhaber: **VOCO GmbH**
**27472 Cuxhaven (DE)**

(72) Erfinder:
- **Fontein, Nils, Dr.**
  **27472 Cuxhaven (DE)**
- **Lübbe, Gerrit, Dr.**
  **27476 Cuxhaven (DE)**
- **Plaumann, Manfred Thomas**
  **27472 Cuxhaven (DE)**

(56) Entgegenhaltungen:
**US-A1- 2003 069 326** **US-A1- 2014 138 864**
**US-B1- 6 566 413**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 020 360 B1

## Beschreibung

Die vorliegende Erfindung betrifft neue radikalisch härtbare, dentale Zusammensetzungen, umfassend mit radikalisch polymerisierbaren Gruppen substituierte kettenförmige und/oder zyklische und/oder in Käfigform vorliegende Polysiloxane mit mindestens 3 Siliziumatomen und/oder deren Mischformen, mit radikalisch polymerisierbaren Gruppen substituierte Disiloxane, optional ein, zwei, drei oder mehrere radikalisch härtbare dentalen Monomere ohne Siliziumatom, Füllstoffe, Initiatoren und/oder Katalysatoren für die radikalische Polymerisation sowie weitere übliche Additive, die aus den erfindungsgemäßen dentalen Zusammensetzungen gehärteten dentalen Produkte sowie deren jeweilige Verwendung als Dentalmaterial, insbesondere als fließfähige Füllungskomposite (sogenannte "Flow-Materialien"), Stumpfaufbaumaterialien und Befestigungszemente. Die Erfindung betrifft ferner ein Verfahren zur Herstellung der dentalen Zusammensetzungen und ein Verfahren zur Herstellung eines jeweiligen dentalen Erzeugnisses. Weiterhin werden erfindungsgemäße Kits, enthaltend die neuen radikalisch härtbaren, dentalen Zusammensetzungen, beansprucht.

Weitere Aspekte der vorliegenden Erfindung und deren bevorzugte Ausgestaltungen ergeben sich aus der folgenden Beschreibung, den Ausführungsbeispielen und den Ansprüchen.

Polysiloxanverbindungen mit mindestens 3 Siliziumatomen im Sinne des vorliegenden Textes besitzen mindestens eine oder mehrere Ketten, die alternierend angeordnete und miteinander verbundene Siliziumatome und Sauerstoffatome besitzen, wobei sich die Ketten auch zu Ringen unterschiedlicher Größe, bzw. zu noch umfangreicheren Strukturen wie Käfigen zusammenschließen und wobei an den Siliziumatomen organische Gruppen (organische Seitenketten) gebunden sind. Diese organischen Gruppen können chemisch sehr verschieden zusammengesetzt sein und dadurch zu einer Vielzahl von Polysiloxanverbindungen mit unterschiedlichen Eigenschaften führen. Häufig besitzen diese organischen Gruppen eine oder mehrere organisch polymerisierbare Gruppen (d.h. reaktive Gruppen), die sich mit z.B. einer oder mehreren organisch polymerisierbaren Gruppen einer anderen Polysiloxanverbindung umsetzen können und dadurch vernetzte polymerisierte Polysiloxanverbindungen bilden. Ketten-, Ring- und Käfigstrukturen können auch in Form gemischter Strukturen auftreten. Sie sind ebenfalls Bestandteil der erfindungsgemäßen dentalen Zusammensetzungen.

Polysiloxanverbindungen sind seit langem bekannt und z.B. erhältlich durch Hydrolyse und Kondensation von Silanen mit hydrolysierbaren Gruppen (siehe z.B. DE 27 58 414 A1) oder durch Hydrosilylierung von Allyl- oder Vinylverbindungen mit SiH-haltigen Verbindungen. Polysiloxanverbindungen können zu einer Vielzahl von Produkten weiterverarbeitet werden, wie z.B. Überzüge, Beschichtungen, Membranen oder Bulkmaterialien. Diese Weiterverarbeitung beruht dabei häufig auf einer Vernetzungsreaktion organisch polymerisierbarer Gruppen in den Polysiloxanverbindungen (z.B. (Meth)acrylatgruppen) und der daraus resultierenden Bildung vernetzter Polysiloxanverbindungen.

Unter "(Meth)acryl" ist im Rahmen des vorliegenden Textes sowohl "Acryl" als auch "Methacryl" zu verstehen.

Eine spezifische Gruppe von Polysiloxanverbindungen enthält in den organischen Gruppen (Seitenketten) neben einer organisch polymerisierbaren Gruppe zusätzliche freie polare funktionelle Gruppen wie z.B. Hydroxy- oder Carboxygruppen.

So betrifft DE 44 16 857 C1 hydrolysierbare und polymerisierbare Silane, Verfahren zu deren Herstellung sowie deren Verwendung zur Herstellung von Kieselsäure(hetero)polykondensaten und (Hetero)Polymerisaten. Hydrolysierbare, organisch modifizierte Silane finden eine breite Anwendung bei der Herstellung kratzfester Beschichtungen für die unterschiedlichsten Substrate, für die Herstellung von Füllstoffen, von Klebe- und Dichtungsmassen oder von Formkörpern.

Die DE 44 16 857 C1 offenbart den Einsatz von Kieselsäure(hetero)polykondensaten (Polysiloxanverbindungen) in härtbaren Dentalmaterialien. Die hier beschriebenen Polysiloxanverbindungen umfassen freie polare funktionelle Gruppen (z.B. Carboxy- oder Hydroxygruppen), die in der Lage sind, geeignete Metallionen / Übergangsmetallionen zu komplexieren (z.B. Ionen des Titans, Zirkoniums oder Zinns). In härtbaren dentalen Zusammensetzungen kann sich dies positiv auf die Röntgenopazität, die Kontakttoxizität und auf den Brechungsindex eines entsprechenden härtbaren bzw. gehärteten Dentalmaterials auswirken.

DE 198 60 364 C2 betrifft polymerisierbare Dentalmassen auf der Basis von zur Aushärtung befähigten Siloxanverbindungen, deren Verwendung und Herstellung. In dieser Druckschrift wird die Herstellung von cyclischen Polysiloxanen und deren Verwendung als Basis für polymerisisierbare Dentalmassen beschrieben. Sie sollen trotz hoher Dichte an zur Polymerisation befähigten Gruppen eine niedrige Viskosität aufweisen, die eine hohe Füllstoffaufnahme ermöglicht, welche zu Massen mit geringem Polymerisationsschrumpf führen. Auch hier befinden sich in den organischen Seitenketten der beschriebenen Polysiloxane neben den polymerisierbaren Einheiten, freie polare Funktionen.

Die freien polaren funktionellen Gruppen, z.B. in den vorstehend genannten Polysiloxanverbindungen führen jedoch regelmäßig auch zu unerwünschten Eigenschaften. So hat sich gezeigt, dass die durch die (freien) polaren funktionellen Gruppen hervorgerufene Hydrophilie der Polysiloxanverbindungen zu einer erhöhten Wasseraufnahme in Gegenwart von Feuchtigkeit führt, wodurch die Nassfestigkeit des härtbaren Dentalmaterials in nachteiliger Weise verringert wird. Vermutlich durch Aufbau interner Wasserstoffbrückenbindungen kommt es zu einer Erhöhung der Viskosität.

Dies wirkt sich dann negativ auf die Handhabbarkeit bei der Herstellung der härtbaren Dentalzusammensetzungen aus.

Es besteht ein beträchtliches Bedürfnis seitens Dentalmedizinern und der Dentalindustrie, Polysiloxanverbindungen weiter an die Anforderungen an ein modernes (härtbares bzw. gehärtetes) Dentalmaterial anzupassen und die vorstehend genannten Nachteile zu minimieren. Derart angepasste Polysiloxanverbindungen sollen dabei dentalmedizinisch verbesserte physikalische Eigenschaften besitzen (bzw. zu dentalmedizinisch verbesserten physikalischen Eigenschaften entsprechender härtbarer/gehärteter Dentalmaterialien führen), z.B. einen geringeren Polymerisationsschrumpf beim Polymerisieren/Vernetzen der Polysiloxanverbindungen (d.h. beim Aushärten), eine erhöhte Festigkeit und/oder eine begrenzte Wasseraufnahme bei gleichzeitig komfortabler Konsistenz des härtbaren Dentalmaterials.

Erste Erfolge bei der Verbesserung der Polysiloxane konnten durch Addition bzw. Substitution unterschiedlicher Substrate an die freien polaren Funktionalitäten der oben beschrieben speziellen Polysiloxane erreicht werden.

EP 1 874 847 B1 betrifft ein Verfahren zur Herstellung von Silanen mit zwei, drei oder sogar mehr Struktureinheiten, die über eine urethan-, säureamid- und/oder carbonsäureestergruppenhaltige Brücke miteinander verknüpft sind und von denen jede mindestens einen organisch polymerisierbaren Rest und mindestens einen Silylrest enthält. Diese Silane sollen sich insbesondere zur Modifizierung der Eigenschaften von Kieselsäure(hetero)polykondensaten und silylgruppenhaltigen, organischen Polymeren eignen. Das offenbarte Verfahren soll sich auch zur Verbrückung bereits vorkondensierter Kieselsäure(hetero)polykondensate eignen.

Die in EP 1 874 847 B1 offenbarten Kieselsäure(hetero)polykondensate (Polysiloxanverbindungen) verfügen über eine freie Hydroxygruppe (d.h. eine freie polare funktionelle Gruppe). Diese freien Hydroxygruppen können mit einem Dicarbonsäurederivat oder Diisocyanat so reagieren, dass Hydroxylgruppen mit einem Dicarbonsäurederivat bzw. Diisocyanat eine Verknüpfung (Verbrückung) bilden. Solche verknüpften Polysiloxanverbindungen besitzen ein deutlich höheres Molekulargewicht, ohne dabei die Doppelbindungsdichte (bedingt durch die organisch polymerisierbaren (Meth)acrylatgruppen) wesentlich zu verringern. Unter Doppelbindungsdichte wird hier der Quotient aus der Anzahl der polymerisierbaren Doppelbindungen in einer Verbindung und dem Molekulargewicht dieser Verbindung verstanden. Das höhere Molekulargewicht wirkt sich positiv auf die Biokompatibilität und den Polymerisationsschrumpf beim Vernetzen der verknüpften Polysiloxanverbindungen aus. Gleichzeitig konnte die Hydrophobizität der Polysiloxanverbindungen gesteigert werden. Es konnte jedoch gezeigt werden, dass sich das höhere Molekulargewicht nachteilig auf die Viskosität der verknüpften Polysiloxanverbindungen (und damit auf die Verarbeitbarkeit bei der Herstellung des härtbaren Dentalmaterials) auswirkt. Die Viskosität steigt mit dem Grad der Verknüpfung, d.h. mit dem Molekulargewicht deutlich an, so dass eine tolerierbare Verarbeitbarkeit bei der Herstellung eines entsprechenden härtbaren Dentalmaterials, das solche verknüpften Polysiloxanverbindungen umfasst, schon bei recht geringem Verknüpfungsgrad nicht mehr zufriedenstellend gegeben ist.

EP 1 685 182 B1 betrifft Silane und daraus gebildete Kieselsäurepolykondensate und - teilkondensate, in denen ein an einem Silicium gebundener, organischer Rest vorhanden ist, der verzweigt ist und an jedem der beiden Zweige mindestens eine eigenständig organisch polymerisierbare Gruppe trägt oder an einem der beiden Zweige eine solche Gruppe, am anderen einen Rest mit einem weiteren Siliziumatom aufweist.

Auch die in EP 1 685 182 B1 offenbarten Polysiloxanverbindungen umfassen freie polare funktionelle Gruppen in Form von Hydroxygruppen. Durch Reaktion von Carbonsäure- oder Isocyanatderivate, die ihrerseits ebenfalls polymerisierbare Doppelbindungen umfassen (z.B. (Meth)acrylatgruppen), können so organisch polymerisierbare Gruppen an freie polare funktionelle Gruppen geknüpft werden. Diese Reaktionsprodukte besitzen regelmäßig eine erhöhte Festigkeit bei gleichzeitig gesteigerter Hydrophobizität und verbesserter Biokompatibilität durch das erhöhte Molekulargewicht.

Es konnte jedoch auch in diesen Fällen gezeigt werden, dass das Einführen zusätzlicher polymerisierbarer Doppelbindungen zu einem erhöhten Polymerisationsschrumpf beim Vernetzen der Polysiloxanverbindungen führt, da sich die Doppelbindungsdichte deutlich erhöht, die Erhöhung des Molekulargewichtes aber nur vergleichsweise gering ist.

WO 2013/041723 A1 offenbart hydrolysierbare und polymerisierbare Silane (einschließlich Kieselsäurepolykondensate, d.h. Siloxane) mit einstellbarer räumlicher Verteilung der funktionellen Gruppen sowie deren Verwendung. Die in WO 2013/041723 A1 offenbarte Lehre betrifft ein Verfahren zur Kettenverlängerung von über Kohlenstoff an Silizium gebundenen Resten in Silanen oder Siloxanen.

WO 2013/053693 A1 offenbart Kieselsäurepolykondensate (Siloxane) mit cyclischen olefinhaltigen Strukturen und Verfahren zu deren Herstellung sowie deren Verwendung. WO 2013/053693 A1 offenbart, dass sich Polymerwerkstoffe mit in weiten Grenzen einstellbaren E-Moduln bei hoher elastischer Dehnung (d.h. ohne Sprödverhalten) und damit einer hohen Bruchzähigkeit aus Kieselsäure(hetero)polykondensaten mit cyclischen olefinhaltigen Strukturen herstellen lassen.

In der noch nicht offengelegten DE 10 2014 210 432 sind Polysiloxanverbindungen beschrieben, die die vorstehend genannten Nachteile aus dem Stand der Technik in einer härtbaren bzw. gehärteten dentalen Zusammensetzung nicht oder zumindest nur noch abgeschwächt aufweisen. Der konzeptuelle Ansatz dieser Systeme sieht vor, die freie funktionelle Gruppe im Silan so umzusetzen, dass keine zusätzlich polymerisierbaren Doppelbindungen ins System eingeführt werden. Stattdessen werden Kohlenwasserstoffreste hohen Molekulargewichts mit wenigstens 11 C-Atomen

in das System eingebaut. Überraschenderweise zeigten sich bei diesen härtbaren dentalen Zusammensetzungen

- eine gute Viskosität der Polysiloxanverbindungen (die Viskosität sollte 50 Pa*s oder weniger bei einer Temperatur von 25°C betragen) und eine damit einhergehende hervorragende Verarbeitbarkeit bei der Herstellung eines die Polysiloxanverbindungen enthaltenden härtbaren Dentalmaterials,
- eine gute Hydrophobizität,
- eine gute Festigkeit, insbesondere eine gute Biegefestigkeit,
- einen sehr geringen Polymerisationsschrumpf beim Vernetzen der Polysiloxanverbindungen, d.h. beim Aushärten des härtbaren Dentalmaterials,
- eine gute Biokompatibilität,
- einen Brechungsindex, der nahezu identisch ist mit dem Brechungsindex üblicher dentaler Gläser.

Durch die in der DE 10 2014 210 432 ergriffenen Maßnahmen wurden somit gleich mehrere Probleme gelöst:

- durch Eliminierung polarer funktioneller Gruppen wurde die Ausbildung intermolekularer Wechselwirkungen unterbunden. Es gelang somit, die Viskosität des Systems trotz eines beachtlichen Molekuklargewichtszuwachses auf einem vergleichsweise niedrigen Niveau zu halten.
- durch Einbau von Kohlenwasserstoffresten relativ hohen Molekulargewichts wurde das Polysiloxanstrukturgerüst räumlich intramolekular aufgeweitet, so dass die Zugänglichkeit der radikalisch polymerisierbaren Gruppen während der Härtung erhöht wurde und damit die Umsetzungsrate optimiert werden konnte. Wie sonst könnte man den Umstand erklären, dass in diesen Systemen bei vergleichsweise reduzierter Doppelbindungsdichte die Festigkeit der Materialien, beispielsweise die Biegefestigkeit der gehärteten dentalen Zusammensetzungen auf einem sehr guten Niveau verbleiben und in vielen Fällen sogar erhöht sind im Vergleich zu den nicht weiter umgesetzten Polysiloxanen.
- durch die Molekulargewichtserhöhung bei Belassen der Funktionalität, also bei einer effektiven Absenkung der Doppelbindungsdichte konnte insbesondere die klinisch vielleicht wichtigste technische Kenngröße einer härtbaren dentalen Zusammensetzung, nämlich der Wert des Volumenschrumpfs während der Aushärtung, auf einen extrem niedrigen Wert eingestellt werden. In der klinischen Praxis ist der therapeutische Erfolg in erster Linie auch davon abhängig, ob das Dentalmaterial beispielsweise eine vom Zahnarzt präparierte Kavität randdicht abschließt. Durch Schrumpfung des Materials bei der Polymerisation können sich Randspalte bilden, durch die Bakterien in den Zahn eindringen und die Therapie dann scheitern lassen.
- Durch Einbau von Kohlenwasserstoffresten relativ hohen Molekulargewichts wurde das Polysiloxanstrukturgerüst auch vergleichsweise hydrophober gemacht, so dass die nicht erwünschte Wasseraufnahme nunmehr extrem geringe Werte annimmt.

Die in der DE 10 2014 210 432 beschriebenen radikalisch härtbaren Zusammensetzungen eigenen sich speziell zur Anwendung in einem therapeutischen Verfahren zur temporären oder permanenten Füllen einer dentalen Kavität. Weiterhin geeignet sind die Systeme zur Anwendung in einem therapeutischen Verfahren als Unterfüllungsmaterial, als Adhäsiv (Bonding), als fließfähiges Kompositmaterial (Flow Material), als Fissurenversiegler, als Kronen- und Brückenmaterial, als Inlay/Onlay und/oder als Stumpfaufbaumaterial.

In eigenen Untersuchungen wurde nun festgestellt, dass sich radikalisch härtbare dentale Zusammensetzungen, die Polysiloxane enthalten, und zwar sowohl die neuen Polysiloxane aus der DE 10 2014 210 432 als auch die zuvor beschriebenen Systeme aus dem älteren Stand der Technik, weiter verbessern lassen, wenn man ihnen radikalisch polymerisierbare Disiloxane zusetzt. Diese Verbesserungen machen sich insbesondere bemerkbar, wenn die radikalisch härtbaren dentalen Zusammensetzungen eine "mittlere" Füllstoffbeladung aufweisen, wie sie beispielsweise für Anwendungen als fließfähige Kompositmaterialien (Flow Materialien), als Stumpfaufbaumaterialien oder als Befestigungszemente vorgesehen sind oder sie weisen eine "niedrige" Füllstoffbeladung auf, wie sie beispielsweise für "Adhäsive" (Bondings), für Fissurenversiegler oder für dentale Lacke angezeigt sind.

Der Begriff "mittlere" Füllstoffbeladung entspricht einem prozentualen Anteil an Füllstoff von etwa 50 - 85 Gew.-% bezogen auf die Gesamtzusammensetzung, wobei der Füllstoff alle unterschiedlichen Fraktionen an nano- und mikroskaligen Füllstoff umfaßt.

Der Begriff "niedrige" Füllstoffbeladung entspricht einem prozentualen Anteil an Füllstoff von etwa 0 - 50 Gew.-% bezogen auf die Gesamtzusammensetzung, wobei der Füllstoff auch hier alle unterschiedlichen Fraktionen an nano- und mikroskaligen Füllstoff umfaßt.

Die oben genannten Füllstoffbereiche sind hier als Richtwerte angegeben und als solche zu verstehen, denn es gibt auch ganz spezielle radikalisch härtbare dentale Zusammensetzungen, die beispielsweise größere Mengen an nanoskaligen Füllstoffen verwenden, so dass sie mit einem Füllstoffgehalt von 70 Gew.-% bezogen auf die Gesamtzusammensetzung als Fissurenversiegler eingesetzt werden. Diese Versiegelungsmaterialien könnten auch als fließfähige

Kompositmaterialien (Flow-Materialien) Verwendung finden.

Die erzielten Verbesserungen richten sich in erster Linie auf die Konsistenz der erfindungsgemäßen radikalisch härtbaren dentalen Zusammensetzungen.

Die Konsistenz der radikalisch härtbaren, dentalen Zusammensetzungen ist von ganz besonderer Bedeutung bei der Anwendung des Dentalmaterials durch eine äußerst engen Applikationsspitze, beispielsweise mittels einer Zweikammermischkartusche, einer Compule oder einer dentalen Spritze, da die (hydrophobe) radikalisch härtbare dentale Masse gut an der (hydrophilen) Zahnsubstanz anfließen muß, um eine erfolgreiche Zahnbehandlung gewährleisten zu können. Die oben beschriebenen wichtigen Eigenschaften des Polymerisats, geringer Schrumpf, geringe Wasseraufnahme oder gute mechanische Festigkeit, werden in der Zahntherapie nicht zu Geltung kommen, wenn die Applikation der dentalen Zusammensetzung nicht optimal am Zahn erfolgen kann.

Auf der einen Seite muß das Dentalmaterial somit besonders fließfähig sein und zwar sowohl bei der Applikation durch eine enge Applikationskanüle bzw. einen statischen Mischer als auch beim Anfließen auf der Zahnsubstanz. Ein widerstandsarmes Fließverhalten durch die Applikationskanüle bzw. den statischen Mischer ist erforderlich, um die Ausdrückkräfte in akzeptablen Grenzen zu halten. Zu hohe Ausdrückkräfte verhindern u.a. eine punktgenaue Applikation und gefährden so den Behandlungserfolg. Ein optimales Anfließen des Dentalmaterials auf der Zahnsubstanz vermindert die Entstehung von Randspalten, verhindert damit die Bildung von Sekundärkaries und trägt so maßgeblich zum Erfolg der Behandlung bei.

Auf der anderen Seite soll das Dentalmaterial aber auch eine gewisse Standfestigkeit aufweisen. Noch in der Spritze, Compule oder Kartusche beugt eine ausreichend hohe Standfestigkeit der Separation des Materials vor und erhöht so seine Lagerstabilität. Zudem soll das Dentalmaterial sobald es auf die Zahnsubstanz bzw. in die Kavität appliziert und dort optimal angeflossen ist wieder eine ausreichend hohe Standfestigkeit haben, um nicht vom Zahn bzw. aus der Kavität zu "laufen".

Erreicht wird dieses Anforderungsprofil von an sich gegensätzlichen Eigenschaften z.B. durch den Einsatz von rheologiemodifizierenden Substanzen wie Aerosilen. Üblicherweise werden etwa ein bis drei Prozent Aerosil eingesetzt, um das gewünschte Schergefälle zu erzielen. Bei Verwendung der Polysiloxane reichen diese Aerosilmengen jedoch nicht aus, um ein ausreichendes Schergefälle einstellen zu können. Um ein ausreichendes Schergefälle zu erzielen, sind hier Aerosilmengen von zum Teil zehn Prozent und mehr notwendig. Diese hohen Aerosilmengen führen dann aber dazu, dass der Gesamtfüllstoffgehalt deutlich sinkt und damit auch die guten Eigenschaften, wie z.B. die hohe Biegefestigkeit und die niedrige Schrumpfung. Durch die erfindungsgemäße Kombination der Polysiloxane mit Disiloxanen wurde überraschenderweise ein Monomersystem gefunden, bei dem die üblichen Aerosilmengen von ein bis drei Prozent bereits genügen, um ein ausreichendes Schergefälle herbeizuführen. Der gefundene Effekt scheint auf dem Zusammenspiel von Polysiloxan und Disiloxan zu beruhen und nicht auf einer reinen Verdünnung des Polysiloxans. Die reine Verdünnung des Polysiloxans bzw. das Absenken des Füllstoffgehaltes führt zu einer erhöhten Fließfähigkeit über den gesamten Bereich. Das heißt, dass das Material entweder bei ausreichender Standfestigkeit nicht genügend fließfähig ist oder dass das Material bei ausreichender Fließfähigkeit nicht genügend standfest ist. Im Extremfall ist das Material sogar weder genügend standfest noch genügend fließfähig.

Die erfindungsgemäßen Kombinationen von Polysilxanen mit Disiloxanen eignen sich also besonders gut zur Herstellung von fließfähigen, härtbaren Dentalmaterialien, die durch eine Applikationskanüle oder einen statischen Mischer appliziert werden, wie fließfähigen Komposite, Stumpfaufbaumaterialien oder Befestigungskomposite. Neben guten Fließeigenschaften in Kanüle bzw. Mischer und einem optimalen Anfließen auf der Zahnsubstanz weisen diese Materialien eine ausreichend hohe Standfestigkeit auf und die durch die Polysiloxane eingebrachten guten physikalischen Eigenschaften können voll zu Tragen kommen.

Darüber hinaus wird durch die Kombination von Polysiloxanen mit Disiloxanen überraschenderweise auch noch der E-Modul des ausgehärteten Dentalmaterials erhöht. Das gehärtete Material kann also seiner Verformung einen größeren Widerstand entgegensetzen und so den stetigen Kaubelastungen besser widerstehen.

Der Einsatz von Disiloxanen in radikalisch härtbaren dentalen Zusammensetzungen, deren chemischer Aufbau auf den klassischen vernetzenden "Dentalmonomeren" wie Bis-GMA (2,2 Bis[p-(2'-hydroxy-3'-methacryloxypropoxy)-phenyl]propan), UDMA (1,6-Bis(methacryloxy-2-ethoxycarbonylamino)-2,4,4-trimethylhexan), TEGDMA (Triethylenglykoldimethacrylat), HEDMA (Hexandioldimethacrylat), etc. beruht, ist aus dem Stand der Technik bekannt.

In einer Publikation mit dem Titel "Synthesis and properties of a polyfluorinated prepolymer multifunctional urethan methacrylate" (J.M. Antonuci, J.W. Stansbury, S. Venz, "Polymeric Materials Science and Engineering", 59, 388-396 (1988)), in der sogenannte "low surface energy resins" untersucht werden, wird das Bis-(methacryloxypropyl)tetramethyldisiloxan (BIS-MPTMS) zusammen mit Triethylenglykoldimethacrylat (TEGDMA) und 1,6-Hexamethylendimethacrylat (HEDMA) als organische Harzmatrix in einer dentalen Kompositzusammensetzung in Form eines Pulver/Flüssigkeitssystems verwendet. Auf der Seite 390 findet sich im Abschnitt "Formulation of composites" der Hinweis, dass BIS-MPTMS ein ausgezeichnetes Mittel ist, die Viskosität zu verringern und eine voluminöse, flexible Struktur aufweist. Es sei über einen großen Konzentrationsbereich mit vielen Dentalharzen mischbar.

In einem Abstract mit dem Titel "Evaluation of siloxane containing dental composites" (J.S. Kuo, J.M. Antonucci,

W. Wu, "Journal of Dental Research Abstracts", 6A, and Abstract No.30 (1985)) wird das BIS-MPTMS gemeinsam mit Bis-GMA und UDMA als organische Harzmatrix verwendet. Auch hier findet sich der Hinweis, BIS-MPTMS sei mit dentalen Basismonomeren über einen großen Bereich (10 - 50 Gew.-%) mischbar. Das Fazit dieser Untersuchung lautet: "Mechanical properties of the siloxane-containing composites were almost comparable to the controls, but, significantly, had reduced WS (water sorption) and enhanced OER (oral environmental resistance)."

In einer neueren Veröffentlichung mit dem Titel "Synthesis of none Bisphenol A structure dimethacrylate monomer and characterization for dental composite applications" (X. Liang, F. Liu, J. He, "Dental Materials", 30, 917 - 925, (2014)) wurde das Reaktionsprodukt (SiMA) der ringöffnenden Additionsreaktion zwischen 1,3-Bis[2(3,4-epoxycyclohex-1-yl)ethyl]tetramethyldisiloxan und Methacrylsäure als Bis-GMA-Alternative zusammen mit TEDGMA als organische Matrix in dentalen Kompositen untersucht. Die Autoren schlussfolgern, dass die "study of SiMA based resin and composite material showed that SiMA had potential to be used in clinic, but mechanical properties of SiMA based resin and composite needed to be improved..." (Seite 923, Punkt 5, "conclusion").

Keines der Dokumente aus dem Stand der Technik zu radikalisch härtbaren dentalen Zusammensetzungen, die Polysiloxane oder Disiloxane als alternative Monomerbausteine zu den klassischen Dentalmonomeren betreffen, haben - neben der Fragestellung zu den mechanischen Eigenschaften der ausgehärteten Dentalzusammensetzung, der Viskosität während der Herstellung des Siloxans und des entsprechenden Dentalkomposits, dem Schrumpfverhalten während der Polymerisation, der Wasseraufnahme des Polymerisats, der Biokompatibilität oder den Brechungsindizes der einzelnen Bestandteile des härtbaren Dentalmaterials - das fundamentale Problem der Ausdrückbarkeit und des Anfließverhaltens an Zahnhartsubstanz der härtbaren Dentalzusammensetzung thematisiert.

Erfindungsgemäß gelöst wird die gestellte Aufgabe durch eine radikalisch härtbare dentale Zusammensetzungen enthaltend

a.) mit radikalisch polymerisierbaren Gruppen substituierte kettenförmige und/oder zyklische und/oder in Käfigform vorliegende Polysiloxane mit mindestens 3 Siliziumatomen und/oder deren Mischformen,

b.) mit radikalisch polymerisierbaren Gruppen substituierte Disiloxane der folgenden Struktur

$$R^1{}_a\ R^2{}_{(3-a)}Si\text{-}O\text{-}SiR^2{}_{(3-b)}R^1{}_b$$

mit

$R^2$: Alkyl, Alkenyl, Aryl, Alkylaryl, Arylalkyl, wobei unterschiedliche $R^2$ gleich oder verschieden sein können,
$R^1$: YZ,
Z: radikalisch polymerisierbare Gruppe, ausgewählt aus den Strukturelementen $-O-(C=O)-CH=CH_2$, $-O-(C=O)-C(CH_3)=CH_2$, $-(C=O)-CH=CH_2$, $-(C=O)-C(CH_3)=CH_2$, $-CH=CH_2$, $-C(CH_3)=CH_2$, $-NH-(C=O)-CH=CH_2$ und $-NH-(C=O)-C(CH_3)=CH_2$,
wobei unterschiedliche Z gleich oder verschieden sein können,
a: 1 oder 2,
b: 1 oder 2,
Y: ein Verbindungselement, das das Siliziumatom mit der radikalisch polymerisierbaren Gruppe verknüpft und aus einer Alkylengruppe besteht,
wobei die Alkylenguppe eine unsubstituierte, durchgängige, geradkettige oder verzweigte Kohlenwasserstoffkette ist oder
wobei die Alkylengruppe eine unsubstituierte, durch eine Urethangruppe, Harnstoffgruppe, Estergruppe, Thiourethangruppe oder Amidgruppe unterbrochene Kohlenwasserstoffgruppe ist oder
wobei die Alkylengruppe eine durch eine Hydroxylgruppe substituierte Kohlenwasserstoffgruppe ist und/oder diese Hydroxylgruppe verestert oder verethert ist oder
wobei die Alkylengruppe eine durch ein Sauerstoffatom und/oder Stickstoffatom und/oder Schwefelatom und/oder Estergruppen und/oder Thioestergruppen unterbrochene und durch eine Hydroxylgruppe substituierte Kohlenwasserstoffgruppe ist und/oder diese Hydroxylgruppe verestert oder verethert ist und
wobei unterschiedliche Y gleich oder verschieden sein können und
wobei Y 20 oder weniger Kohlenstoffatome enthält und
wobei YZ so gewählt wird, dass Z immer möglichst viele Atome aufweist,

c.) optional ein, zwei, drei oder mehrere radikalisch härtbare Monomere ohne Siliziumatom,

d.) 85 Gewichtsprozent oder weniger Füllstoffe umfassend:

d.)1. 1 - 20 Gew.-% organisch oberflächenmodifizierte anorganische Nanopartikel mit einer mittleren Teilchengröße kleiner 200 nm,

d.)2. 30 - 84 Gew.-% anorganische Mikropartikel mit einer mittleren Teilchengröße im Bereich von 0,4 μm und 10 μm sowie

d.)3. 0 - 30 Gew.-% weitere Füllstoffe, die nicht d.) 1. und d.) 2. entsprechen, wobei die Gewichtsangaben auf die Gesamtmasse der radikalisch härtbaren dentalen Zusammensetzung bezogen sind,

e.) Initiatoren und/oder Katalysatoren für die radikalische Polymerisation und
f.) weitere übliche Additive.
wobei die Zusammensetzung die Komponente

a.) zu 10-35 Gew.-%,
b.) zu 2-25 Gew.-%,
c.) zu 0-20 Gew.-%,
d.) zu 50-85 Gew.-%,
e.) zu 0,001-5 Gew.-% und
f.) zu 0,001-20 Gew.-%

enthält und wobei die jeweiligen Gewichtsprozente auf die Gesamtmasse der Zusammensetzung bezogen sind.

Bei einer bevorzugten erfindungsgemäßen Zusammensetzung handelt es sich um eine chemische und/oder durch Licht induziert induziert radikalisch härtbare dentale Zusammensetzung.

Bei einer ganz bevorzugten erfindungsgemäßen Zusammensetzung handelt es sich um eine radikalisch härtbare dentale Zusammensetzung, wobei die Polysiloxane a.) durch Hydrolyse oder Teilhydrolyse und anschließende Kondensation oder Cokondensation von ein, zwei, drei oder mehr Verbindungen $R^1{}_aR^2{}_bSiX_c$ mit

X: Halogen oder Alkoxy
$R^2$: Alkyl, Alkenyl, Aryl, Alkylaryl, Arylalkyl, wobei unterschiedliche $R^2$ gleich oder verschieden sein können,
$R^1$: YZ,
Z: radikalisch polymerisierbare Gruppe, ausgewählt aus den Strukturelementen $-O-(C=O)-CH=CH_2$, $-O-(C=O)-C(CH_3)=CH_2$, $-(C=O)-CH=CH_2$, $-(C=O)-C(CH_3)=CH_2$, $-CH=CH_2$, $-C(CH_3)=CH_2$, $-NH-(C=O)-CH=CH_2$ und $-NH-(C=O)-C(CH_3)=CH_2$,
wobei unterschiedliche Z gleich oder verschieden sein können,
a: 1 oder 2,
b: 0 oder 1,
c: 2 oder 3,
a + b + c = 4,
Y: ein Verbindungselement, das das Siliziumatom mit der radikalisch polymerisierbaren Gruppe verknüpft und aus einer Alkylengruppe besteht,
wobei die Alkylenguppe eine unsubstituierte, durchgängige, geradkettige oder verzweigte Kohlenwasserstoffkette ist oder
wobei die Alkylengruppe eine unsubstituierte, durch eine Urethangruppe, Harnstoffgruppe, Estergruppe, Thiourethangruppe oder Amidgruppe unterbrochene Kohlenwasserstoffgruppe ist oder
wobei die Alkylengruppe eine durch eine Hydroxylgruppe substituierte Kohlenwasserstoffgruppe ist oder diese Hydroxylgruppe verestert oder verethert ist oder wobei die Alkylengruppe eine durch ein Sauerstoffatom und/oder Stickstoffatom und/oder Schwefelatom und/oder Estergruppen und/oder Thioestergruppen unterbrochene und durch eine Hydroxylgruppe substituierte Kohlenwasserstoffgruppe ist oder diese Hydroxylgruppe verestert oder verethert ist und
wobei unterschiedliche Y gleich oder verschieden sein können und
wobei Y 20 oder weniger Kohlenstoffatome enthält und
wobei YZ so gewählt wird, dass Z immer möglichst viele Atome aufweist.

erhalten werden.

Bestandteil a.) - mit radikalisch polymerisierbaren Gruppen substituierte kettenförmige und/oder zyklische und/oder in Käfigform vorliegende Polysiloxane mit mindestens 3 Siliziumatomen und/oder deren Mischformen.

Mit radikalisch polymerisierbaren Gruppen substituierte kettenförmige und/oder zyklische und/oder in Käfigform vorliegende Polysiloxane mit mindestens 3 Siliziumatomen und/oder deren Mischformen können über den Sol-Gel Prozeß durch gezielte Hydrolyse und Kondensation von entsprechend funktionalisierten Derivaten von Alkoxiden des Siliziums oder von Halogensilanen synthetisiert werden. Diese Herstellverfahren sind in der Literatur vielfach beschrieben. In der Regel geht man bei einer solchen Synthese von einem Standardsilan wie beispielsweise dem Isocyanatopropyldiethoxysilan aus, das in einem ersten Schritt, ebenfalls in einer Standardreaktion, beispielsweise in einer Isocyanat-Alkohol Polyaddition, beispielsweise mit dem Glycerin-1,3-dimethacrylat zum entsprechenden Urethan umgesetzt wird. Die hierbei erhaltene Verbindung besteht auf der einen Seite aus dem Siliziumatom, das mit hydrolysier- und kondensierbaren Gruppen bestückt sind und das über einen sogenannten Abstandshalter, bestehend aus einer Alkylgruppe (hier eine Propylgruppe) und einer Urethangruppe als strukturelles Verbindungselement zu einem weiteren funktionellen Struktursegment, in diesem Fall zu zwei radikalisch polymerisierbare Methacrylatgruppen übergeht. Ein solch einfaches Syntheseverfahren kann in vielfacher Weise modifiziert werden, da die Reaktionsmöglichkeiten zwischen entsprechend funktionalisierten Silanen und geeigneten Reaktanden unbegrenzt erscheinen. Entsprechend groß sind die Synthesevorschläge in der Literatur. Die Ausgangsverbindung umfasst somit ein anorganisch kondensierbares Strukturelement, ein variabel gestaltbares Verbindungselement sowie ein radikalisch vernetzbares organisches Grundgerüst. In einer katalytisch gesteuerten Hydrolyse und Kondensation wird das Polysiloxan als ein anorganisches Kondensat, substituiert mit radikalisch polymerisierbaren Gruppen, gewonnen. Ob das Polykondensat in Form von Ketten, Ringen oder dreidimensionalen Käfigformen, bzw. in den entsprechenden Mischformen vorliegt, hängt von den genauen Bedingungen der Kondensation ab. Dazu zählen neben den Reaktionsbedingungen (pH-Wert, Menge an Lösungsmittel und Wasser, Art und Menge des Katalysators, Reaktionstemperatur, Art der Aufbereitung, etc.) auch die Strukturformen des Ausgangssilans, wobei die Anzahl der Alkoxygruppen, die Anzahl der radikalisch polymerisierbaren Gruppen, die chemische Art des Verbindungselements sowie die Kettenlänge des Abstandshalters von Bedeutung sind. Angaben hierzu finden sich sowohl in der wissenschaftlichen Literatur wie in der Patentliteratur.

Die Polysiloxane verfügen als Bindeglied zwischen anorganischer und organischer Chemie über besondere Materialeigenschaften. Da sie zudem physiologisch inert sind, also keine nennenswerte Toxizität besitzen, sind sie speziell für Anwendungen in der Medizin bedeutsam. Grundlage für die kaum vorhandene Toxizität der Polysiloxane ist die geringe biologische Angreifbarkeit der Silizium-Kohlenstoffbindungen und die eingeschränkte Diffusionsfähigkeit der stark hydrophoben Polymerketten durch Zellmembranen, weshalb sie sich besonders zur Implantation (in Zähne) eignen sollten.

1

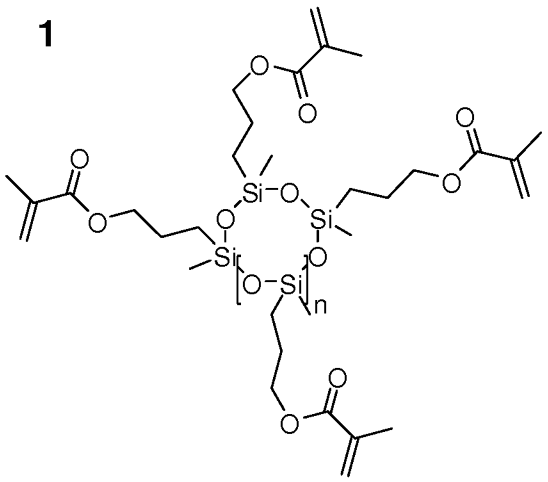

2

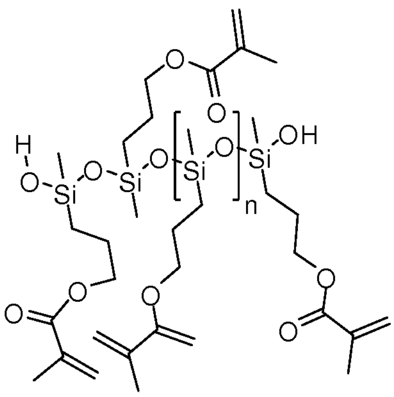

**3**

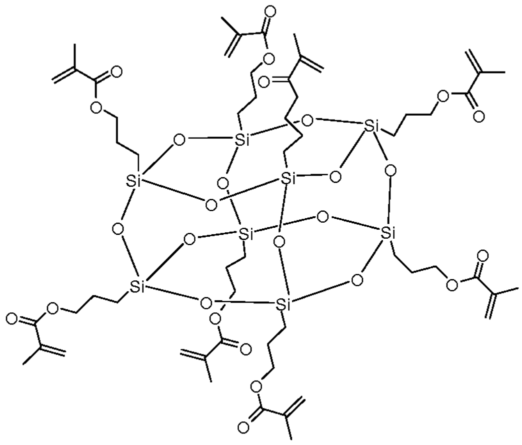

**4**

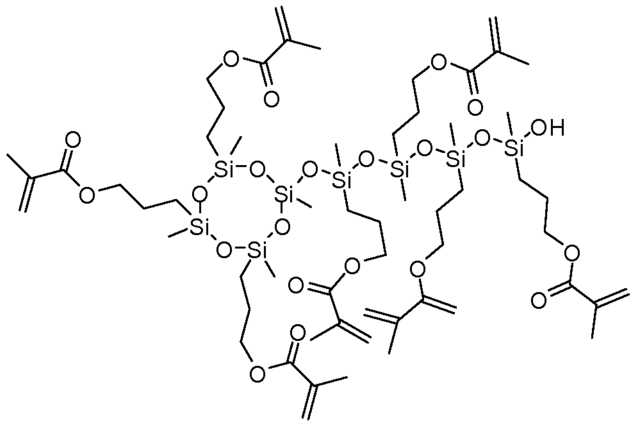

Bestandteil b.) - mit radikalisch polymerisierbaren Gruppen substituierte Disiloxane der folgenden Struktur $R^1_a R^2_{(3-a)}Si\text{-}O\text{-}SiR^2_{(3-b)}R^1_b$ mit

$R^2$: Alkyl, Alkenyl, Aryl, Alkylaryl, Arylalkyl, wobei unterschiedliche $R^2$ gleich oder verschieden sein können,
$R^1$: YZ,
Z: radikalisch polymerisierbare Gruppe, ausgewählt aus den Strukturelementen $-O-(C=O)-CH=CH_2$, $-O-(C=O)-C(CH_3)=CH_2$, $-(C=O)-CH=CH_2$, $-(C=O)-C(CH_3)=CH_2$, $-CH=CH_2$, $-C(CH_3)=CH_2$, $-NH-(C=O)-CH=CH_2$ und $-NH-(C=O)-C(CH_3)=CH_2$,
wobei unterschiedliche Z gleich oder verschieden sein können,
a: 1 oder 2,
b: 1 oder 2,
Y: ein Verbindungselement, das das Siliziumatom mit der radikalisch polymerisierbaren Gruppe verknüpft und aus einer Alkylengruppe besteht,
wobei die Alkylenguppe eine unsubstituierte, durchgängige, geradkettige oder verzweigte Kohlenwasserstoffkette ist oder
wobei die Alkylengruppe eine unsubstituierte, durch eine Urethangruppe, Harnstoffgruppe, Estergruppe, Thiourethangruppe oder Amidgruppe unterbrochene Kohlenwasserstoffgruppe ist oder
wobei die Alkylengruppe eine durch eine Hydroxylgruppe substituierte Kohlenwasserstoffgruppe ist und/oder diese Hydroxylgruppe verestert oder verethert ist oder
wobei die Alkylengruppe eine durch ein Sauerstoffatom und/oder Stickstoffatom und/oder Schwefelatom und/oder Estergruppen und/oder Thioestergruppen unterbrochene und durch eine Hydroxylgruppe substituierte Kohlenwasserstoffgruppe ist und/oder diese Hydroxylgruppe verestert oder verethert ist und
wobei unterschiedliche Y gleich oder verschieden sein können und
wobei Y 20 oder weniger Kohlenstoffatome enthält und
wobei YZ so gewählt wird, dass Z immer möglichst viele Atome aufweist,

Disiloxane zeichnen sich durch eine außergewöhnliche Kettenbeweglichkeit aus, da die Verbindungen eine freie Rotation um die Silizium-Sauerstoffverbindungen aufweisen, die u.a. auch durch den Größenunterschied zwischen dem Sauerstoffatom und dem Siliziumatom begründet ist. Dies führt zu Verbindungen, die aufgrund der Flexibilität ihres Strukturgerüsts eine auffallend niedrige Viskosität besitzen. Weiterhin führt die Abschirmung der Sauerstoffatome durch die Kohlenwasserstoffgruppen am Silizium zu einer ausgeprägten Hydrophobie und zu einer stark eingeschränkten Wechselwirkung der Ketten untereinander, so dass das Spreiten auf Oberflächen wie beispielsweise auf Zahnhartsubstanz begünstigt werden sollte und ein extrem gutes Anfließen der Polysiloxane beispielsweise an präparierte Kavitätenwänden zur Folge haben dürfte.

Neben einigen kommerziell erhältlichen erfindungsgemäßen Disiloxanen (z.B. 1,3-Bis(3-methacryloxypropyl)tetramethylsisiloxan, 1,3-Bis(3-methacryloxy-2-hydroxypropoxypropyl)tetra-methyldisiloxan, 1,3-Bis[(acryloxymethyl)phenethyl]tetramethyldisiloxan) gibt es für die Herstellung der erfindungsgemäßen Disiloxane zahlreiche Synthesestrategien.

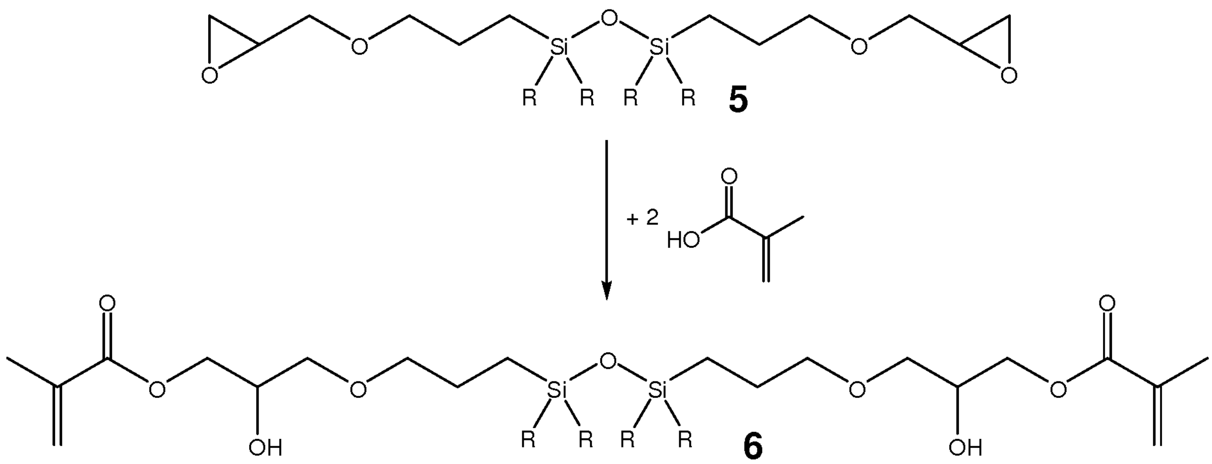


5

6

Das 1,3-Bis(3-methacryloxy-2-hydroxypropoxypropyl)disiloxan **6** lässt sich durch Umsetzung des entsprechenden 1,3-Bis(3-glycidoxypropyl)disiloxans **5** mit Methacrylsäure herstellen.

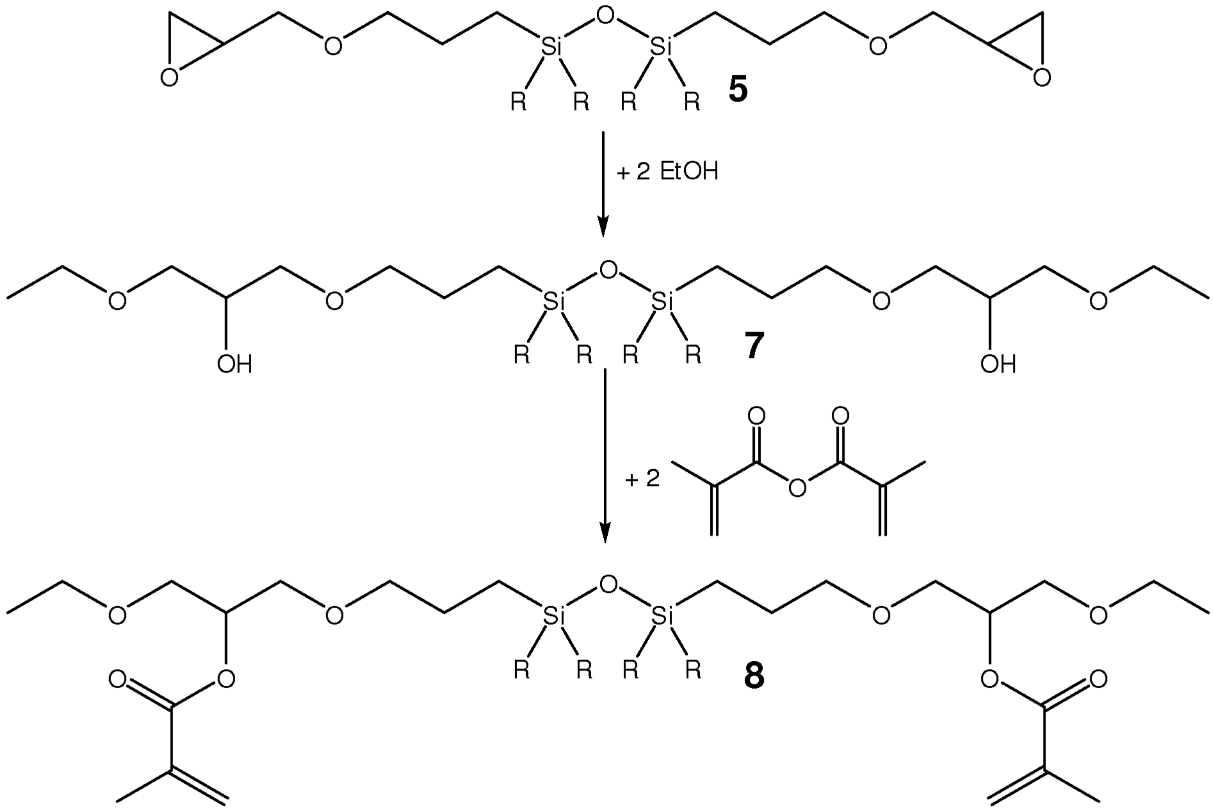


5

7

8

Aus dem 1,3-Bis(3-glycidoxypropyl)disiloxans **5** lässt sich durch Umsetzung mit Ethanol und anschließend Methacrylsäureanhydrid das Disiloxan **8** synthetisieren.

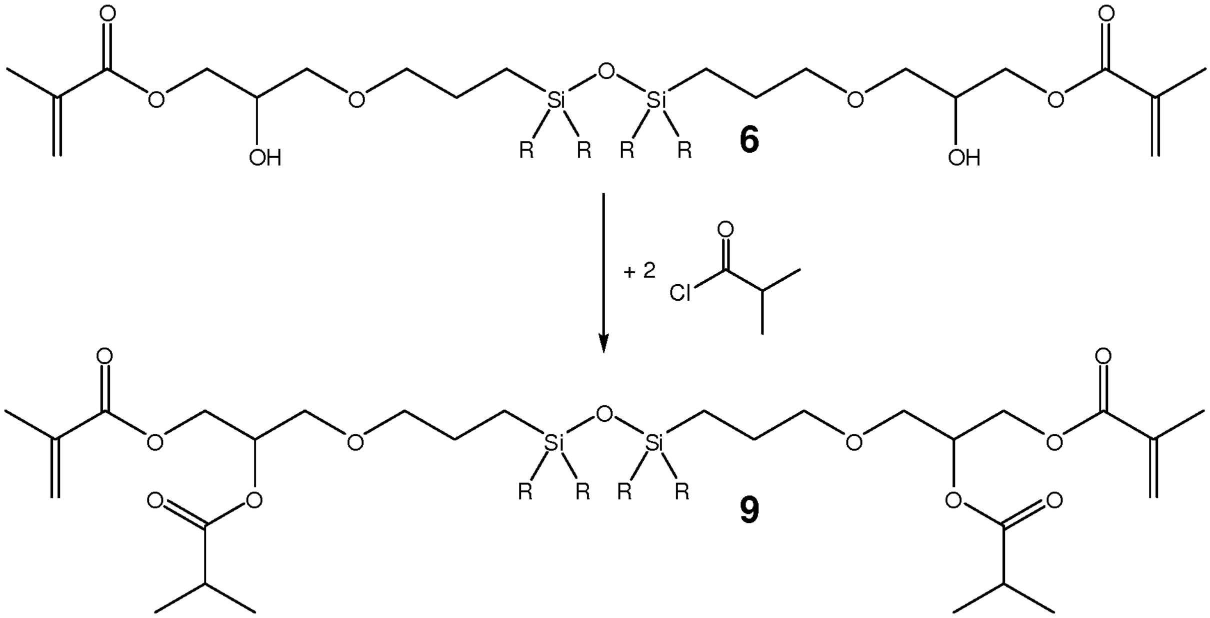


6

9

Aus dem 1,3-Bis(3-methacryloxy-2-hydroxypropoxypropyl)disiloxan **6** lassen sich durch weitere Umsetzung mit unterschiedlichen Säurechloriden oder -anhydriden die entsprechenden Disiloxane **9**, **10**, **11**, **12** und **13** herstellen.

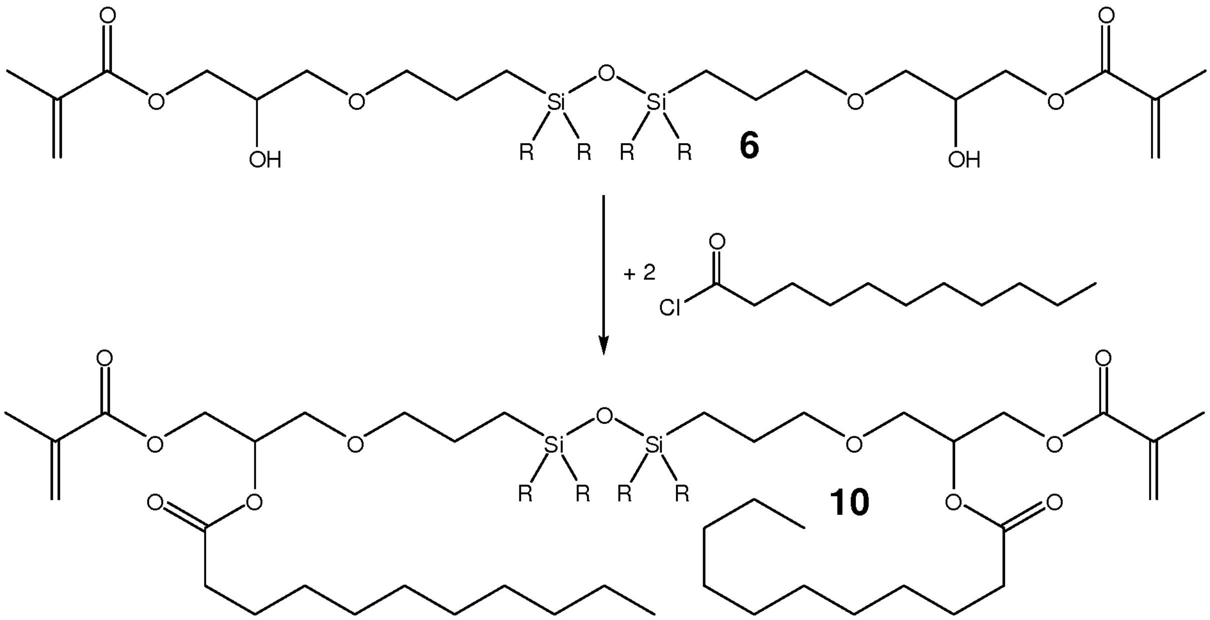


6

10

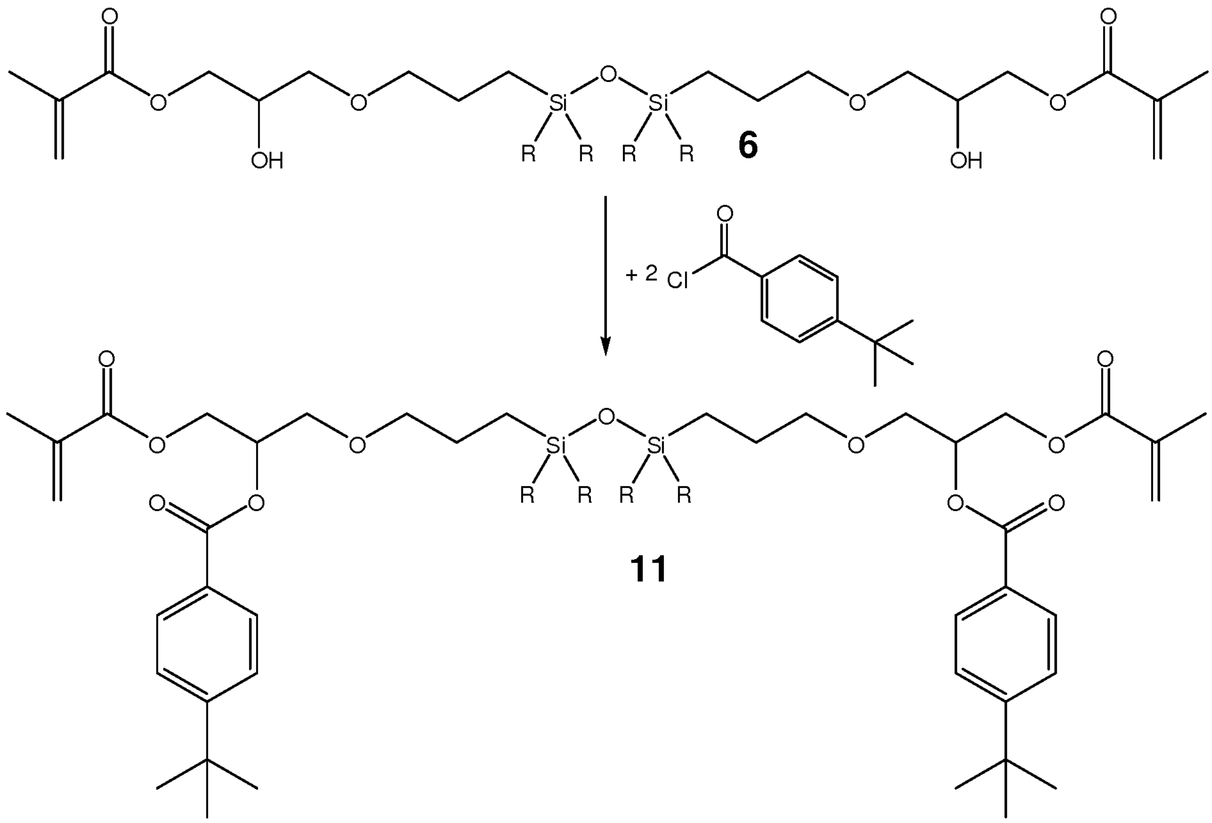


6

11

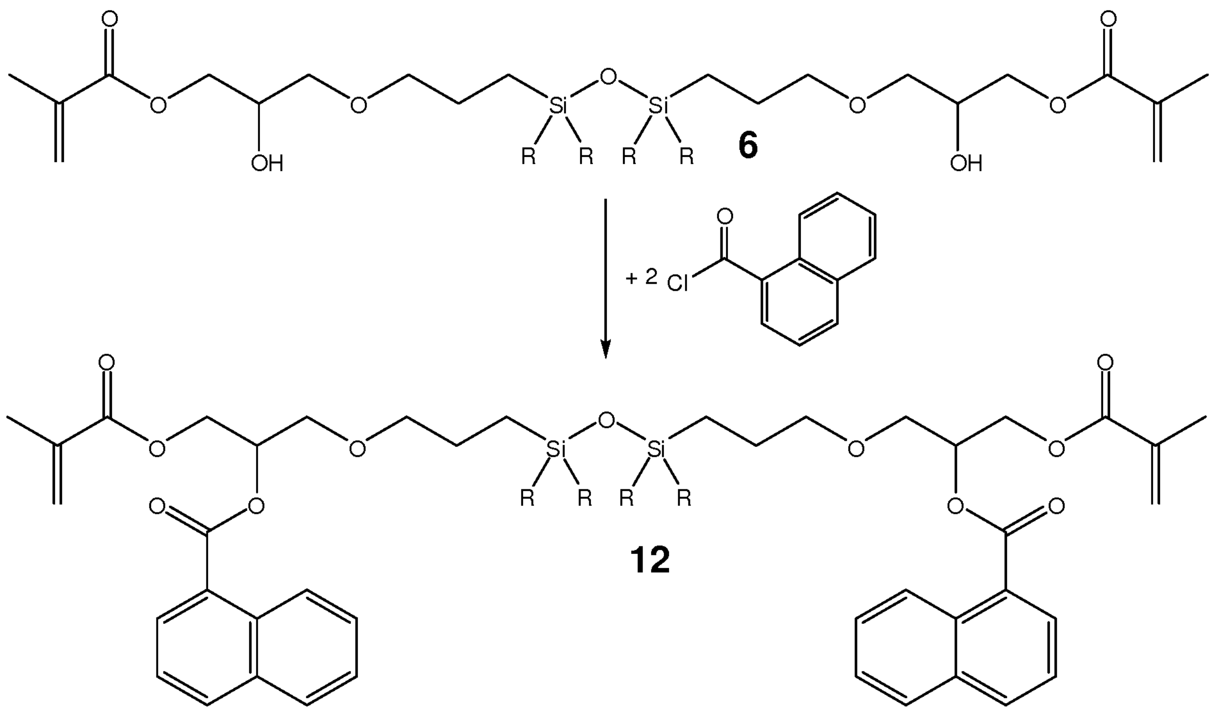


6

12

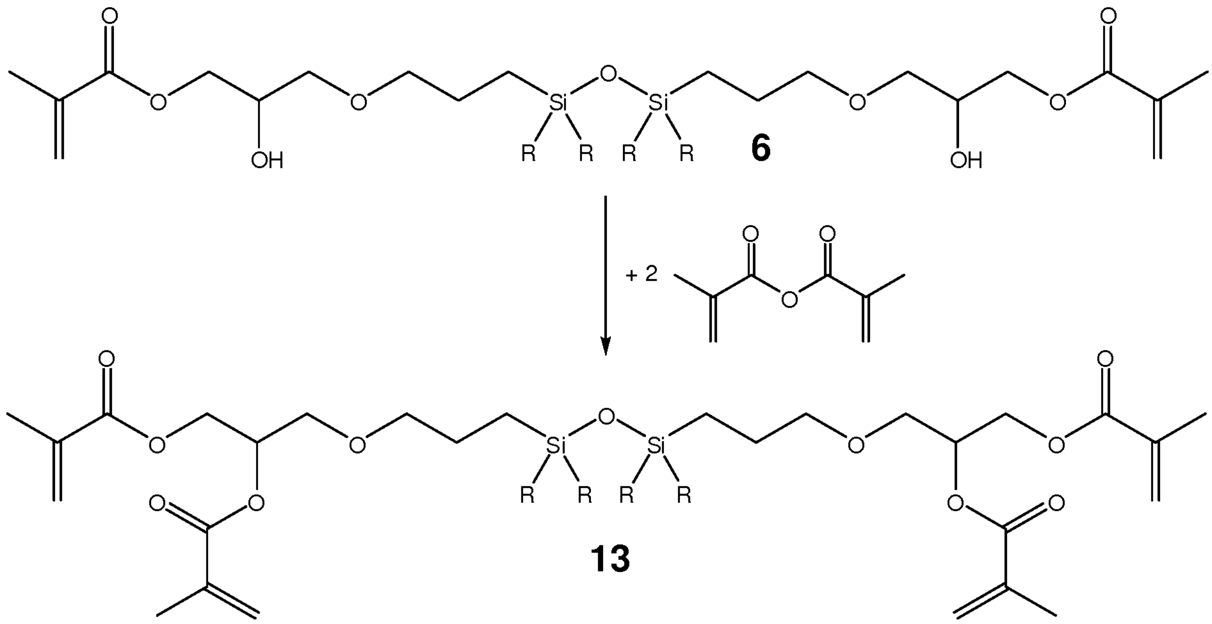


6

13

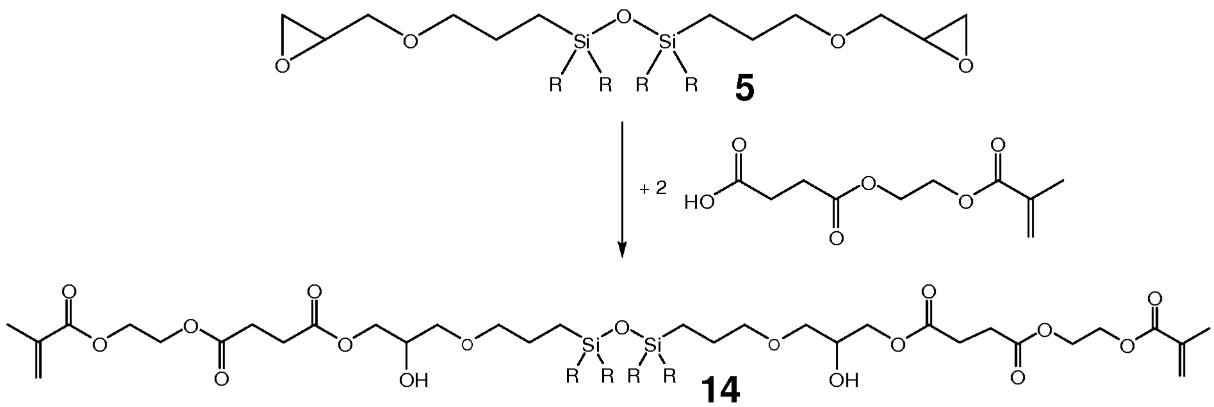


5

14

Aus dem 1,3-Bis(3-glycidoxypropyl)disiloxans **5** lässt sich auch durch Umsetzung mit Bernsteinsäuremono(2-methacryloxyethyl)ester das entsprechende Disiloxan **14** synthetisieren.

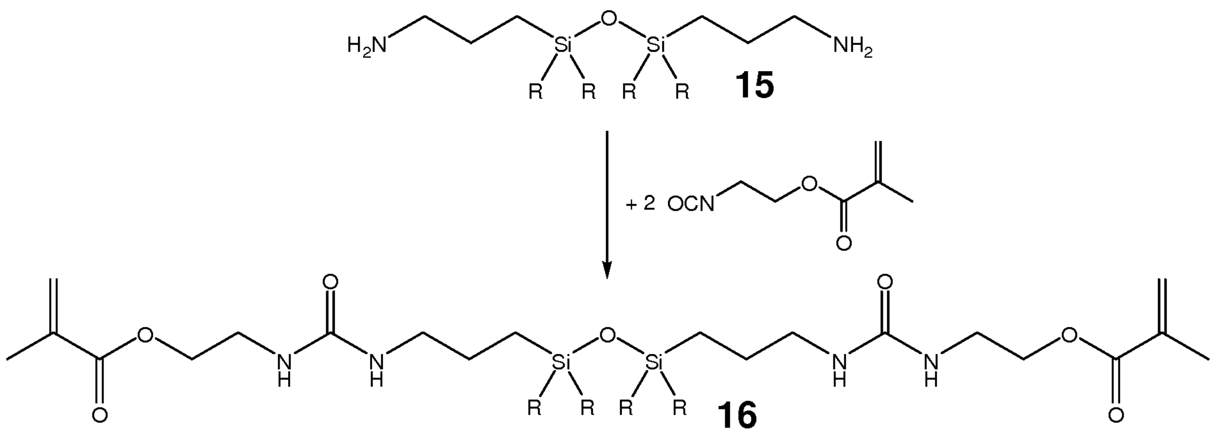


15

16

Aus dem kommerziell erhältlichen 1,3-Bis(3-aminopropyl)disiloxan **15** erhält man durch Umsetzung mit Methacrylsäure-2-isocyanatoethylester das entsprechende Harnstoff-Derivat **16**.

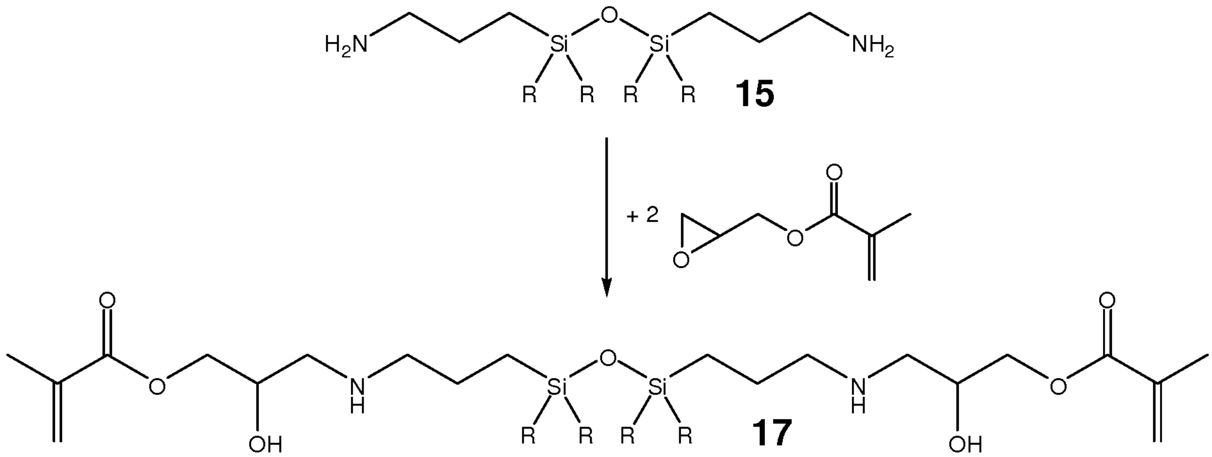


15

17

Aus dem 1,3-Bis(3-aminopropyl)disiloxan **15** lassen sich durch Umsetzung mit 2 bzw. 4 Äquivalenten Methacrylsäureglycidylester die entsprechenden Disiloxane **17** bzw. **18** herstellen.

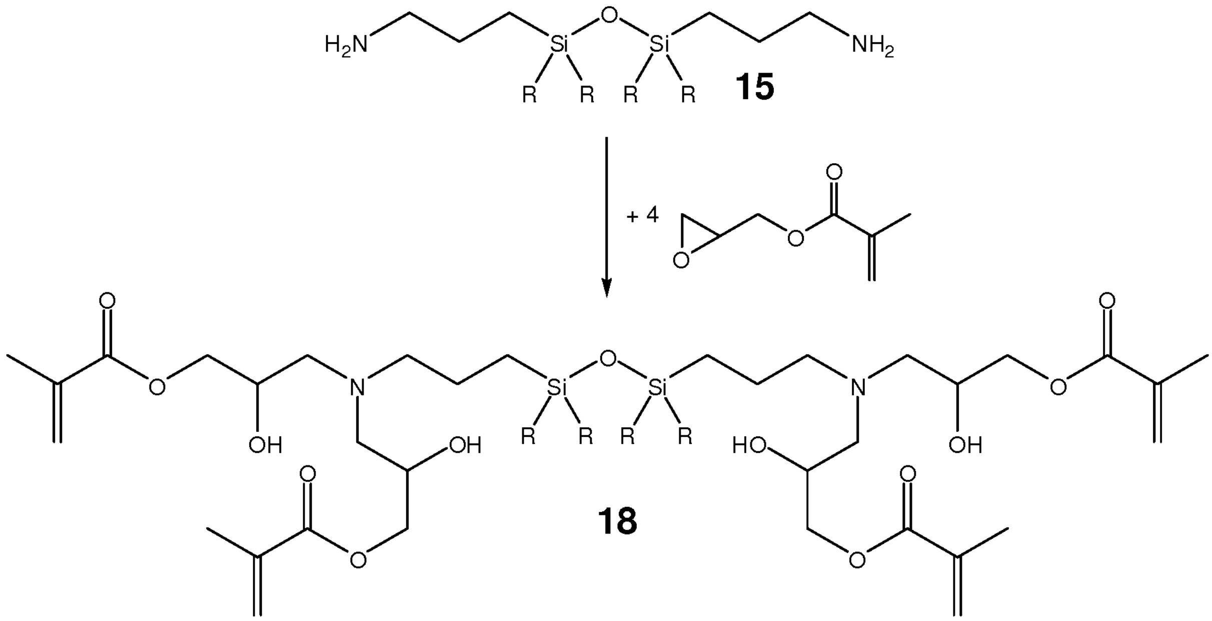


15

18

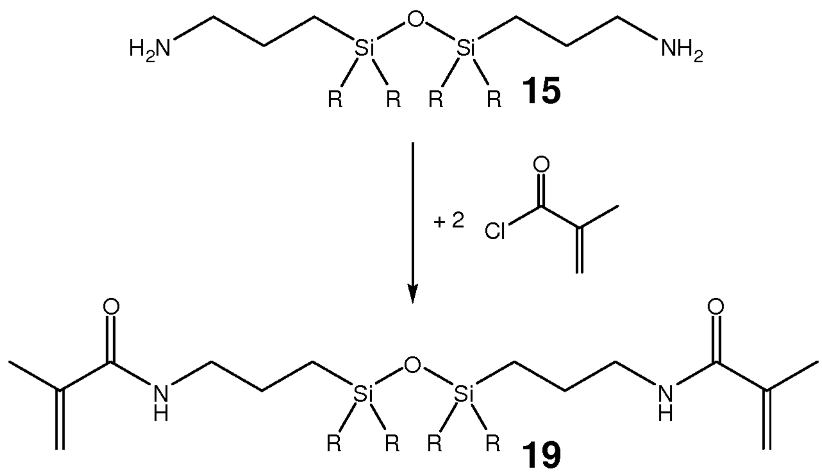


15

19

Ebenfalls aus dem 1,3-Bis(3-aminopropyl)disiloxan **15** ist das 1,3-Bis(3-methacrylamidopropyl)disiloxan **19** durch Umsetzung mit Methacrylsäurechlorid erhältlich.

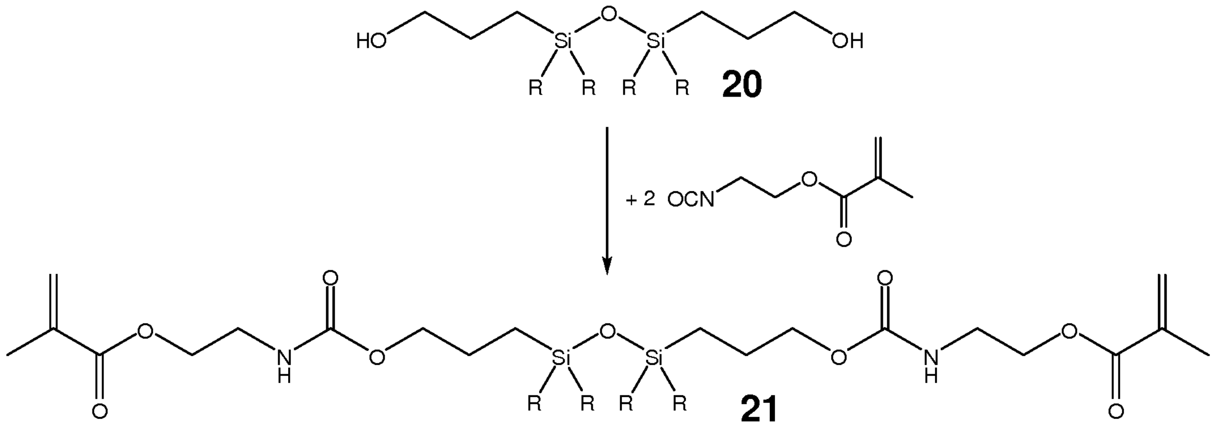


20

21

Aus dem kommerziell erhältlichen 1,3-Bis(3-hydroxypropyl)disiloxan **20** erhält man durch Umsetzung mit Methacrylsäure-2-isocyanatoethylester das entsprechende Urethan-Derivat **21**.

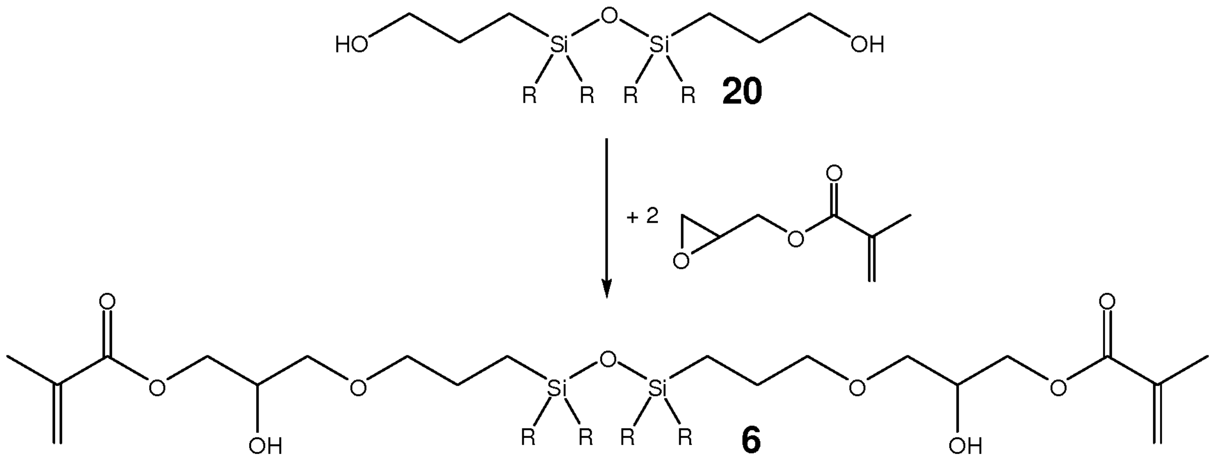


20

6

Aus dem 1,3-Bis(3-hydroxypropyl)disiloxan **20** ist durch Umsetzung mit Methacrylsäureglycidylester auch wiederum das entsprechende 1,3-Bis(3-methacryloxy-2-hydroxypropoxypropyl)disiloxan **6** erhältlich.

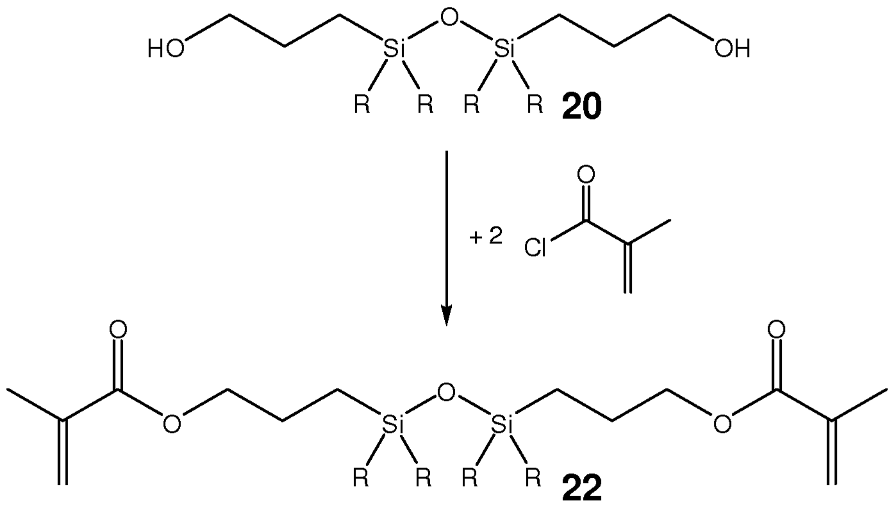


20

22

Ebenfalls aus dem 1,3-Bis(3-hydroxypropyl)disiloxan **20** erhält man durch Umsetzung mit Methacrylsäurechlorid das entsprechende 1,3-Bis(3-methacryloxypropyl)disiloxan **22**.

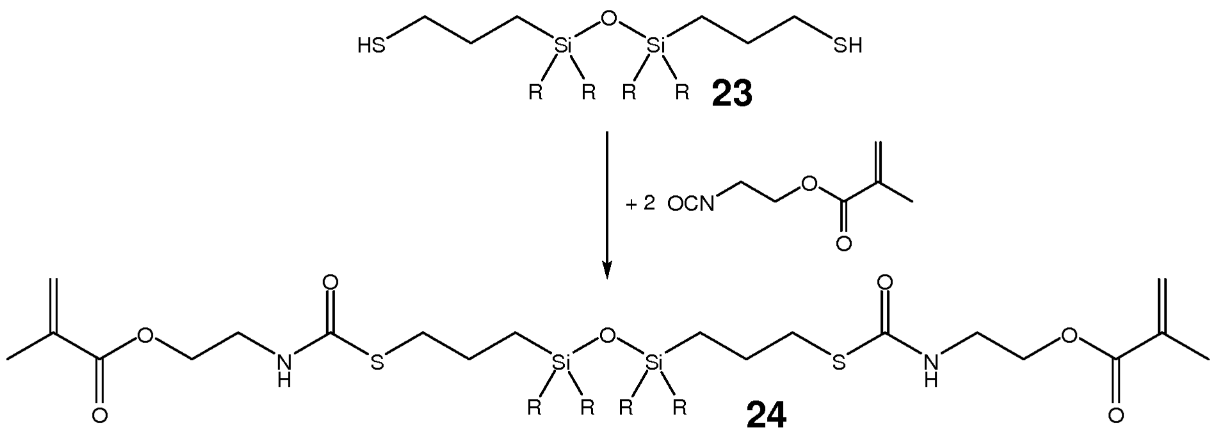


23

24

Aus dem kommerziell erhältlichen 1,3-Bis(3-mercaptopropyl)disiloxan **23** erhält man durch Umsetzung mit Methacrylsäure-2-isocyanatoethylester das entsprechende Thiourethan-Derivat **24**.

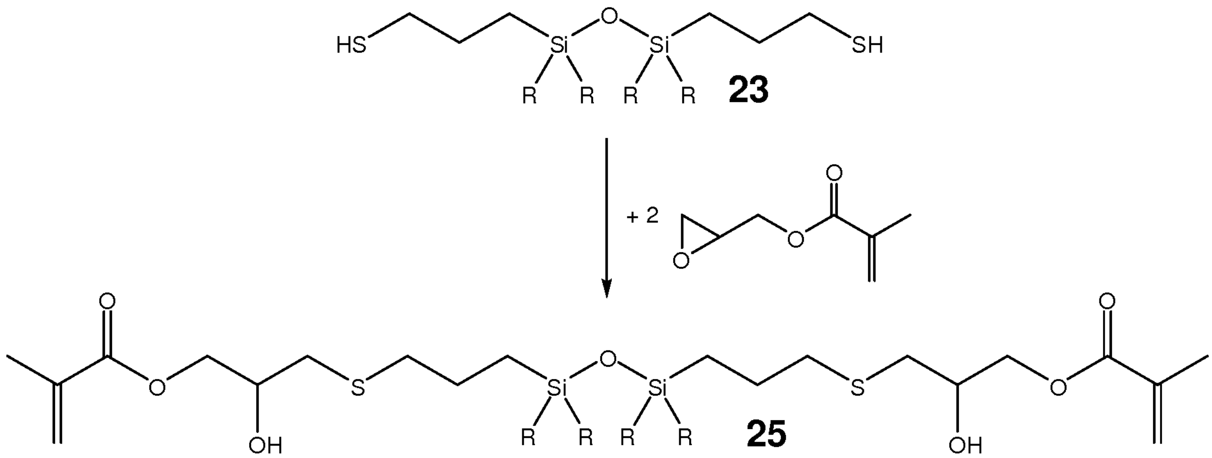


23

25

Aus dem 1,3-Bis(3-mercaptopropyl)disiloxan **23** ist durch Umsetzung mit Methacrylsäureglycidylester auch das entsprechende Disiloxan **25** erhältlich.

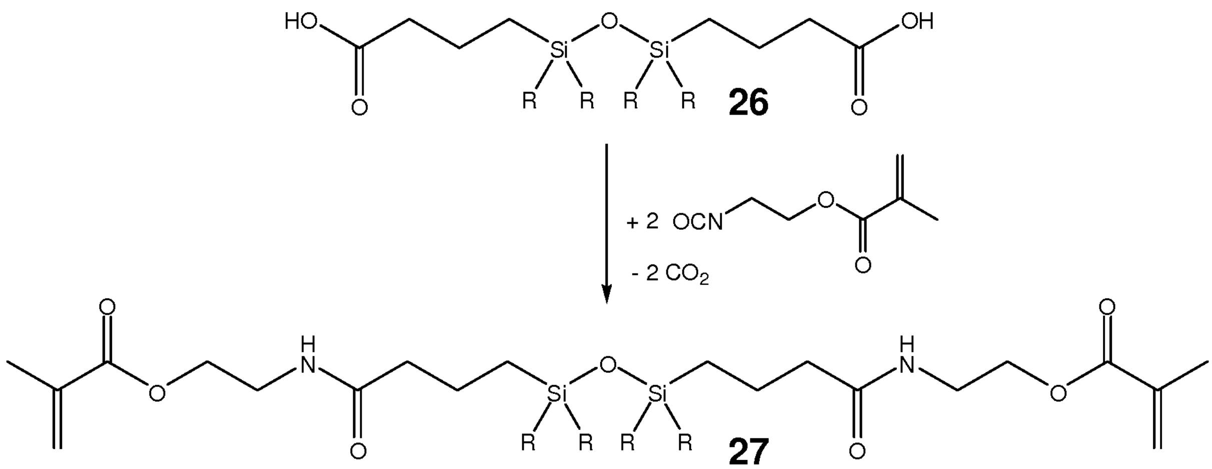


26

27

Aus dem kommerziell erhältlichen 1,3-Bis(3-carboxypropyl)disiloxan **26** erhält man durch Umsetzung mit Methacrylsäure-2-isocyanatoethylester das entsprechende Amid-Derivat **27**.

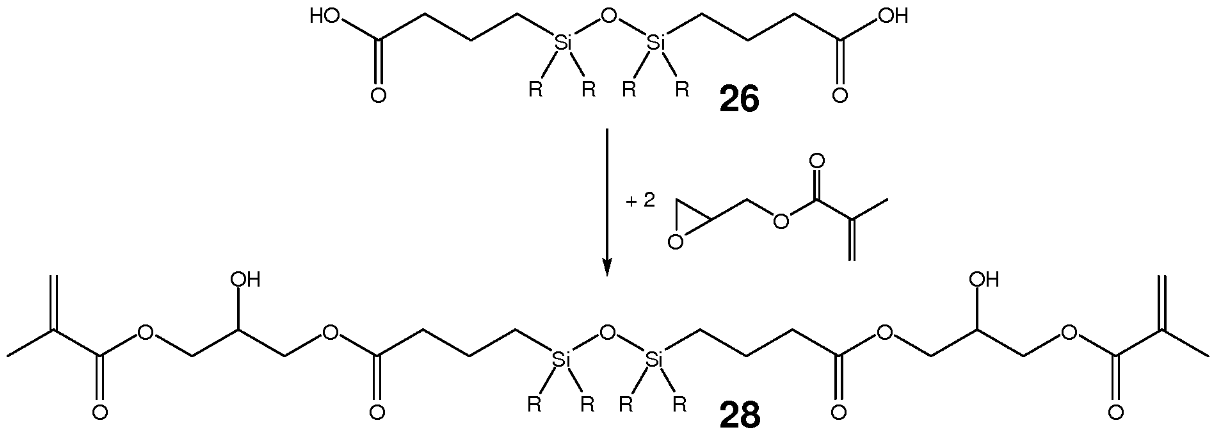


26

28

Aus dem 1,3-Bis(3-carboxypropyl)disiloxan **26** ist durch Umsetzung mit Methacrylsäureglycidylester auch das entsprechende Disiloxan **28** erhältlich.

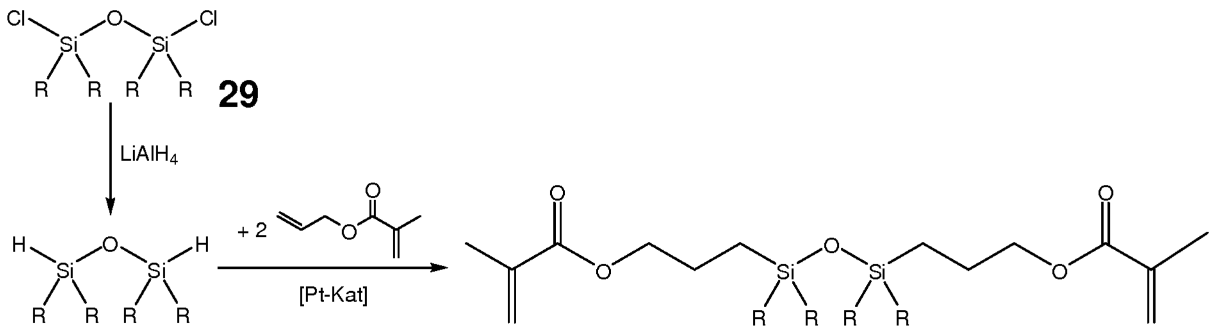


29

30

22

Selbstverständlich sind auch ausgehend von den Dichlorodisiloxanen durch typische Silanchemie eine Vielzahl von (meth)acrylsubstituierten Disiloxanen erhältlich. Beispielhaft sei hier die Umsetzung mit Lithiumaluminiumhydrid zum Dihydrodisiloxan und anschließende Platin-katalysierte Umsetzung mit Allylmethacrylat zum entsprechenden 1,3-Bis(3-methacryloxypropyl)disiloxan **22** erwähnt.

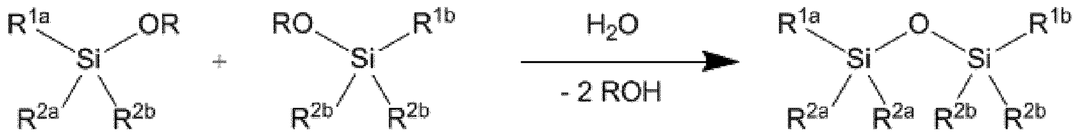


31a 31b 32

Selbstverständlich sind die erfindungsgemäßen Disiloxane nicht nur durch Funktionalisierung von Disiloxanen erhältlich, sondern lassen sich auch ausgehend von Monoalkoxysilanen durch Hydrolyse und Kondensation darstellen. So sind beispielsweise auch die unsymmetrischen Disiloxane **32** durch Umsetzung unterschiedlicher Monoalkoxysilane **31**a/b erhältlich. Für die Funktionalisierung der Monoalkoxysilane sind unter anderem die gleichen Funktionalisierungsreaktionen denkbar, die bereits für die Disiloxane beschrieben wurden.

Bestandteil c.) - optional ein, zwei, drei oder mehrere radikalisch härtbare Monomere ohne Siliziumatom

Die radikalisch härtbaren Monomeren ohne Si-Atom sind Monomere, die bevorzugt eine, zwei oder mehr ethylenische Gruppen aufweisende Substanzen sind, wie beispielsweise, ohne darauf beschränkt zu sein, die in der Dentalchemie üblicherweise eingesetzten (Meth)acrylatmonomere.

Die (Meth)acrylatmonomere können monofunktionell sowie polyfunktionell sein.

Vorzugsweise eingesetzte monofunktionelle (Meth)acrylatmonomere stellen die Ester der (Meth)acrylsäure mit Alkylgruppen von 1 bis 12 C-Atomen sowie Ester der (Meth)acrylsäure, die aromatische Gruppen mit 6 bis 12 C-Atomen enthalten, wobei die Alkylgruppen und aromatischen Gruppen, die die Ester bilden, Substituenten wie Hydroxylgruppen und Etherbindungen enthalten können, dar.

In der Patentliteratur ist eine Vielzahl weiterer Verbindungen genannt (beispielsweise auch in der DE 39 41 629 A1, welche im Wege der Verweisung Bestandteil der vorliegenden Anmeldung ist), die allesamt Ester der Acryl- oder Methacrylsäure sind und sich zum Einsatz in einem härtbaren Gemisch eignen.

Die radikalisch polymerisierbaren Monomere können auch mindestens eine ethylenische Doppelbindung aufweisende Hydroxylverbindungen sein. Dabei können vorzugsweise die in der Dentalchemie üblicherweise eingesetzten Hydroxylverbindungen von (Meth)acrylaten eingesetzt werden.

Auch können als Beispiele polyfunktioneller (Meth)acrylatmonomere Di(meth)acrylate von Alkylenglykol mit 2 bis 20 C-Atomen, Di(meth)acrylate von Oligomeren des Alkylenglykols, Polyalkylenglykoldi(meth)acrylat, Di(meth)acrylate von Bisphenol A bzw. vom Diglycidylether von Bisphenol A genannt werden.

Besonders bevorzugt sind weiterhin radikalisch härtbare Verbindungen, die auf ein zentrales polyalicyclisches Strukturelement beruhen, wie beispielsweise 3(4), 8(9)-Bis((meth)acryloyloxymethyl)tricyclo[5.2.1.$0^{2,6}$]decan, alkoxyliertes 3(4), 8(9)-Bis((meth)acryloyloxymethyl)tricyclo[5.2.1.$0^{2,6}$]decan, 2,3-Bis((meth)acryloyloxymethyl)-bicyclo[2.2.1]heptan, alkoxyliertes 2,3-Bis((meth)acryloyloxymethyl)bicyclo[2.2.1]heptan, 1,3,5-Tri(meth)acryloyloxytricyclo[3.3.1.$1^{3,7}$]decan, alkoxyliertes Tri(meth)acryloyloxytricyclo-[3.3.1.$1^{3,7}$]decan sowie (Meth)acrylsäureester von Tricyclo[5.2.1.$0^{2,6}$]decan-3(4),8(9)-dimethanol, alkoxyliertem Tricyclo[5.2.1.$0^{2,6}$]decan-3(4),8(9)-dimethanol, Bicyclo[2.2.1]heptan-2,3-dimethanol, alkoxyliertem Bicyclo[2.2.1]heptan-2,3-dimethanol, 1,3,5-Adamantantriol, alkoxyliertem 1,3,5-Adamantantriol, wobei zwischen dem polyalicyclischen Strukturelement und den (Meth)acrylsäureestern Urethan-, Harnstoff-, Amid-, Allophanat-, Acylharnstoff- oder Biuretgruppen angeordnet sind.

Angaben zur Herstellung dieser substituierten (Meth)acrylsäureester finden sich in den Patentanmeldungen EP 11 183 333, EP 11 183 328, EP 11 183 345, EP 11 183 338, EP 11 183 342 und EP 11 188 086 sowie in den in diesen Dokumenten zitierten Schriftstücken. Diese Literaturstellen sind im Wege der Verweisung ebenfalls Bestandteil der vorliegenden Anmeldung.

Bevorzugt sind ebenfalls Urethan(meth)acrylatester, Reaktionsprodukte aus 2 Mol eines (Meth)acrylats mit einer Hydroxylgruppe und einem Mol eines Diisocyanats.

In einem bevorzugten erfindungsgemäßen härtbaren Gemisch enthält Bestandteil c.) ein oder mehrere (Meth)acrylat-Monomere gewählt aus der Gruppe bestehend aus 3(4), 8(9)-Bis((meth)acryloyloxymethyl)tricyclo[5.2.1.$0^{2,6}$]decan, alkoxyliertem 3(4), 8(9)-Bis((meth)acryloyloxymethyl)tricyclo[5.2.1.$0^{2,6}$]decan, 2,3-Bis((meth)acryloyloxymethyl)-bicyclo[2.2.1]heptan, alkoxyliertem 2,3-Bis((meth)acryloyloxymethyl)bicyclo[2.2.1]heptan, 1,3,5-Tri(meth)acryloyloxytricyclo[3.3.1.$1^{3,7}$]decan, alkoxyliertem Tri(meth)acryloyloxytricyclo-[3.3.1.$1^{3,7}$]decan, (Meth)acrylsäureester von Tricyclo[5.2.1.$0^{2,6}$]decan-3(4),8(9)-dimethanol, alkoxyliertem Tricyclo[5.2.1.$0^{2,6}$]decan-3(4),8(9)-dimethanol, Bicyclo[2.2.1]heptan-2,3-dimethanol, alkoxyliertem Bicyclo[2.2.1]heptan-2,3-dimethanol, 1,3,5-Adamantantriol, alkoxyliertem 1,3,5-Adamantantriol, wobei zwischen dem polyalicyclischen Strukturelement und den (Meth)acrylsäureestern Urethan-, Harnstoff-, Amid-, Allophanat-, Acylharnstoff- oder Biuretgruppen angeordnet sind, Ethylenglykoldi(meth)acrylat, 1,6-Hexandioldi(meth)acrylat (HEDMA), Triethylenglykoldi(meth)acrylat (TEGDMA), 1,12-Dodecandioldi(meth)acrylat, Bisphenol-A-di(meth)acrylat, alkoxyliertes Bisphenol-A-di(meth)acrylat, Bisphenol-B-di(meth)acrylat, alkoxyliertes Bisphenol-B-di(meth)acrylat, Bisphenol-C-di(meth)acrylat, alkoxyliertes Bisphenol-C-di(meth)acrylat, Bisphenol-F-di(meth)acrylat, alkoxyliertes Bisphenol-F-di(meth)acrylat, Polyethylenglykoldi(meth)acrylat, 7,7,9-Trimethyl-4,13-dioxo-5,12-diazahexadecan-1,16-dioxydi(meth)acrylat (UDMA), Butandioldi(meth)acrylat, Tetraethylenglykoldi(meth)acrylat, Neopentylglykoldi(meth)acrylat, 2-Hydroxypropyl-1,3-di(meth)acrylat, 3-Hydroxypropyl-1,2-di(meth)acrylat, Pentaerythritoldi(meth)acrylat, Di(meth)acrylaten des Dihydroxymethyltricyclo[5.2.1,$0^{2,6}$]decans, 2-Hydroxyethyl(meth)acrylat, 2-Hydroxypropyl(meth)acrylat, 3-Hydroxypropyl(meth)acrylat, 1,2-Dihydroxypropyl(meth)acrylat, 1,3-Dihydroxypropyl(meth)acrylat, 2,2-Bis[4-[3-(meth)acryloyloxy-2-hydroxypropoxy]phenyl]propan (Bis-GMA), Trimethylolpropantri(meth)acrylat, Trimethylolethantri(meth)acrylat, Pentaerythritoltri(meth)- acrylat, Trimethylolmethantri(meth)acrylat, Pentaerythritoltetra(meth)acrylat, Ditrimethylol-propantetra(meth)acrylat, Pentaerythritolhexa(meth)acrylat, Butylenglykoldi(meth)acrylat, Propylenglykoldi(meth)acrylat, Nonandioldi(meth)acrylat, Decandioldi(meth)acrylat, Glycerolmono(meth)acrylat, Glyceroldi(meth)acrylat, Trimethylolpropanmono(meth)acrylat,

Trimethylolpropandi(meth)acrylat, Sorbitolmono-, di-, tri-, tetra- oder penta(meth)acrylat, Methyl(meth)acrylat, Ethyl(meth)acrylat, Propyl(meth)acrylat, Butyl(meth)acrylat, Hexyl(meth)acrylat, Tetrahydrofurfuryl(meth)acrylat, Lauryl(meth)acrylat, Cyclohexyl(meth)-acrylat, Allyl(meth)acrylat, Glycidyl(meth)acrylat, 2-Ethoxyethyl(meth)acrylat, Methoxypoly-ethylenglykol(meth)acrylat, Isobornyl(meth)acrylat, 2-(N,N-Dimethylamino)ethyl(meth)acrylat, N-Methylol(meth)acrylamid, Diaceton(meth)acrylamide, 2,2-Bis[4-(meth)acryloyloxyphenyl]-propan, 2,2-Bis[4-(meth)acryloyloxyethoxyphenyl]propan, 2,2-Bis[4-(meth)acryloyloxydiethoxyphenyl]propan, 2,2-Bis[4-(meth)acryloyloxytriethoxyphenyl]propan 2,2-Bis[4-(meth)acryloyloxytetraethoxyphenyl]propan, 2,2-Bis[4-(meth)acryloyloxypentaethoxyphenyl]-propan, 2,2-Bis[4-(meth)acryloyloxydipropoxyphenyl]propan, 2,2-Bis[4-(meth)acryloyloxy-ethoxyphenyl]-2-[4-(meth)acryloyloxydiethoxyphenyl]propan, 2-[4-(Meth)acryloyloxydiethoxy-phenyl]-2-[4-(meth)acryloyloxytriethoxyphenyl]propan, 2-[4-(Meth)acryloyloxdipropoxy-phenyl]-2-[4-(meth)acryloyloxytriethoxyphenyl]propan, 2,2-Bis[4-(meth)acryloyloxyisopropoxy phenyl]propan, Hydroxypivalinsäureneopentylglykoldi(meth)acrylat, Acetoacetoxyethyl-(meth)acrylat, Polypropylenglykoldi(meth)acrylat, Glycerolalkoxylatdimethacrylat, Neopentylglykol(meth)acrylat, N,N-(1,2-Dihydroxyethylen)bisacrylamid, 2,2-Bis[4-(meth)acryloyloxypentaethoxyphenyl]propan, 2,2-Bis[4-(meth)acryloyloxypolyethoxyphenyl]-propan, Diethylenglykoldi(meth)acrylat, Dipentaerythritoltetra(meth)acrylat, Dipentaerythritolhexa(meth)acrylat, N,N-(2,2,4-Trimethylhexamethylen)bis[2-(aminocarboxy)propan-1,3-diol]-tetra(meth)acrylat, das Kondensationsprodukt von 3, (4)- (Meth)acryloxymethyl-8, (9) - hydroxymethyltricyclo- [5.2.1.0$^{2,6}$]decan mit Dicarbonsäuren, 2-Ethylhexyl(meth)acrylat, Tridecyl(meth)acrylat, Stearyl(meth)acrylat, Benzyl(meth)acrylat, Methoxydiethylenglykol(meth)acrylat, Dicyclopentenyl(meth)acrylat, Phenyl(meth)acrylat, Pentaerythritolmono(meth)acrylat, Dipentaerythritolmono(meth)acrylat, Caprolacton modifiziertes Tetrahydrofurfuryl(meth)acrylat.

Bestandteil d.) - Füllstoffe

Eine erfindungsgemäße radikalisch härtbare dentale Dentalzusammensetzung enthält einen Anteil an Füllstoffpartikeln von 85 Gew.-% oder weniger, vorzugsweise von 78 Gew.-% oder weniger, bezogen auf die Gesamtmasse der erfindungsgemäßen Dentalzusammensetzung.

Als Bestandteil d.) können organische und/oder anorganische Füllstoffe eingesetzt werden.

Organische Füllstoffpartikel umfassen oder bestehen aus beispielsweise ein oder mehr Verbindungen ausgewählt aus der Gruppe bestehend aus Polyvinylacetat und Copolymere des Polyvinylacetats mit einer oder mehreren polymerisierbaren Verbindungen, Polystyrol, Polyethylen, Polypropylen, Wachse wie Polyethylenwachs, Polybutylen, Polybutadien, Copolymere des Butadiens und des Styrols, Polyacrylnitril, Harze wie Kolophoniumharz oder Kohlenwasserstoffharze, Poly(meth)acrylatester, d.h. Umsetzungsprodukte von Poly(meth)acrylsäure mit linearen oder verzweigten aliphatischen, aromatischen oder cycloaliphatischen Alkoholen wie Methanol, Ethanol, Propanol, Isopropanol, den isomeren Butanolen und höheren Homologen der genannten Alkohole mit bis zu 22 Kohlenstoffatomen, Cyclohexanol, Benzylalkohol und dergleichen, Polydialkylmaleinate wie Dibutylmaleinat und deren Copolymere und Silylgruppen-haltige Polymere wie Polyvinylsilane oder Copolymere von Vinylsilan mit einem oder mehreren der genannten Monomeren. Die organischen Füllstoffe können alleine oder als Mischungen eingesetzt werden.

Die anorganischen Füllstoffe können ebenfalls allein oder als Mischungen eingesetzt werden. Zur Optimierung der Produkteigenschaften können die anorganischen Füllstoffe in unterschiedlichen Korngrößen in die Rezepturen eingebracht werden. Die Füllstoffe können eine unimodale oder polymodale, beispielsweise eine bimodale Verteilung aufweisen.

Als anorganische Füllstoffe können kompakte Gläser und unterschiedliche Kieselsäuren in verschiedenen Größen und Zuständen (monodispers, polydispers) zum Einsatz kommen.

Geeignete anorganische Bestandteile sind beispielsweise amorphe Materialien auf der Basis von Mischoxiden aus $SiO_2$, $ZrO_2$ und/oder $TiO_2$ sowie Füllstoffe wie Quarz-Glaskeramik oder Glaspulver, Bariumsilikatgläser, Bariumfluorsilikatgläser, Strontiumsilikatgläser, Strontiumborsilikat, Li/Al-Silikatgläser, Bariumgläser, Calciumsilikate, Natriumaluminiumsilikate, Fluoraluminiumsilikatgläser, Oxide von Aluminium oder Silicium, Zeolithe, Apatit, Zirkonsilikate, schwerlösliche Metallsalze wie Bariumsulfat oder Calciumfluorid sowie röntgenopake Füllstoffe wie Ytterbiumfluorid.

Zum besseren Einbau in die Polymermatrix können die Füllstoffe organisch oberflächenmodifiziert sein. Beispielhaft genannt sei die Oberflächenbehandlung der Füllstoffe mit einem Silan. Als Haftvermittler eignet sich besonders das Methacryloxypropyltrimethoxysilan.

Zur Einstellung der Rheologie können die radikalisch härtbaren dentalen Zusammensetzungen unterschiedliche Kieselsäuren, bevorzugt pyrogene Kieselsäuren, enthalten.

Bevorzugt enthalten die erfindungsgemäßen härtbaren Zusammensetzungen nanoskalige Feststoffteilchen. Bei den nanoskaligen Feststoffteilchen handelt es sich um Partikel mit einer mittleren Teilchengröße von nicht mehr als 200 nm, bevorzugt nicht mehr als 100 nm und insbesondere nicht mehr als 70 nm. Die nanoskaligen anorganischen Feststoffteilchen sind bevorzugt solche von Oxiden, Sulfiden, Seleniden und Telluriden von Metallen, Mischmetallen und deren Mischungen. Besonders bevorzugt sind nanoskalige Teilchen von $SiO_2$, $TiO_2$, $ZrO_2$, ZnO, $SnO_2$ und $Al_2O_3$ und deren Mischungen. Die Herstellung der nanoskaligen Feststoffteilchen erfolgt auf bekannte Weise, z.B. durch Flammpyrolyse, Plasmaverfahren, Gasphasenkondensation, Kolloidtechniken, Präzipitationsverfahren, Sol-Gel Verfahren, etc.

In einer bevorzugten Ausgestaltung liegen die nanoskaligen Teilchen in nicht agglomerierter und/oder nicht aggregierter Form vor, beispielsweise dispergiert in einem Medium, vorzugsweise in monodisperser Form.

Um eine gute Einbindung der Nanopartikel in die Polymermatrix einer erfindungsgemäßen radikalisch härtbaren dentalen Zusammensetzung zu ermöglichen, sind die Oberflächen der Nanopartikel ebenfalls organisch oberflächenmodifiziert, d.h. ihre Oberflächen weisen organische Strukturelemente auf. Beispielhaft genannt sei die Oberflächenbehandlung der Füllstoffe mit einem Silan. Als Haftvermittler eignet sich auch hier besonders das Methacryloxypropyltrimethoxysilan.

In einer weiteren bevorzugten Ausgestaltung sind die nanoskaligen Teilchen somit nicht agglomerierte und/oder

nicht aggregierte, organisch oberflächenmodifizierte Nanopartikel mit einer mittleren Partikelgröße kleiner 200 nm, vorzugsweise kleiner 100 nm, besonders bevorzugt kleiner 70 nm), die wiederum bevorzugt silanisiert sind.

Kommerziell erhältliche nanoskalige, nicht-agglomerierte und nicht aggregierte Kieselsole, die erfindungsgemäß eingesetzt werden können, sind beispielsweise unter der Bezeichnung "NALCO COLLOIDAL SILICAS" (Nalco Chemical Co.), "Ludox colloidal silica" (Grace) oder "Highlink OG (Clariant) im Handel.

In einer bevorzugten Ausgestaltung umfasst der Füllstoffanteil einer erfindungsgemäßen radikalisch härtbaren dentalen Zusammensetzung eine Mischung eines ersten Füllstoffs d.)1. in Form nicht agglomerierter, nicht aggregierter organisch oberflächenmodifizierter Nanopartikel mit einer mittleren Partikelgröße kleiner 200 nm und eines zweiten Füllstoffs d.)2. in Form von Mikropartikeln mit einer mittleren Partikelgröße im Bereich von 0,4 $\mu$m bis 10 $\mu$m. Durch die Kombination von d.)1. Nanopartikeln und d.)2. Mikropartikeln in einer erfindungsgemäßen radikalisch härtbaren dentalen Zusammensetzung wird eine vollständige und gleichmäßige Volumenfüllung des Kompositmaterials erzielt. Dadurch wird sowohl die Schrumpfung der radikalisch härtbaren Zusammensetzung beim Aushärten der Polymermatrix als auch die Empfindlichkeit der erfindungsgemäßen Zusammensetzung gegen Abrasion vermindert.

Der Anteil organisch oberflächenmodifizierter Nanopartikel in einer bevorzugten erfindungsgemäßen radikalisch härtbaren dentalen Zusammensetzung mit einer mittleren Partikelgröße kleiner 200 nm ist größer als 1 Gew.-%, vorzugsweise größer als 2 Gew.-% und besonders bevorzugt größer als 3 Gew.-%. In eigenen Untersuchungen hat sich gezeigt, dass bei einem Gehalt von 1 Gew.- % oder weniger an nicht agglomerierten und/oder nicht aggregierten, organisch oberflächenmodifizierten Nanopartikeln mit einer mittleren Partikelgröße kleiner 200 nm die radikalisch härtbare dentale Zusammensetzung im Einzelfall nicht mehr ausreichend abriebfest ist. Dies ist wahrscheinlich u.a. darauf zurückzuführen, dass bei einem Gehalt von 1 Gew.- % oder weniger an den besagten Nanopartikeln die Bereiche zwischen den Mikropartikeln mit einer mittleren Partikelgröße von 0,4 $\mu$m bis 10 $\mu$m nicht mehr ausreichend gefüllt sind. Andererseits hat sich gezeigt, dass bei einem Gehalt von mehr als 20 Gew.- % an nicht agglomerierten und/oder aggregierten, organisch oberflächenmodifizierten Nanopartikeln mit einer mittleren Partikelgröße kleiner 200 nm die Verarbeitbarkeit der Zusammensetzung nicht mehr ausreichend ist. Aufgrund des hohen Feststoffgehalts wird dann seine Viskosität zu hoch.

Die Materialien für die erfindungsgemäß einzusetzenden Nanopartikel sind vorzugsweise Oxide oder Mischoxide und bevorzugt ausgewählt aus der Gruppe bestehend aus Oxiden und Mischoxiden der Elemente Silizium, Titan, Yttrium, Strontium, Barium, Zirkonium, Hafnium, Niob, Tantal, Wolfram, Wismut, Molybdän, Zinn, Zink, Ytterbium, Lanthan, Cer, Aluminium und deren Mischungen. Die bevorzugten oxidischen Nanopartikel sind dabei, wie dargelegt, nicht agglomeriert und/oder nicht aggregiert sowie organisch oberflächenbehandelt.

Innerhalb einer erfindungsgemäßen radikalisch härtbaren dentalen Zusammensetzung bewirken die Mikropartikel eine weitgehende gleichmäßige Füllung des Volumens, wobei die verbleibenden Hohlräume zwischen den Mikropartikeln durch die oben beschriebenen Nanopartikel (Komponente d.)2.) zumindest teilweise gefüllt werden. Unter Mikropartikeln werden in Zusammenhang mit der vorliegenden Erfindung Partikel mit einer mittleren Partikelgröße von 400 nm bis 10 $\mu$m verstanden. Vorzugsweise ist die mittlere Partikelgröße kleiner als 5 $\mu$m. Es hat sich gezeigt, dass die mit den Mikropartikeln bereits erreichbare Volumenfüllung der radikalisch härtbaren dentalen Zusammensetzung umso vollständiger und gleichmäßiger ist, je kleiner die Mikropartikel sind.

Die Mikropartikel der Komponente d.)2. können eine monomodale oder polymodale, beispielsweise eine bimodale Partikelgrößenverteilung aufweisen. Mikropartikel mit einer bimodalen oder multimodalen Partikelgrößenverteilung sind erfindungsgemäß bevorzugt, da mit ihnen eine vollständigere Volumenfüllung erreichbar ist als bei allgemeiner Verwendung von Mikropartikeln mit monomodaler Partikelgrößenverteilung. Bei Vorliegen einer bi- oder multimodalen Partikelgrößenverteilung bewirken die Partikel der Fraktionen mit der größeren Partikelgröße eine grobe Ausfüllung des Volumens, während die Partikel der Fraktion mit der kleineren Partikelgröße soweit möglich die Bereiche zwischen den Partikeln der Fraktionen mit der größeren Partikelgröße ausfüllen werden. Die noch verbleibenden Hohlräume werden wie oben beschrieben durch Nanopartikel gefüllt.

Ganz besonders bevorzugt wird somit in einer erfindungsgemäßen radikalisch härtbaren dentalen Zusammensetzung eine Komponente d.)2. eingesetzt, welche zwei oder mehr Fraktionen von Mikropartikeln enthält, wobei sich die mittleren Partikelgrößen der Fraktionen unterscheiden.

Vorzugsweise enthält Komponente d.)2. zumindest zwei Mikropartikel-Fraktionen, wobei deren mittlere Partikelgrößen um mindestens 0,5 $\mu$m, bevorzugt um mindestens 0,7 $\mu$m, voneinander abweichen. In manchen Ausgestaltungen beträgt die Differenz der mittleren Partikelgrößen der Mikropartikel-Fraktionen mindestens 1,0 $\mu$m.

Die Mikropartikel verschiedener Fraktionen können aus dem gleichen oder aus verschiedenen Materialien bestehen; es können dabei auch mehrere Fraktionen von Mikropartikeln vorliegen, deren mittlere Partikelgröße annähernd gleich ist oder in einem bestimmten Bereich liegt, wobei die Materialien der Partikel sich zwischen den Fraktionen unterscheiden.

Besonders bevorzugt umfasst eine erfindungsgemäße radikalisch härtbare dentale Zusammensetzung eine Komponente d.)2., welche eine oder mehrere erste Mikropartikelfraktionen, die jeweils eine mittlere Partikelgröße im Bereich von 1 $\mu$m bis 10 $\mu$m, vorzugsweise 1 $\mu$m bis 5 $\mu$m, besitzen, und eine oder mehrere zweite Mikropartikelfraktionen, die jeweils eine mittlere Partikelgröße im Bereich von > 0,4 μm bis < 1 μm (d.h. größer als 0,4 μm, aber kleiner als 1 μm), vorzugsweise 0,5 μm bis 0,8 μm, besitzen.

Bevorzugt liegt das Verhältnis der Gesamtmasse der ersten Mikropartikelfraktionen zur Gesamtmasse der zweiten Mikropartikelfraktionen im Bereich von 1 : 1 bis 12 : 1, vorzugsweise im Bereich von 1,5 : 1 bis 8 : 1.

Bevorzugt liegt das Verhältnis der mittleren Korngröße der oder einer ersten Mikropartikelfraktion zur mittleren Korngröße der oder einer zweiten Mikropartikelfraktion der Komponente d.)2. im Bereich von 1,5 : 1 bis 10 : 1, vorzugsweise im Bereich von 2 : 1 bis 5 : 1.

In einer besonders bevorzugten erfindungsgemäßen radikalisch härtbaren dentalen Zusammensetzung umfasst die Komponente d.)2. eine oder mehrere erste Mikropartikelfraktionen, die jeweils eine mittlere Partikelgröße im Bereich von 1 μm bis 10 μm, vorzugsweise 1 μm bis 5 μm, besitzen, und eine oder mehrere zweite Mikropartikelfraktionen, die jeweils eine mittlere Partikelgröße im Bereich von > 0,4 μm bis < 1 μm, vorzugsweise 0,5 μm bis 0,8 μm, besitzen; wobei das Verhältnis der Gesamtmasse der ersten Mikropartikelfraktionen zur Gesamtmasse der zweiten Mikropartikelfraktionen im Bereich von 1 : 1 bis 12 : 1, vorzugsweise 1,5 : 1 bis 8 : 1 und/oder das Verhältnis der mittleren Korngröße der oder einer ersten Mikropartikelfraktion zur mittleren Korngröße der oder einer zweiten Mikropartikelfraktion der Komponente d.)2. im Bereich von 1,5 : 1 bis 10 : 1, vorzugsweise 2 : 1 bis 5 : 1 liegt.

In einer besonders bevorzugten erfindungsgemäßen radikalisch härtbaren dentalen Zusammensetzung wird zumindest ein Teil der Mikropartikel der Komponente d.)2. durch organisch oberflächenmodifizierte Partikel, vorzugsweise silanisierte Partikel gebildet und/oder es wird zumindest ein Teil der Mikropartikel der Komponente d.)2. durch Dentalglas-Partikel gebildet; vorzugsweise sind zumindest ein Teil der Mikropartikel der Komponente d.)2. organisch oberflächenmodifizierte Dentalglas-Partikel, vorzugsweise silanisierte Dentalglas-Partikel.

Bevorzugt zeichnet sich in diesen Fällen Komponente d.)2. durch eine bi- oder multimodale Partikelgrößenverteilung aus, insbesondere eine bi- oder multimodale Partikelgrößenverteilung mit den oben beschriebenen bevorzugten Merkmalen.

Neben den Komponenten d.)1. und d.)2. kann die radikalisch härtbare dentale Zusammensetzung zu der Mischung von Füllstoffpartikeln zusätzlich weitere Füllstoffe als Komponente d.)3. umfassen.

So können z.B. verstärkend wirkende Füllstoff-Materialien, wie Glasfasern, Polyamid- oder Kohlenstofffasern eingesetzt werden. Eine erfindungsgemäße radikalisch härtbare dentale Zusammensetzung kann auch feinteilige Splitter oder Perlpolymerisate enthalten, wobei die Perlpolymerisate Homo- oder Copolymere organischer härtbarer Monomerer sein können.

In einer besonders bevorzugten Ausführungsform enthält eine erfindungsgemäße radikalisch härtbare dentale Zusammensetzung einen röntgenopaken Füllstoff. Ganz besonders bevorzugt enthält die erfindungsgemäße Zusammensetzung nanoskaliges $YbF_3$ und/oder $BaSO_4$.

Qualitative und quantitative Charakterisierung der Füllstoffpartikel:

Die nachfolgend beschriebenen Schritte in der qualitativen und quantitativen Charakterisierung der Füllstoffpartikel (insbesondere von nanoskaligen Füllstoffpartikeln) sind dem Fachmann gut bekannt und in der Literatur umfassend beschrieben.

Harz-/Füllstofftrennung:

In einem ersten Schritt werden 1 g einer erfindungsgemäßen radikalisch härtbaren dentalen Zusammensetzung (nachfolgend auch Kompositmaterial genannt) in 10 ml Aceton resuspendiert und die erhaltene Suspension anschließend mit einer Zentrifuge für 10 min bei 5000 U/min zentrifugiert. Der Überstand (nachfolgend Harzphase genannt) wird in ein Sammelglas abdekantiert und der Rückstand in 5 ml Aceton aufgeschlämmt. Es wird erneut für 10 min bei 5000 U/min zentrifugiert, dekantiert und der Rückstand in 5 ml Aceton erneut aufgeschlämmt. Die Schritte Zentrifugieren, Dekantieren und Aufschlämmen werden noch zweimal unter identischen Bedingungen wiederholt. Die von den Harzphasen getrennte Gesamtmenge an Rückständen wird getrocknet und die Gesamtmenge an Harzphasen am Rotationsverdampfer von Aceton befreit.

Nach Durchführung des ersten Schrittes umfasst die getrocknete Gesamtmenge an Rückständen regelmäßig solche Füllstoffpartikel, die eine Partikelgröße von ca. 400 nm oder größer 400 nm besitzen (nachfolgend makroskopische Füllstoffpartikel genannt). Die von Aceton befreite Gesamtmenge an Harzphasen (nachfolgend Harzanteil genannt) umfasst neben polymerisierbaren Monomeren außerdem regelmäßig Füllstoffpartikel mit einer Partikelgröße von ca. 400 nm oder insbesondere kleiner 400 nm (nachfolgend nanoskalige Teilchen genannt). Dieses Verfahren stellt somit sicher, dass das dentale Kompositmaterial durch die Zentrifugation in (i) einen Anteil makroskopischer Füllstoffpartikel, wobei speziell die dentalen Gläser im Größenordnungsbereich von größer 400 nm bis in den hohen Mikrometerbereich betroffen sind, und (ii) einen Harzanteil umfassend nanoskaligen Teilchen vollständig aufgetrennt wird.

Die mittlere Partikelgröße $d_{50}$ der erfindungsgemäß einzusetzenden makroskopischen Füllstoffpartikel der

Füllstoffkomponente (d2) einer erfindungsgemäßen Zusammensetzung wird mittels Lichtstreuung (Laserbeugung) bestimmt, vorzugsweise mit einem Partikelgrößenmessgerät Beckman Coulter LS 13320.

Die nanoskaligen Teilchen, die sich in dem Harzanteil befinden, können beispielsweise sowohl nicht-aggregierte und/oder nicht agglomerierte, beispielsweise auch röntgenopake Partikel sein, beispielsweise $YbF_3$ oder $BaSO_4$ mit Partikelgrößen in einem Bereich von ca. 3 nm bis 200 nm, vorzugsweise von 5 nm bis 200 nm, besonders bevorzugt von 7 nm bis 100 nm und ganz besonders bevorzugt von 7 nm bis 70 nm aufweisen, als auch nicht-röntgenopake Kieselsäuren, die beispielsweise als pyrogene Kieselsäuren in Form von Aggregaten und/oder Agglomeraten mit einer Partikelgröße in einem Bereich von ca. 150 nm bis ca. 300 nm vorliegen oder auch Kieselsäuren, die nach dem Sol-Gel Verfahren (oder auch aus Wasserglas) synthetisiert werden und die ebenfalls nicht-aggregiert und/oder nicht-agglomeriert vorliegen und Partikelgrößen in einem Bereich von ca. 3 nm bis 200 nm, vorzugsweise von 5 nm bis 200 nm, besonders bevorzugt von 7 nm bis 100 nm und ganz besonders bevorzugt von 7 nm bis 70 nm aufweisen.

Der Gesamtmassenanteil anorganischer Partikel in dem Harzanteil wird durch Differenzwiegung nach Veraschung eines entsprechenden Harzanteils gravimetrisch bestimmt.

TEM in Kombination mit EELS:

In einem zweiten Schritt werden die Füllstoffpartikel in dem Harzanteil einer qualitativen und quantitativen Charakterisierung unterzogen. Hierzu wird TEM (Transmissionselektronenmikroskopie) in Verbindung mit EELS (Elektronenenergieverlustspektroskopie) eingesetzt.

Mittels TEM werden die Partikelgrößen der einzelnen Partikel sowie deren Anzahl ermittelt; eine Elementarbestimmung einzelner Partikel erfolgt mittels EELS.

Zur Durchführung der kombinierten TEM/EELS-Charakterisierung wird in einem ersten Schritt durch Verdünnung mit härtbarem Harz die Konzentration der nanoskaligen Teilchen im Harzanteil zunächst reduziert. Dadurch wird weitestgehend ausgeschlossen, dass eine "Überlagerung" von nanoskaligen Teilchen in den späteren Bildern beobachtet wird. Eine solche "Überlagerung" würde die Partikelcharakterisierung verfälschen. Eigene Untersuchungen haben gezeigt, dass die optimale Partikelkonzentration (d.h. der Volumenanteil der Füllstoffpartikel) für solche Untersuchungen bei 1 Vol. % liegt, bezogen auf die Gesamtmasse der verdünnten Probe.

Aus den durch Verdünnung mit härtbarem Harz gewonnenen verdünnten Harzanteilen werden in einem zweiten Schritt durch Aushärtung Stäbchen hergestellt. Aus diesen Stäbchen werden anschließend mit einem Ultradiamantmesser (beispielsweise Ultramikrotom ULTRCAT UCT, LEICA, Wetzlar) mehrere 300 nm dünne Ultradünnschnitte angefertigt. Die Ultradünnschnitte werden zur Stabilisierung auf Kupfer-TEM-Grids transferiert. Es resultieren Dünnschnittpräparate. Diese Dünnschnittpräparate werden dann mit 120 kV Beschleunigungsspannung in einem TEM mittels Hellfeld-Abbildungen untersucht.

Eine TEM-Untersuchung an den vorstehend beschriebenen Dünnschichtpräparaten erlaubt es, nicht aggregierte und nicht agglomerierte nanoskalige Partikel von aggregierten und/oder agglomerierten Partikeln (z.B. Kieselsäuren, wie beispielsweise Aerosilen), zu unterscheiden (zur Identifizierung der chemischen Zusammensetzung siehe die nachfolgenden Ausführungen).

Sofern hochauflösende Abbildungen untersucht werden sollen, können Ultradünnschnitte mit Schichtdicken kleiner 100 nm hergestellt und untersucht werden.

In einem dritten Schritt werden die Füllstoffpartikel in den Ultradünnschnitten bzw. Dünnschnittpräparaten mittels EELS-Punktanalysen chemisch charakterisiert, so dass die chemische Zusammensetzung einzelner Partikel bekannt wird (zur Bestimmung der Oberflächenmodifikation von Partikeln siehe nachfolgende Punkte).

Die volumen- oder gewichtsbezogenen Anteile von (ggf. auch mehrerer) Partikelfraktionen werden in einem vierten Schritt wie folgt aus einer TEM Aufnahme ermittelt: Der im Mikroskop betrachtete Bildausschnitt einer TEM-Aufnahme stellt eine Fläche dar, deren Kantenlängen a und b mittels der Legende bestimmt werden. Multipliziert mit der Dicke c des Ultradünnschnittes ergibt sich ein Gesamtvolumen $V_{Gesamt}$ für den in der TEM betrachteten Bereich. Dieses Gesamtvolumen $V_{Gesamt}$ ist die Summe aus dem Harzvolumen $V_{Harz}$ und dem Volumen aller Partikel $V_{Partikel}$ innerhalb dieses Volumens (das Volumen aller Partikel umfasst ggf. mehrere Gruppen von Partikeln, z.B. sortiert nach verschiedenen Kriterien wie z.B. der Größe). Es gilt $V_{Gesamt} = a * b * c = V_{Harz} + V_{Partikel}$.

Das Volumen einzelner Partikel (und damit das Volumen aller Partikel in dem betrachteten Volumen) ist rechnerisch zugänglich über das Kugelvolumen der einzelnen Partikel. Hierzu wird in der TEM-Aufnahme der Durchmesser bzw. Radius eines entsprechenden Partikels bestimmt. Das daraus errechnete Kugelvolumen, multipliziert mit der Dichte des entsprechenden Materials, aus dem das Partikel besteht (Material identifizierbar mittels EELS), ergibt die Masse des Partikels. Das Harzvolumen, zugänglich aus dem Gesamtvolumen minus dem Partikelvolumen, multipliziert mit der Harzdichte, ergibt die Harzmasse. Die Harzdichte ergibt sich weitestgehend aus der Dichte des zur Verdünnung eingesetzten Harzes und ggf. der Dichte des verdünnten Harzanteils (letztere kann ggf. bei der Berechnung der Harzdichte vernachlässigt werden, wenn der Anteil des verdünnten Harzes vernachlässigbar ist). Der Anteil der Partikel (oder einer Gruppe von Partikeln) in Gewichtsprozent errechnet sich aus $m_P*100/(m_{Partikel}+m_{Harz})$, wobei $m_P$ die Masse der betrachteten Partikelfraktion in dem betrachteten Volumen, $m_{Partikel}$ die Masse aller Partikel im betrachteten Volumen und $m_{Harz}$ die Masse des Harzes in dem betrachteten Volumen bedeutet. Bei der abschließenden Berechnung des Gewichtsanteils der zu betrachtenden Partikelfraktion wird der Verdünnungsfaktor entsprechend berücksichtigt.

Bestimmung organischer Oberflächenmodifikationen:

Vorabbetrachtung:

Viele bekannte röntgenopake Füllstoffmaterialien (wie z.B. Ytterbiumfluorid oder Bariumsulfat) weisen den Nachteil auf, dass sie nur schwer in die Matrix (Harzmatrix) aus polymerisierbaren Monomeren (die sogenannte organische Harzphase) einzuarbeiten sind, weil sie keine hinreichenden chemischen Bindungen (Anbindungsmöglichkeiten) mit den hydrophoben Gruppen des Mediums eingehen. Glasartige Füllstoffe lassen sich beispielsweise mit Hilfe der Silanisierung über Si-OH Gruppen hervorragend in die Harzmatrix dentaler Kompositmaterialien einarbeiten. Bei Ytterbiumfluorid und Bariumsulfat sind solche Gruppen auf den Oberflächen nicht vorhanden; sie sind somit nicht silanisierbar und führen in einem gehärteten Dentalmaterial zu einer unzureichenden physikalischen und chemischen Resistenz (siehe hierzu WO 2005/011621 A1, Seite 2 unten).

Die in einem erfindungsgemäßen härtbaren Dentalmaterial eingesetzten röntgenopaken nanoskaligen Teilchen werden somit auf ihren Oberflächen keine Silane aufweisen. Vielmehr erfolgt die Verknüpfung über Stickstoff-, Sauerstoff-, Schwefel- und/oder Phosphoratomen (siehe hierzu wiederum WO 2005/011621 A1 sowie unsere Ausführungen weiter oben im Text).

Abtrennung polymerisierbarer Monomere von nanoskaligen Teilchen:

"Cross-Flow"-Verfahren:

Die Abtrennung polymerisierbarer Monomere von nanoskaligen Teilchen erfolgt beispielsweise in einem dem Fachmann bekannten "Cross-Flow"-Verfahren mittels Ultrafiltrationsmembranen.

Hierbei wird ein Harzanteil enthaltend nanoskalige Teilchen, polymerisierbare Monomere und ggf. ein geeignetes Verdünnungsmittel aus einem Behältnis mittels einer Pumpe in einen Kreislauf aus bestimmten Membranen gepumpt, wobei die polymerisierbaren Monomere die Poren der Membranen passieren und als Filtrat separiert werden, während die nanoskaligen Teilchen innerhalb des Kreislaufs (und damit im Behältnis) verbleiben.

Für diesen Trennungsgang ist beispielsweise das System "Vivaflow 50" von "Sartorius Stedim Biotech GmbH, Göttingen" geeignet. Pumpenantrieb (7554-95) und Pumpenkopf entstammen der Reihe "Masterflex L/S" von "Cole-Palmer Instrument Co.", Illinois, USA. Der Betrieb der Pumpe wird während der Filtration auf 2,5 bar eingestellt. Zwei Trennmembranen des Typs "50,000 MWCO (PES)" werden hintereinander geschaltet. Der MWCO (Molecular Weight Cut Off) gibt hierbei die Trenngrenze an, d.h. die Größe der Moleküle, die noch effizient die Membran passieren können. Dieser Wert wird in Dalton angegeben. Die erhaltenen Fraktionen werden anschließend wie unten beschrieben, untersucht.

Sedimentations-Feld-Fluss-Fraktionierung (SF3):

Besser noch als das "Cross-Flow"-Verfahren ist die Durchführung einer Sedimentations-Feld-Fluss-Fraktionierung (SF3). Dabei lassen sich insbesondere unterschiedliche Partikelfraktionen voneinander und zusätzlich vom Harzanteil trennen. Voraussetzung hierbei ist, dass sich die unterschiedlichen Partikelfraktionen ausreichend in Größe und/oder Dichte voneinander unterscheiden.

Entsprechende Geräte, die eine hierfür notwendige Trennsäule enthalten, sind bei der Firma Postnova Analytics GmbH, Landsberg, erhältlich. Das die Trennsäule enthaltende Modul trägt die Bezeichnung CF2000 Centrifugal FFF und wird durch die weiteren Module PN7140 (Eluent Organizer), PN1130 (Isocratic Pump), PN5300 (Autosampler), PN3621 MALS (21-Multi-Angle Light Scattering Detector) und PN8050 (Fraction Collector) ergänzt. In dieser Kombination erlaubt die Centrifugal FFF-Anlage nicht nur die analytische, sondern auch die präparative Trennung von Partikelfraktionen. Die erhaltenen Fraktionen werden anschließen wie unten beschrieben, untersucht.

Charakterisierung der Oberflächenmodifikation:

Eine wie oben hergestellte und anschließend von Lösungsmitteln befreite Probe, die nanoskalige Teilchen in Form eines Pulvers enthält, wird anschließend mittels spektroskopischer Verfahren untersucht (z.B. mittels 1H-NMR, 13C-NMR, 15N-NMR, 29Si-NMR und 31P-NMR sowie IR).

Signale, die sich nicht einem Silan, beispielsweise dem gamma-Methacryloxypropylsilylrest, zuordnen lassen,

werden organischen Oberflächenmodifikationen zugeordnet, die nicht auf Silanen basieren, z.B. Oberflächenmodifikationen mittels organischer Verbindungen auf Oberflächen von Ytterbiumfluorid bzw. Bariumsulfatpartikeln.

Die Anteile organisch oberflächenmodifizierter Partikel bzw. nicht organisch oberflächenmodifizierter Partikel können regelmäßig auch durch Auswertung der Intensitäten entsprechender Schwingungsbanden im IR-Spektrum bestimmt werden. Hierzu werden üblicherweise Referenz-Schwingungsbanden (Referenzkurven) organisch oberflächenmodifizierter bzw. nicht organisch oberflächenmodifizierter Partikel mit den entsprechenden chemischen Zusammensetzungen herangezogen.

Charakterisierung mittels Bildanalyse und Ramanspektroskopie:

Dem Fachmann sind zusätzliche Verfahren bzw. Kopplungen von Verfahren bekannt, die eine qualitative und quantitative Charakterisierung der Füllstoffpartikel erlauben. Insoweit sei beispielsweise verwiesen auf den Artikel "Chemische Identität einzelner Partikel" von Deborah Huck-Jones und Renate Hessemann in "Nachrichten aus der Chemie", Volume 62, September 2014, Seiten 886 und 887. Die darin offenbarte Kombination von Bildanalyse und Ramanspektroskopie eignet sich regelmäßig auch zur Charakterisierung der Füllstoffpartikel im Rahmen der vorliegenden Erfindung. Dies gilt insbesondere für Proben, die nach der oben beschriebenen Harzfüllstofftrennung erhalten werden. Eine geeignete Bildanalyse ist z.B. auch die oben im Text beschriebene TEM-Analyse.

<u>Bestandteil e.) - Initiatoren und/oder Katalysatoren für die radikalische Polymerisation</u>

Eine erfindungsgemäße radikalisch härtbare dentale Zusammensetzung ist vorzugsweise lichthärtbar und/oder chemisch härtbar. Bevorzugt ist eine erfindungsgemäße radikalisch härtbare dentale Zusammensetzung wobei Bestandteil e.) einen oder mehrere Lichthärtungsinitiatoren und/oder einen oder mehrere Initiatoren zur chemischen Aushärtung umfasst oder aus diesen besteht.

Bevorzugte erfindungsgemäße radikalisch härtbare dentale Zusammensetzungen sind lichthärtbar (photohärtbar) und umfassen Lichthärtungsinitiatoren. Beispiele für einen Lichthärtungsinitiator schließen Substanzen ein, die nur photosensibilisierend wirken sowie Kombinationen aus Sensibilisator und Beschleuniger.

Beispiele für Photosensibilisatoren sind alpha-Diketone, Benzoinalkylether, Thioxanthone, Benzophenone, Acylphosphinoxide, Acylgermanium-Verbindungen, Acetophenone, Ketale, Titanocene, sensibilisierende Farbstoffe, etc. Die Sensibilisatoren können alleine oder in Kombination angewendet werden. Konkrete Substanzbeispiele der unterschiedlichen Klassen finden sich beispielsweise in der DE 10 2006 019 092 A1, oder in der DE 39 41 629 C2, welche im Wege der Verweisung Bestandteil der vorliegenden Anmeldung sind.

Beispiele für Beschleuniger, die zusammen mit den Sensibilisatoren eingesetzt werden, sind tertiäre Amine, sekundäre Amine, Barbitursäuren, Zinnverbindungen, Aldehyde und Schwefelverbindungen. Konkrete Substanzbeispiele der unterschiedlichen Klassen finden sich in der DE 10 2006 019 092 oder in der DE 39 41 629 C2, welche im Wege der Verweisung Bestandteil der vorliegenden Anmeldung sind.

Weitere geeignete Initiatoren sowie Initiatorkombinationen sind in der DE 601 16 142 beschrieben, welche im Wege der Verweisung Bestandteil der vorliegenden Anmeldung sind.

Die im Rahmen der vorliegenden Erfindung verwendbaren Photoinitiatoren sind dadurch charakterisiert, dass sie durch Absorption von Licht im Wellenlängenbereich von 300 nm bis 700 nm, bevorzugt von 350 nm bis 600 nm und besonders bevorzugt von 380 nm bis 500 nm, gegebenenfalls in Kombination mit einem oder mehreren Coinitiatoren, die Aushärtung einer erfindungsgemäßen radikalisch härtbaren dentalen Zusammensetzung bewirken können.

Das Absorptionsmaximum von Campherchinon (CQ) liegt bei ca. 470 nm und somit im Bereich des blauen Lichts. Campherchinon (CQ) zählt zu den $PI_2$-Initiatoren und wird regelmäßig zusammen mit einem Coinitiator eingesetzt.

Vorzugsweise enthält ein erfindungsgemäßes Kompositmaterial die Kombination eines alpha-Diketons und eines aromatischen tertiären Amins, bevorzugt ist die Kombination von Campherchinon (CQ) und Ethyl-*p*-*N,N*-dimethylaminobenzoat (DABE).

Ebenfalls bevorzugt ist die weitere Kombination des Systems "alpha-Diketon/aromatisches tertiäres Amin" mit einem Phosphinoxid, insbesondere mit dem Phenyl-bis(2,4,6-trimethylbenzoyl)phosphinoxid und/oder dem 2,4,6-Trimethylbenzoyldiphenylphosphinoxid. Bezüglich der Strukturen geeigneter Phosphinoxide zum Einsatz in einer erfindungsgemäßen radikalisch härtbaren dentalen Zusammensetzung wird auf die Druckschriften DE 38 01 511 C2, DE 10 2006 050 153 A1, EP 0 184 095 B1, DE 42 31 579 C2, EP 0 366 977 B1, US 7,081,485 B2, DE 32 36 026 A1, US 2007/0027229 A1, EP 0 262 629 B1, EP 0 073 413, US 7,148,382 B2, US 5,761,169, DE 197 08 294 A1, EP 0 057 474, EP 0 047 902 A, EP 0 007 508, DE 600 29 481 T2, EP 0 980 682 B1, EP 0 948 955 B1, EP 1 236 459 B1 und EP 0 173 567 A2 verwiesen, welche im Wege der Verweisung Bestandteil der vorliegenden Anmeldung sind.

Die in diesen Druckschriften angegebenen Phosphinoxide eigenen sich besonders allein oder in Kombination mit dem System "alpha-Diketon/Amin" als Photopolymerisationsinitiatorsystem in einer erfindungsgemäßen radikalisch härtbaren dentalen Zusammensetzung.

Die EP 1 905 415 beschreibt polymerisierbare Dentalzusammensetzungen mit Acylgermanium-Verbindungen als Initiatoren.

Alternativ können auch Boratsalze, wie sie beispielsweise in US 4,772,530, US 4,954,414, US 4,874,450, US 5,055,372 und US 5,057,393 beschrieben sind, als Photoinitiatoren Verwendung finden, welche im Wege der Verweisung Bestandteil der vorliegenden Anmeldung sind.

Weitere geeignete Photoinitiatoren sind in J.-P. Fouassier, Photoinitiation, Photopolymerization and Photocuring, Hanser Publishers, Munich, Vienna, New York 1995 sowie in J.F. Rabek (Hrsg.), Radiation Curing in Polymer Science and Technology, Vol. II, Elsevier Applied Science, London, New York 1993 beschrieben, welche im Wege der Verweisung Bestandteil der vorliegenden Anmeldung sind.

Dem Fachmann sind diverse Initiatoren für eine chemische Aushärtung bekannt. Es sei insoweit exemplarisch auf die EP 1 720 506 verwiesen. Initiatoren zur chemischen Aushärtung werden auch in den oben bereits erwähnten Druckschriften DE 10 2006 019 092 sowie in der DE 39 41 629 beschrieben.

Bevorzugte Initiatoren zur chemischen Härtung sind Benzoylperoxid, Lauroylperoxid insbesondere Dibenzoylperoxid in Kombination mit Aminen wie N,N-Dimethyl-p-toluidin, N,N-Dihydroxyethyl-p-toluidin sowie strukturverwandten Aminen.

Die Peroxide und die Amine werden dabei auf zwei unterschiedliche Komponenten des Dentalmaterials aufgeteilt. Beim Mischen der aminhaltigen Komponente (sogenannte Basispaste) mit der peroxidhaltigen Komponente (sogenannte Initiator- oder Katalysatorpaste) wird durch die Reaktion von Amin und Peroxid (Redoxreaktion) die radikalische Reaktion initiiert.

Dualhärtende Systeme umfassen eine Kombination aus Photoinitiatoren und Initiatoren zur chemischen Aushärtung.

Beispielsweise kann die Basispaste zusätzlich einen Photoinitiator enthalten, so dass die Basispaste entweder allein als lichthärtender oder zusammen mit der Initiatorpaste als licht- und selbsthärtende dentale Zusammensetzung eingesetzt werden kann.

Neben den oxidativ wirksamen organischen Peroxidverbindungen können als Redoxsysteme auch Barbitursäuren bzw. Barbitursäurederivate sowie Malonylsulfamide verwendet werden.

Von den Barbitursäuresystemen sind die sogenannten "Bredereck-Systeme" von hoher Bedeutung. Beispiele geeigneter "Bredereck-Systeme" sowie Verweise auf die entsprechende Patentliteratur findet man in der EP 1 839 640 sowie in DE 1495520, WO 02/092021 oder in WO 02/092023, welche im Wege der Verweisung Bestandteil der vorliegenden Anmeldung sind.

Anstelle der Barbitursäuren können auch deren Salze verwendet werden. Beispiele hierzu finden sich in den folgenden Dokumenten: EP 1 872 767, EP 2 070 506, EP 1 881 010, DE 10 2007 050 763, US 6,288,138, DE 11 2006 001 049, US 7,214,726 sowie EP 2 070 935.

Geeignete Malonylsulfamide sind in der EP 0 059 451 beschrieben welche im Wege der Verweisung Bestandteil der vorliegenden Anmeldung ist. Bevorzugte Verbindungen sind dabei 2,6-Dimethyl-4-isobutylmalonylsulfamid, 2,6-Diisobutyl-4-propylmalonylsulfamid, 2,6-Dibutyl-4-propylmalonylsulfamid, 2,6-Dimethyl-4-ethylmalonylsulfamid sowie 2,6-Diocytyl-4-isobutylmalonylsulfamid.

Ferner können Schwefelverbindungen in der Oxidationsstufe +2 oder +4 wie Natriumbenzolsulfinat oder Natriumparatoluolsulfinat eingesetzt werden.

Zur Beschleunigung der Aushärtung kann die Polymerisation in Gegenwart von Schwermetallverbindungen wie Ce, Fe, Cu, Mn, Co, Sn oder Zn durchgeführt werden, wobei Kupferverbindungen besonders bevorzugt sind. Die Schwermetallverbindungen werden bevorzugt in Form löslicher organischer Verbindungen eingesetzt. Bevorzugte Kupferverbindungen sind dabei Kupferbenzoat, Kupferacetat, Kupferethylhexanoat, Kupferdi(methacrylat), Kupferacetylacetonat und Kupfernaphthenat.

<u>Bestandteil f.)</u> - weitere übliche Additive

Eine erfindungsgemäße radikalisch härtbare dentale Zusammensetzung umfasst in manchen Fällen ein oder mehrere weitere(s) Additiv(e).

Diese Additive können verschiedene Funktionen haben. Übliche Additive für den Einsatz in dentalen Werkstoffen sind dem Fachmann bekannt, je nach gewünschter Funktion wird er das oder die geeigneten Additive auswählen. Beispielhaft sind im Folgenden typische Additive und ihre Funktionen beschrieben.

Radikalisch lichthärtbare dentale Zusammensetzungen, wie sie erfindungsgemäß bevorzugt sind, enthalten vorzugsweise einen oder mehrere Inhibitor(en), auch Stabilisator(en) genannt. Diese werden üblicherweise zugesetzt, um eine Spontanpolymerisation zu vermeiden. Sie reagieren mit vorzeitig entstehenden Radikalen, die abgefangen werden, verhindern eine vorzeitige Polymerisation und erhöhen die Lagerstabilität der lichthärtbaren dentalen Zusammensetzung. Gängige Inhibitoren sind Phenolderivate wie Hydrochinonmonomethylether (HQME) oder 2,6-Di-tert.butyl-4-methylphenol (BHT). Weitere Inhibitoren wie und tert.-Butylhydroxyanisol (BHA), 2,2 Diphenyl-1-picryl-hydrazyl-, Galvinoxyl-, Triphenylmethyl-Radikale, 2,3,6,6,-Tetramethylpiperidinyl-1-oxylradikale (TEMPO) sowie Derivate von TEMPO oder Phenothiazin sowie Derivate dieser Verbindung werden in der EP 0 783 880 B1 beschrieben welche im Wege der Verweisung Bestandteil der vorliegenden Anmeldung sind. Alternative Inhibitoren sind in der DE 101 19 831 A1 oder in der EP 1 563 821 A1 angegeben, welche im Wege der Verweisung Bestandteil der vorliegenden Anmeldung sind.

Eine erfindungsgemäß bevorzugte radikalisch härtbare dentale Zusammensetzung umfasst somit als Additiv ein oder mehrere Polymerisationsinhibitoren zur Erhöhung der Lagerstabilität der Zusammensetzung, vorzugsweise ausgewählt aus der Gruppe bestehend aus Hydrochinonmonomethylether (HQME), Phenolen, dabei vorzugsweise 2,6-Di-tert.butyl-4-methylphenol (BHT) und tert.-Butylhydroxyanisol (BHA), 2,2-Diphenyl-1-picrylhydrazyl-Radikalen, Galvinoxyl-Radikalen, Triphenylmethyl-Radikalen, 2,3,6,6,-Tetramethylpiperidinyl-1-oxylradikal (TEMPO) sowie dessen Derivaten und Phenothiazin sowie dessen Derivaten.

Eine erfindungsgemäße radikalisch härtbare Zusammensetzung kann als Additiv eine oder mehrere fluoridabgebende Substanzen, dabei vorzugsweise Natriumfluorid und/oder Aminfluoride umfassen.

UV-Absorber, die beispielsweise durch ihre konjugierten Doppelbindungssysteme und aromatischen Ringe befähigt sind, UV Strahlung zu absorbieren, sind in manchen Fällen Bestandteil einer erfindungsgemäßen radikalisch härtbaren dentalen Zusammensetzung. Beispiele an UV-Absorbern sind 2-Hydroxy-4-methoxybenzophenon, Salicylsäurephenylester 3-(2'-Hydroxy-5'-methylphenyl)benzotriazol oder Diethyl-2,5-dihydroxyterephthalat.

Da die Zähne möglichst naturgetreu wiederherzustellen sind, ist es notwendig, erfindungsgemäße dentale Zusammensetzungen in den unterschiedlichsten Farbtönen bereitzustellen. Man benutzt zu diesem Zweck in der Regel anorganische Farbstoffe sowie organische Pigmente in sehr geringen Mengen, die somit in bevorzugten Ausgestaltungen als Additiv eingesetzt werden.

Weitere optionale Additive sind Aromastoffe, dentale Arzneimittel, organische Polymere und Oligomere, vorzugsweise Weichmacher, Mikrobizide, vorzugsweise Bakterizide, grenzflächenaktive Substanzen, vorzugsweise Tenside, Konservierungsmittel oder Molekulargewichtsregler.

In einer bevorzugten erfindungsgemäßen Zusammensetzung umfasst die Zusammensetzung die Komponente

a.) zu 15 - 25 Gew.-%,

b.) zu 5 - 15 Gew.-%,

c.) zu 0 - 10 Gew.-%,

d.) zu 60 - 78 Gew.-%,

e.) zu 0,1 - 2 Gew.-% und

f.) zu 0,001 - 10 Gew.-%,

wobei die jeweiligen Gewichtsprozente auf die Gesamtmasse der Zusammensetzung bezogen sind.

In besonders bevorzugten Fällen enthält eine erfindungsgemäße radikalisch härtbare dentale Zusammensetzung keinen Bestandteil c.).

In besonders bevorzugten Fällen umfasst eine erfindungsgemäße radikalisch härtbare dentale Zusammensetzung das 1,3-Bis(3-methacryloxypropyl)tetramethyldisiloxan als Bestandteil b.).

In einem ganz besonders bevorzugten Fall enthält eine erfindungsgemäße radikalisch härtbare dentale Zusammensetzung keinen Bestandteil c.) und das 1,3-Bis(3-methacryloxypropyl)tetramethyldisiloxan als Bestandteil b.).

Die vorliegende Erfindung betrifft auch die gehärtete dentale Zusammensetzung, erhältlich aus der erfindungsgemäßen radikalisch härtbaren dentalen Zusammensetzung mittels radikalischer Polymerisation der in der Zusammensetzung enthaltenen Polysiloxanverbindung(en) und der Disiloxanverbindung(en) sowie gegebenenfalls weiterer im Dentalmaterial enthaltener polymerisierbarer Bestandteile. Die Polymerisation bzw. Vernetzung erfolgt mittels der in den (Meth)acrylatgruppen enthaltenen organisch polymerisierbaren Doppelbindungen.

Das zu bevorzugten Ausführungsformen erfindungsgemäßer radikalisch härtbarer dentaler Zusammensetzungen Gesagte gilt für die erfindungsgemäßen radikalisch gehärteten dentalen Zusammensetzungen entsprechend.

Die vorliegende Erfindung betrifft ebenfalls eine erfindungsgemäße radikalisch härtbare dentale Zusammensetzung zur Anwendung in einem therapeutischen Verfahren als fließfähiges Kompositmaterial (Flow-Material), als Befestigungszement und/oder als Stumpfaufbaumaterial.

Eigene Untersuchungen haben gezeigt, dass die erfindungsgemäßen radikalisch härtbaren dentalen Zusammensetzungen und die erfindungsgemäßen radikalisch gehärteten Zusammensetzungen zu hervorragenden Ergebnissen bei den vorstehend genannten spezifischen Anwendungen führen.

Die vorliegende Erfindung betrifft außerdem ein Verfahren zur Herstellung einer radikalisch härtbaren dentalen

Zusammensetzung mit den folgenden Schritten:

- Bereitstellen der Bestandteile a.), b.), c.), d.), e.), f.) und
- Mischen der Bestandteile

Die vorliegende Erfindung betrifft darüber hinaus auch ein Verfahren zur Herstellung einer radikalisch gehärteten dentalen Zusammensetzung mit den folgenden Schritten:

- Bereitstellen der Bestandteile a.), b.), c.), d.), e.), f.),
- Mischen der Bestandteile und
- Härten des Gemischs

In erfindungsgemäßen Verfahren zur Herstellung einer radikalisch härtbaren dentalen Zusammensetzung, die mittels Strahlungshärtung polymerisierbar ist, ist die Herstellung eines einkomponentigen Systems bevorzugt. In diesem Fall enthält die härtbare Zusammensetzung sämtliche für eine Lichthärtung erforderlichen Bestandteile in einer einzigen Komponente und eine Härtung wird durch Bestrahlen mit Licht einer definierten Wellenlänge initiiert.

Vorzugsweise wird das einkomponentige System als Bestandteil eines erfindungsgemäßen Kits eingesetzt. Die vorliegende Erfindung betrifft somit auch ein Kit, umfassend

- Ein, zwei oder mehr als zwei erfindungsgemäße lichthärtende Einkomponentendentalmaterialen in einer Flow Spritze und/oder Compule,
- optional ein, zwei oder mehr als zwei Bondings,
- optional ein, zwei oder mehr als zwei Ätzgele,
- optional eine oder mehr als eine Farbskala,
- optional einen oder mehr als einen Pinsel.

In erfindungsgemäßen Verfahren zur Herstellung einer radikalisch härtbaren dentalen Zusammensetzung, die (a) mittels Lichthärtung und mittels chemischer Härtung härtbar ist (dualhärtend) oder (b) nur durch chemische Härtung härtbar ist, ist die Herstellung eines mehrkomponentigen Systems bevorzugt. Härtbare dentale Zusammensetzungen, die mittels chemischer Härtung härtbar sind, werden in der Regel als zweikomponentige Systeme formuliert, so dass die eine Polymerisation auslösenden Substanzen und die polymerisierbaren Verbindungen in separaten Komponenten vorliegen und erst durch Vermischen der getrennten Komponenten eine Aushärtung erfolgt. Da dualhärtende Systeme immer auch chemisch härtend sind, werden diese Zusammensetzungen ebenfalls zweikomponentig formuliert und hergestellt.

Vorzugsweise wird das zweikomponentige System als Bestandteil eines erfindungsgemäßen Kits eingesetzt. Die vorliegende Erfindung betrifft somit auch ein Kit zu einem Stumpfaufbaumaterial, umfassend

- Erfindungsgemäßes Stumpfaufbaumaterial in einer 2-Komponentenspritze
- Statische Mischer (zum Mischen der beiden Komponenten)
- Ein Stößel oder Dispenser (zum Applizieren des Stumpfaufbaumaterials durch den statischen Mischer)
- Optional ein kompatibles Bonding
- Optional ein oder mehrere Wurzelstifte
- Optional Formhilfen und/oder Matritzen
- Optional weiteres Zubehör (Pinsel, Bohrer usw.)

Die vorliegende Erfindung betrifft somit auch ein Kit zu einem Befestigungsmaterial, umfassend

- Erfindungsgemäßes Befestigungsmaterial in einer 2-Komponentenspritze
- Statische Mischer (zum Mischen der beiden Komponenten)
- Ein Stößel oder Dispenser (zum Applizieren des Befestigungsmaterials durch den statischen Mischer)
- Optional ein kompatibles Bonding
- Optional weitere Haftvermittler (z.B. für Keramik, Metall usw.)
- Optional Try-In-Pasten
- Optional weiteres Zubehör (Pinsel, usw.)

Beispiele

Eingesetzte Substanzen:

| | |
|---|---|
| Polysiloxan I: | Methacryl-POSS (MA0735, Hybrid Plastics Inc.) |
| Polysiloxan II: | Kondensationsprodukt aus 3-Methacryloxypropyldimethoxymethylsilan |
| Polysiloxan III: | Kondensationsprodukt aus 3-Methacryloxypropyltrimethoxysilan |
| Polysiloxan IV: | Kondensationsprodukt aus 3-[(2-Hydroxy-3-methacryloxy)propoxy]propyldimethoxymethylsilan |
| Disiloxan I: | 1,3-Bis(3-methacryloxypropyl)tetramethyldisiloxan |
| Disiloxan II: | 1,3-Bis[3-[(2-hydroxy-3-methacryloxy)propoxy]propyl]-tetramethyldisiloxan |
| Bis-GMA: | 2,2-Bis[4-(2-hydroxy-3-methacryloxypropoxy)phenyl]propan |
| TEGDMA: | Triethylenglycoldimethacrylat |
| MDP: | 10-Methacryloxyoxydecylphosphat |
| CQ: | DL-Kampferchinon |
| DABE: | 4-Dimethylaminobenzoesäureethylester |
| BHT: | 2,6-Di(tert-butyl)hydroxytoluol |
| DEPT: | N,N-Bis(2-hydroxyethyl)-p-toluidin |
| NTPB: | Natriumtetraphenylborat |
| BPO: | Dibenzoylperoxid |

Synthese der Polysiloxane

Synthese von Polysiloxan II

100 g (0,42 mol) 3-Methacryloxypropyldimethoxymethylsilan werden in 400 ml Essigester gelöst. Es werden 10 ml 1N HCl-Lösung zugetropft und für 72 h bei 30°C gerührt. Es wird mit 2N NaOH-Lösung ausgeschüttelt, mit Wasser gewaschen und die organische Phase über Magnesiumsulfat getrocknet. Nach Zusatz von BHT wird zunächst bei 40 °C abrotiert und anschließend werden Lösungsmittelreste (z.B. Wasser- und Alkoholreste) unter Vakuum mittels einer Ölpumpe abgezogen, um die Alkohol- und Wasserreste zu entfernen. Es resultiert ein flüssiges Harz mit einer Viskosität von 3 Pa*s bei 25 °C.
$n_D^{20}$ = 1,466

Synthese von Polysiloxan III

100 g (0,40 mol) 3-Methacryloxypropyltrimethoxysilan werden in 400 ml Essigester gelöst. Es werden 15 ml 1N HCl-Lösung zugetropft und für 72 h bei 30°C gerührt. Es wird mit 2N NaOH-Lösung ausgeschüttelt, mit Wasser gewaschen und die organische Phase über Magnesiumsulfat getrocknet. Nach Zusatz von BHT wird zunächst bei 40 °C abrotiert und anschließend werden Lösungsmittelreste (z.B. Wasser- und Alkoholreste) unter Vakuum mittels einer Ölpumpe abgezogen, um die Alkohol- und Wasserreste zu entfernen. Es resultiert ein flüssiges Harz mit einer Viskosität von 20 Pa*s bei 25 °C.
$n_D^{20}$ = 1,479

Synthese von Polysiloxan IV

a) Synthese einer monomeren Silaneinheit (vgl. EP 1 685 182 B1, Bsp. 3):

Zur Vorlage von 100 g (0,402 mol) 3-Glycidyloxypropyldiethoxymethylsilan werden unter trockener Atmosphäre ein Additionskatalysator, BHT als Stabilisator und anschließend 38,05 g (0,442 mol) Methacrylsäure zugetropft und bei ca. 80°C gerührt (ca. 24 h). Die Umsetzung wird über die Abnahme der Carbonsäurekonzentration mittels Säuretitration sowie dem Epoxidumsatz mittels Ramanspektroskopie/Epoxidtitration verfolgt. Die für die Epoxidgruppe charakteristische Bande wird im Ramanspektrum bei 1256 $cm^{-1}$ detektiert. Der Epoxid- bzw. Carbonsäureumsatz liegt bei 99 % bzw. 88 % (Folge des Carbonsäureüberschusses).

b) Hydrolyse bzw. Kondensation der monomeren Silaneinheit zu einer Polysiloxanverbindung (vgl. EP 1 685 182 B1, Bsp. 6):

Nach Zugabe von Essigester (1000 ml/mol monomerer Silaneinheit) und Wasser zur Hydrolyse mit HCl als Katalysator zur synthetisierten monomeren Silaneinheit wird bei 30°C gerührt. Der Verlauf der Hydrolyse wird durch Wassertitration verfolgt. Die Aufarbeitung erfolgt nach mehrtägigem Rühren bei 30°C durch mehrmaliges Ausschütteln mit wässriger NaOH und anschließendem Ausschütteln mit Wasser und Filtration über einen hydrophobierten Filter.

Nach Zusatz von BHT wird zunächst bei 40 °C abrotiert und anschließend werden Lösungsmittelreste (z.B. Wasser- und Alkoholreste) unter Vakuum mittels einer Ölpumpe abgezogen, um die Alkohol- und Wasserreste zu entfernen. Es resultiert ein flüssiges Harz mit einer Viskosität von 4,5 Pa*s bei 25 °C.
$n_D^{20} = 1,483$

Synthese der Disiloxane

Synthese von Disiloxan II

Zur Vorlage von 100 g (0,28 mol) 1,3-Bis(glycidoxypropyl)disiloxan werden unter trockener Atmosphäre ein Additionskatalysator, BHT als Stabilisator und anschließend 26,10 g (0,30 mol) Methacrylsäure zugetropft und bei ca. 80°C gerührt (ca. 24 h). Die Umsetzung wird über die Abnahme der Carbonsäurekonzentration mittels Säuretitration sowie dem Epoxidumsatz mittels Ramanspektroskopie/Epoxidtitration verfolgt. Die für die Epoxidgruppe charakteristische Bande wird im Ramanspektrum bei 1256 $cm^{-1}$ detektiert. Der Epoxid- bzw. Carbonsäureumsatz liegt bei 99 % bzw. 88 % (Folge des Carbonsäureüberschusses). Es werden 300 ml Essigester zugegeben. Anschließend wird mit 2N NaOH-Lösung ausgeschüttelt, mit Wasser gewaschen und die organische Phase über Magnesiumsulfat getrocknet. Nach Zusatz von BHT wird das Lösungsmittel unter Vakuum entfernt. Es resultiert ein dünnflüssiges Harz mit einer Viskosität von 0,3 Pa*s bei 25 °C.
$n_D^{20} = 1,466$

Lichthärtende Polysiloxan/Disiloxan-Harze (Beispiele 1-4)

CQ und DABE wurden in den jeweiligen Monomeren (Polysiloxan und Disiloxan) gelöst. Die Lösungen wurden bei -0,9 bar Vakuum entlüftet. Es wurde die Biegefestigkeit (BF) der einzelnen Harze bestimmt und aus diesen Messungen der Elastizitätsmodul (E-Modul) berechnet.

Überraschenderweise wird dabei für die Mischungen aus Polysiloxan und Disiloxan ein höherer E-Modul gefunden als für die jeweiligen beiden Einzelkomponenten. Das Maximum für den E-Modul wurde bei einem Verhältnis von Polysiloxan zu Disiloxan von etwa 2:1 gefunden (siehe Abbildung 1).

Vergleichsbeispiel 1:

Tabelle 1: Zusammensetzung und Eigenschaften Poly-/Disiloxan-Harze 1

| | 1-A | 1-B | 1-C | 1-D | 1-E |
|---|---|---|---|---|---|
| Polysiloxan I | 99,25 | 82,71 | 66,17 | 49,625 | 0,00 |
| Disiloxan I | 0,00 | 16,54 | 33,08 | 49,625 | 99,25 |

(fortgesetzt)

| | 1-A | 1-B | 1-C | 1-D | 1-E |
|---|---|---|---|---|---|
| CQ | 0,30 | 0,30 | 0,30 | 0,30 | 0,30 |
| DABE | 0,45 | 0,45 | 0,45 | 0,45 | 0,45 |
| BF | 9,9 MPa | 20,8 MPa | 25,2 MPa | 28,2 MPa | 36,3 MPa |
| E-Modul | 560 MPa | 960 MPa | 1040 MPa | 1020 MPa | 880 MPa |

Vergleichsbeispiel 2:

Tabelle 2: Zusammensetzung und Eigenschaften Poly-/Disiloxan-Harze 2

| | 2-A | 2-B | 2-C | 2-D | 2-E |
|---|---|---|---|---|---|
| Polysiloxan II | 99,25 | 82,71 | 66,17 | 49,625 | 0,00 |
| Disiloxan I | 0,00 | 16,54 | 33,08 | 49,625 | 99,25 |
| CQ | 0,30 | 0,30 | 0,30 | 0,30 | 0,30 |
| DABE | 0,45 | 0,45 | 0,45 | 0,45 | 0,45 |
| BF | 13,0 MPa | 22,3 MPa | 23,0 MPa | 27,8 MPa | 36,3 MPa |
| E-Modul | 920 MPa | 1140 MPa | 1200 MPa | 1130 MPa | 880 MPa |

Vergleichsbeispiel 3:

Tabelle 3: Zusammensetzung und Eigenschaften Poly-/Disiloxan-Harze 3

| | 3-A | 3-B | 3-C | 3-D | 3-E |
|---|---|---|---|---|---|
| Polysiloxan III | 99,25 | 82,71 | 66,17 | 49,625 | 0,00 |
| Disiloxan I | 0,00 | 16,54 | 33,08 | 49,625 | 99,25 |
| CQ | 0,30 | 0,30 | 0,30 | 0,30 | 0,30 |
| DABE | 0,45 | 0,45 | 0,45 | 0,45 | 0,45 |
| BF | 19,6 MPa | 31,3 MPa | 32,0 MPa | 31,7 MPa | 36,3 MPa |
| E-Modul | 1050 MPa | 1360 MPa | 1380 MPa | 1250 MPa | 880 MPa |

Vergleichsbeispiel 4:

Tabelle 4: Zusammensetzung und Eigenschaften Poly-/Disiloxan-Harze 4

| | 4-A | 4-B | 4-C | 4-D | 4-E |
|---|---|---|---|---|---|
| Polysiloxan IV | 99,25 | 82,71 | 66,17 | 49,625 | 0,00 |
| Disiloxan II | 0,00 | 16,54 | 33,08 | 49,625 | 99,25 |
| CQ | 0,30 | 0,30 | 0,30 | 0,30 | 0,30 |
| DABE | 0,45 | 0,45 | 0,45 | 0,45 | 0,45 |
| BF | 25,7MPa | 36,3 MPa | 38,1 MPa | 34,5 MPa | 28,4 MPa |

(fortgesetzt)

| | 4-A | 4-B | 4-C | 4-D | 4-E |
|---|---|---|---|---|---|
| E-Modul | 1260 MPa | 1530 MPa | 1550 MPa | 1470 MPa | 1090 MPa |

Herstellung und Eigenschaften von fließfähigen Kompositmaterialien (Beispiel 5)

Für die Herstellung fließfähigen Komposit-Pasten wurden die Bestandteile jeweils eingewogen an einem Speed-Mixer™ DAC 600.1 VAC-P (Hauschild & Co KG, Hamm, Deutschland) homogenisiert, an einem Dreiwalzenstuhl (Exakt, Norderstedt, Deutschland) gewalzt und anschließend am SpeedMixer™ DAC 600.1 VAC-P bei -0,9 bar Vakuum entlüftet. Von den einzelnen Pasten wurden die Viskosität und die Spreitbarkeit gemessen.

Die Pasten wurden in 1 ml-Spritzen (schwarz, 3-teilig, Luerlock, mit Silikon-O-Ring) der Firma Transcodent GmbH & Co. KG abgefüllt. Für die Bestimmung der Ausdrückbarkeit und der Ausdrückkraft wurden Kanülen (Typ 41, VOCO GmbH) verwendet.

Tabelle 5: Zusammensetzungen der fließfähigen Komposite

| | 5-A | 5-B (Vergleich) | 5-C (Vergleich) | 5-D (Vergleich) | 5-E (Vergleich) |
|---|---|---|---|---|---|
| Polysiloxan IV | 20,00 | 35,00 | 0,00 | 40,00 | 45,00 |
| Disiloxan I | 10,00 | 0,00 | 20,00 | 0,00 | 0,00 |
| Bariumsilikat–Glas (1,5 µm) silanisiert | 57,40 | 53,40 | 62,40 | 47,90 | 42,40 |
| Bariumsilikat–Glas (0,7 µm) silanisiert | 9,00 | 8,00 | 14,00 | 6,00 | 4,00 |
| Aerosil R8200 | 2,50 | 2,50 | 2,50 | 5,00 | 7,50 |
| CQ | 0,40 | 0,40 | 0,40 | 0,40 | 0,40 |
| DABE | 0,60 | 0,60 | 0,60 | 0,60 | 0,60 |
| BHT | 0,10 | 0,10 | 0,10 | 0,10 | 0,10 |

Tabelle 5 (Fortsetzung): Zusammensetzungen der fließfähigen Komposite

| | 5-F (Vergleich) | 5-G | 5-H (Vergleich) | 5-I (Vergleich) | 5-J |
|---|---|---|---|---|---|
| Polysiloxan IV | 32,00 | 15,00 | 6,00 | 0,00 | 30,00 |
| Disiloxan I | 0,00 | 7,50 | 3,00 | 10,00 | 15,00 |
| Bis-GMA | 0,00 | 5,00 | 16,00 | 10,00 | 0,00 |
| TEGDMA | 0,00 | 2,50 | 8,00 | 10,00 | 0,00 |
| Bariumsilikat–Glas (1,5 µm) silanisiert | 56,40 | 57,40 | 54,40 | 57,40 | 31,40 |
| Bariumsilikat–Glas (0,7 µm) silanisiert | 8,00 | 9,00 | 9,00 | 9,00 | 20,00 |
| Aerosil R8200 | 2,50 | 2,50 | 2,50 | 2,50 | 2,50 |
| CQ | 0,40 | 0,40 | 0,40 | 0,40 | 0,40 |
| DABE | 0,60 | 0,60 | 0,60 | 0,60 | 0,60 |
| BHT | 0,10 | 0,10 | 0,10 | 0,10 | 0,10 |

Tabelle 5 (Fortsetzung II): Zusammensetzungen der fließfähigen Komposite

| | 5-K | 5-L | 5-M | 5-N | 5-O |
|---|---|---|---|---|---|
| Polysiloxan IV | 20,00 | 18,00 | 16,00 | 16,00 | 15,00 |
| Disiloxan I | 10,00 | 9,00 | 8,00 | 0,00 | 7,50 |
| Disiloxan II | 0,00 | 0,00 | 0,00 | 8,00 | 0,00 |
| Bis-GMA | 0,00 | 0,00 | 0,00 | 0,00 | 5,00 |
| TEGDMA | 0,00 | 0,00 | 0,00 | 0,00 | 2,50 |
| Bariumsilikat–Glas (1,5 µm) silanisiert | 51,40 | 53,40 | 49,40 | 49,40 | 51,40 |
| Bariumsilikat–Glas (0,7 µm) silanisiert | 7,00 | 8,00 | 7,00 | 7,00 | 7,00 |
| nanosklaliges $SiO_2$ (40 nm) silanisiert | 8,00 | 8,00 | 16,00 | 16,00 | 8,00 |
| Aerosil R8200 | 2,50 | 2,50 | 2,50 | 2,50 | 2,50 |
| CQ | 0,40 | 0,40 | 0,40 | 0,40 | 0,40 |
| DABE | 0,60 | 0,60 | 0,60 | 0,60 | 0,60 |
| BHT | 0,10 | 0,10 | 0,10 | 0,10 | 0,10 |

Tabelle 6: Eigenschaften der fließfähigen Komposite

| | 5-A | 5-B | 5-C | 5-D | 5-E |
|---|---|---|---|---|---|
| BF | 110 MPa | 94 MPa | 75 MPa | 82 MPa | 71 MPa |
| E-Modul | 6,0 GPa | 5,5 GPa | 5,0 GPa | 5,0 GPa | 4,5 GPa |
| Schrumpfung | 2,5% | 1,8% | 3,5% | 2,4% | 3,1% |
| Viskosität (0,01/s) | 10,0 kPa*s | 0,8 kPa*s | 6,5 kPa*s | 2,6 kPa*s | 7,2 kPa*s |
| Viskosität (10/s) | 49 Pa*s | 53 Pa*s | 48 Pa*s | 61 Pa*s | 68 Pa*s |
| Ausdrückbarkeit | gut | schwer | gut | schwer | akzeptabel |
| Ausdrückkraft | 33 N | 112 N | 40 N | 101 N | 79 N |
| Spreitbarkeit | 61 mm | 35 mm | 31 mm | 38 mm | 40 mm |

Tabelle 6 (Fortsetzung): Eigenschaften der fließfähigen Komposite

| | 5-F | 5-G | 5-H | 5-I | 5-J |
|---|---|---|---|---|---|
| BF | 118 MPa | 115 MPa | 118 MPa | 110 MPa | 91 MPa |
| E-Modul | 7,6 GPa | 7,0 GPa | 7,2 GPa | 6,5 GPa | 5,1 GPa |
| Schrumpfung | 1,6% | 3,1% | 4,0% | 3,6% | 3,4% |
| Viskosität (0,01/s) | 11,6 kPa*s | 11,4 kPa*s | 9,5 kPa*s | 7,8 kPa*s | 6,8 kPa*s |
| Viskosität (10/s) | 370 Pa*s | 58 Pa*s | 55 Pa*s | 51 Pa*s | 43 Pa*s |
| Ausdrückbarkeit | sehr schwer | gut | gut | gut | sehr gut |
| Ausdrückkraft | 189 N | 45 N | 42 N | 30 N | 25 N |
| Spreitbarkeit | 24 mm | 51 mm | 57 mm | 55 mm | 67 mm |

Tabelle 6 (Fortsetzung II): Eigenschaften der fließfähigen Komposite

| | 5-K | 5-L | 5-M | 5-N | 5-O |
|---|---|---|---|---|---|
| BF | 113MPa | 116 MPa | 121 MPa | 125 MPa | 118 MPa |
| E-Modul | 6,5 GPa | 6,9 GPa | 7,3 GPa | 7,5 GPa | 7,2 GPa |
| Schrumpfung | 2,4% | 2,2% | 2,1% | 2,0% | 3,1% |
| Viskosität (0,01/s) | 10,8 kPa*s | 11,4 kPa*s | 11,9 kPa*s | 11,8 kPa*s | 12,1 kPa*s |
| Viskosität (10/s) | 51 Pa*s | 59 Pa*s | 63 Pa*s | 62 Pa*s | 61 Pa*s |
| Ausdrückbarkeit | gut | gut | akzeptabel | akzeptabel | gut |
| Ausdrückkraft | 41 N | 55 N | 81 N | 79 N | 55 N |
| Spreitbarkeit | 55 mm | 51 mm | 48 mm | 47 mm | 49 mm |

Die Ergebnisse zeigen deutlich, dass durch Kombination von Polysiloxanen und Disiloxanen die Eigenschaften des Flow Materials deutlich verbessert werden. Wie schon bei Betrachtung der Poly-/Disiloxan-Harzmischungen gezeigt, treten auch im Komposit synergistische Effekte auf. Werden die unterschiedlichen Pasten auf eine vergleichbare verarbeitbare Viskosität (bei 10 $s^{-1}$) eingestellt (5-A bis C), zeigt sich, dass neben dem erwarteten reinen Verdünnungseffekt bei Kombination des speziellen niederviskosen Disiloxan mit dem hochviskosen Polysiloxan unerwartet bessere Ergebnisse in puncto Festigkeits- und Elastizitätseigenschaften erreicht werden im Vergleich zu den Pasten mit entweder nur Polysiloxan (5-B) oder nur Disiloxan (5-C).

Die besseren Festigkeitseigenschaften können nicht über einen höheren Füllstoffgehalt erklärt werden, da die Paste 5-C auf Basis des Disiloxans trotz des erhöhten Feststoffgehaltes eine geringere Festigkeit aufweist. Auch die Vernetzungseigenschaften des Disiloxans können nicht als Begründung herangezogen werden, da sie wie die Untersuchungen an den Harzen gezeigt haben, eigentlich ein höheres Vernetzungspotenzial aufweisen.

In Abbildung 2 sind die Kurven für die Viskosität in Abhängigkeit von der Scherrate für das erfindungsgemäße Flow 5-A sowie für die beiden Vergleichsmaterialien 5-B und 5-F dargestellt. Flow 5-A weist die für ein fließfähiges Komposit typische Viskositätskurve auf. Bei hoher Scherrate ist die Viskosität niedrig und das Flow kann gut durch die Kanüle fließen. Bei niedriger Scherrate ist die Viskosität hoch und das Material standfest.

Bei 5-B ist die Viskosität so eingestellt, dass sie bei einer Scherrate von 10 $s^{-1}$ etwa Beispiel 5-A entspricht. Dafür ist die Viskosität bei 0,01 $s^{-1}$ deutlich niedriger und das Material dementsprechend weniger standfest. Bei 5-F ist die Viskosität so eingestellt, dass sie bei einer Scherrate von 0,01 $s^{-1}$ etwa Beispiel 5-A entspricht. Dafür ist hier die Viskosität bei 10 $s^{-1}$ deutlich höher und das Material lässt sich nur sehr schwer ausdrücken.

Erhöht man ausgehend vom reinen Polysiloxan-Material 5-B den Aerosilgehalt (5-D und E), so erhöht sich wie erwartet das Schergefälle, die Viskosität nährt sich langsam 5-A an und die Ausdrückbarkeit wird besser (siehe Abbildung 3). Gleichzeitig nimmt aber der erreichbare Füllstoffgehalt ab und damit werden Biegefestigkeit und Schrumpfung schlechter.

Herstellung und Eigenschaften von Stumpfaufbaumaterialien (Beispiel 6)

Für die Herstellung der Basis- und Katalysator-Pasten der Stumpfaufbaumaterialien wurden die Bestandteile jeweils eingewogen an einem SpeedMixer™ DAC 600.1 VAC-P (Hauschild & Co KG, Hamm, Deutschland) homogenisiert, an einem Dreiwalzenstuhl (Exakt, Norderstedt, Deutschland) gewalzt und anschließend am SpeedMixer™ DAC 600.1 VAC-P bei -0,9 bar Vakuum entlüftet. Von den einzelnen Pasten wurden die Viskosität und die Spreitbarkeit gemessen.

Die Stumpfaufbaumaterialien wurden in Doppelkammerspritzen (5 ml, 1:1, SDL X05-01-52, PED X05-01-SI) der Firma Sulzer Mixpac AG (Haag, Schweiz) abgefüllt. Zum Mischen wurden die passenden, statischen Mischer ML 2.5-08-D und IOT 212-20 der Firma Sulzer Mixpac AG auf die Spritzen aufgesetzt und die beiden Komponenten mit einem Stössel (PLH X05-01-46) ausgedrückt und homogen gemischt.

Tabelle 7: Zusammensetzungen der Stumpfaufbaumaterialien

| | 6-A | 6-B (Vergleich) | 6-C (Vergleich) | 6-D (Vergleich) | 6-E (Vergleich) |
|---|---|---|---|---|---|
| Basis-Paste | | | | | |
| Polysiloxan IV | 20,00 | 30,00 | 40,00 | 0,00 | 0,00 |
| Disiloxan I | 10,00 | 0,00 | 0,00 | 30,00 | 20,00 |
| Bis-GMA | 0,00 | 0,00 | 0,00 | 0,00 | 0,00 |
| TEGDMA | 0,00 | 0,00 | 0,00 | 0,00 | 0,00 |
| Bariumsilikat–Glas (3,5 µm) silanisiert | 55,95 | 55,95 | 47,95 | 55,95 | 63,95 |
| Bariumsilikat–Glas (0,7 µm) silanisiert | 10,00 | 10,00 | 8,00 | 10,00 | 12,00 |
| nanosklaliges $SiO_2$ (40 nm) silanisiert | 0,00 | 0,00 | 0,00 | 0,00 | 0,00 |
| Aerosil R8200 | 3,00 | 3,00 | 3,00 | 3,00 | 3,00 |
| DEPT | 0,60 | 0,60 | 0,60 | 0,60 | 0,60 |
| CQ | 0,18 | 0,18 | 0,18 | 0,18 | 0,18 |
| DABE | 0,27 | 0,27 | 0,27 | 0,27 | 0,27 |
| | | | | | |
| Kat-Paste | | | | | |
| Polysiloxan IV | 20,00 | 30,00 | 40,00 | 0,00 | 0,00 |
| Disiloxan I | 10,00 | 0,00 | 0,00 | 30,00 | 20,00 |
| Bis-GMA | 0,00 | 0,00 | 0,00 | 0,00 | 0,00 |
| TEGDMA | 0,00 | 0,00 | 0,00 | 0,00 | 0,00 |
| Bariumsilikat-Glas (3,5 µm) silanisiert | 55,95 | 55,95 | 47,95 | 55,95 | 63,95 |
| Bariumsilikat–Glas (0,7 µm) silanisiert | 10,00 | 10,00 | 8,00 | 10,00 | 12,00 |
| nanosklaliges $SiO_2$ (40 nm) silanisiert | 0,00 | 0,00 | 0,00 | 0,00 | 0,00 |
| Aerosil R8200 | 3,00 | 3,00 | 3,00 | 3,00 | 3,00 |
| BPO | 1,00 | 1,00 | 1,00 | 1,00 | 1,00 |
| BHT | 0,05 | 0,05 | 0,05 | 0,05 | 0,05 |

Tabelle 7 (Fortsetzung): Zusammensetzungen der Stumpfaufbaumaterialien

| | 6-F | 6-G | 6-H | 6-I |
|---|---|---|---|---|
| Basis-Paste | | | | |
| Polysiloxan IV | 16,00 | 10,00 | 18,00 | 16,00 |
| Disiloxan I | 8,00 | 5,00 | 9,00 | 8,00 |
| Bis-GMA | 4,00 | 10,00 | 0,00 | 0,00 |
| TEGDMA | 2,00 | 5,00 | 0,00 | 0,00 |
| Bariumsilikat–Glas (3,5 µm) silanisiert | 55,95 | 55,95 | 51,95 | 47,95 |
| Bariumsilikat–Glas (0,7 µm) silanisiert | 10,00 | 10,00 | 8,00 | 6,00 |
| nanosklaliges $SiO_2$ (40 nm) silanisiert | 0,00 | 0,00 | 9,00 | 18,00 |
| Aerosil R8200 | 3,00 | 3,00 | 3,00 | 3,00 |
| DEPT | 0,60 | 0,60 | 0,60 | 0,60 |
| CQ | 0,18 | 0,18 | 0,18 | 0,18 |
| DABE | 0,27 | 0,27 | 0,27 | 0,27 |
| | | | | |
| Kat-Paste | | | | |
| Polysiloxan IV | 16,00 | 10,00 | 18,00 | 16,00 |
| Disiloxan I | 8,00 | 5,00 | 9,00 | 8,00 |
| Bis-GMA | 4,00 | 10,00 | 0,00 | 0,00 |
| TEGDMA | 2,00 | 5,00 | 0,00 | 0,00 |
| Bariumsilikat–Glas (3,5 µm) silanisiert | 55,95 | 55,95 | 51,95 | 47,95 |
| Bariumsilikat–Glas (0,7 µm) silanisiert | 10,00 | 10,00 | 8,00 | 6,00 |
| nanosklaliges $SiO_2$ (40 nm) silanisiert | 0,00 | 0,00 | 9,00 | 18,00 |
| Aerosil R8200 | 3,00 | 3,00 | 3,00 | 3,00 |
| BPO | 1,00 | 1,00 | 1,00 | 1,00 |
| BHT | 0,05 | 0,05 | 0,05 | 0,05 |

Tabelle 8: Eigenschaften der Stumpfaufbaumaterialien

| | 6-A | 6-B (Vergleich) | 6-C (Vergleich) | 6-D (Vergleich) | 6-E (Vergleich) |
|---|---|---|---|---|---|
| BF | 108 MPa | 115 MPa | 97 MPa | 75 MPa | 85 MPa |
| E-Modul | 7,0 GPa | 7,2 GPa | 6,2 GPa | 5,2 GPa | 5,8 GPa |
| Schrumpfung | 2,6% | 1,8% | 3,1% | 4,2% | 3,6% |
| Viskosität (0,01/s, Basis) | 8,9 kPa*s | 15,1 kPa*s | 1,5 kPa*s | 5,8 kPa*s | 6,7 kPa*s |
| Viskosität (10/s, Basis) | 60 Pa*s | 105 Pa*s | 58 Pa*s | 25 Pa*s | 68 Pa*s |
| Viskosität (0,01/s, Kat) | 9,1 kPa*s | 15,8 kPa*s | 1,6 kPa*s | 6,1 kPa*s | 6,9 kPa*s |
| Viskosität (10/s, Kat) | 61 Pa*s | 108 Pa*s | 60 Pa*s | 26 Pa*s | 70 Pa*s |
| Ausdrückbarkeit | gut | sehr schwer | schwer | sehr gut | gut |
| Ausdrückkraft | 62 N | 225 N | 121 N | 49 N | 77 N |
| Spreitbarkeit (Basis) | 60 mm | 37 mm | 41 mm | 70mm | 49 mm |
| Spreitbarkeit (Kat) | 58 mm | 36 mm | 40 mm | 68 mm | 48 mm |

Tabelle 8 (Fortsetzung): Eigenschaften der Stumpfaufbaumaterialien

| | 6-F | 6-G | 6-H | 6-I |
|---|---|---|---|---|
| BF | 111 MPa | 112 MPa | 115 MPa | 120 MPa |
| E-Modul | 7,1 GPa | 7,0 GPa | 7,3 GPa | 7,5 GPa |
| Schrumpfung | 2,7% | 2,8% | 2,4% | 2,2% |
| Viskosität (0,01/s, Basis) | 9,5 kPa*s | 9,9 kPa*s | 9,8 kPa*s | 10,5 kPa*s |
| Viskosität (10/s, Basis) | 61 Pa*s | 61 Pa*s | 65 Pa*s | 68 Pa*s |
| Viskosität (0,01/s, Kat) | 9,9 kPa*s | 10,2 kPa*s | 10,1 kPa*s | 10,8 kPa*s |
| Viskosität (10/s, Kat) | 62 Pa*s | 63 Pa*s | 67 Pa*s | 69 Pa*s |
| Ausdrückbarkeit | gut | gut | gut | akzeptabel |
| Ausdrückkraft | 71 N | 76 N | 76 N | 85 N |
| Spreitbarkeit (Basis) | 55 mm | 51 mm | 54 mm | 49 mm |
| Spreitbarkeit (Kat) | 53 mm | 49 mm | 53 mm | 48 mm |

Ausgehend von 6-A steigt bei ausschließlicher Verwendung von Polysiloxan (6-B) die Biegefestigkeit geringfügig an, das Material lässt sich aber nur noch mit sehr hohem Kraftaufwand durch einen statischen Mischer ausdrücken und mischen. Senkt man den Füllstoffgehalt (6-C), ist das Material immer noch schwer ausdrückbar und die Biegefestigkeit nimmt ab.

Bei ausschließlicher Verwendung von Disiloxan (6-D), ist das Material sehr gut ausdrückbar, aber die Biegefestigkeit ist schlecht. Erhöht man den Füllstoffgehalt (6-E), ist bei vergleichbarer Ausdrückbarkeit die Biegefestigkeit immer noch deutlich schlechter.

Herstellung und Eigenschaften Befestiaunaskompositen (Beispiel 7)

Für die Herstellung der Basis- und Katalysator-Pasten der Befestigungskomposite wurden die Bestandteile jeweils eingewogen an einem SpeedMixer™ DAC 600.1 VAC-P (Hauschild & Co KG, Hamm, Deutschland) homogenisiert, an einem Dreiwalzenstuhl (Exakt, Norderstedt, Deutschland) gewalzt und anschließend am SpeedMixer™ DAC 600.1 VAC-P bei -0,9 bar Vakuum entlüftet. Von den einzelnen Pasten wurden die Viskosität und die Spreitbarkeit gemessen.

Die Befestigungskomposite wurden in Doppelkammerspritzen (5 ml, 1:1, SDL X05-01-52, PED X05-01-SI) der Firma Sulzer Mixpac AG (Haag, Schweiz) abgefüllt. Zum Mischen wurden die passenden, statischen Mischer ML 2.5-08-D und IOT 212-20 der Firma Sulzer Mixpac AG auf die Spritzen aufgesetzt und die beiden Komponenten mit einem Stössel (PLH X05-01-46) ausgedrückt und homogen gemischt.

Tabelle 9: Zusammensetzungen der Befestigungskomposite

| | 7-A | 7-B | 7-C | 7-D | 7-E |
|---|---|---|---|---|---|
| Basis-Paste | | | | | |
| Polysiloxan IV | 22,00 | 22,00 | 22,00 | 25,00 | 18,00 |
| Disiloxan I | 11,00 | 0,00 | 11,00 | 12,50 | 9,00 |
| Disiloxan II | 0,00 | 11,00 | 0,00 | 0,00 | 0,00 |
| Bariumsilikat-Glas (1,5 μm) silanisiert | 43,95 | 43,95 | 43,75 | 36,45 | 47,95 |
| Bariumsilikat-Glas (0,7 μm) silanisiert | 20,00 | 20,00 | 20,00 | 22,50 | 6,00 |
| nanosklaliges $SiO_2$ (40 nm) silanisiert | 0,00 | 0,00 | 0,00 | 0,00 | 16,00 |
| Aerosil R8200 | 2,00 | 2,00 | 2,00 | 2,50 | 2,00 |
| DEPT | 0,60 | 0,60 | 0,40 | 0,60 | 0,60 |
| NTPB | 0,00 | 0,00 | 0,40 | 0,00 | 0,00 |
| CQ | 0,18 | 0,18 | 0,18 | 0,18 | 0,18 |
| DABE | 0,27 | 0,27 | 0,27 | 0,27 | 0,27 |
| | | | | | |
| Kat-Paste | | | | | |
| Polysiloxan IV | 22,00 | 22,00 | 18,00 | 25,00 | 18,00 |
| Disiloxan I | 11,00 | 0,00 | 9,00 | 12,50 | 9,00 |
| Disiloxan II | 0,00 | 11,00 | 0,00 | 0,00 | 0,00 |
| MDP | 0,00 | 0,00 | 9,00 | 0,00 | 0,00 |
| Bariumsilikat-Glas (1,5 μm) silanisiert | 43,95 | 43,95 | 40,95 | 36,45 | 47,95 |
| Bariumsilikat-Glas (0,7 μm) silanisiert | 20,00 | 20,00 | 20,00 | 22,50 | 6,00 |
| nanosklaliges $SiO_2$ (40 nm) silanisiert | 0,00 | 0,00 | 0,00 | 0,00 | 16,00 |
| Aerosil R8200 | 2,00 | 2,00 | 2,00 | 2,50 | 2,00 |
| BPO | 1,00 | 1,00 | 1,00 | 1,00 | 1,00 |
| BHT | 0,05 | 0,05 | 0,05 | 0,05 | 0,05 |

Tabelle 10: Eigenschaften der Befestigungskomposite

| | 7-A | 7-B | 7-C | 7-D | 7-E |
|---|---|---|---|---|---|
| BF | 104 MPa | 113 MPa | 102 MPa | 91 MPa | 119 MPa |
| E-Modul | 6,8 GPa | 7,1 GPa | 6,5 GPa | 6,1 GPa | 7,6 GPa |
| Schrumpfung | 2,7% | 2,4% | 2,7% | 3,5% | 2,2% |
| Viskosität (0,1/s, Basis) | 8,4 kPa*s | 9,6 kPa*s | 8,8 kPa*s | 7,9 kPa*s | 10,3 kPa*s |
| Viskosität (10/s, Basis) | 55 Pa*s | 60 Pa*s | 56 Pa*s | 49 Pa*s | 64 Pa*s |
| Viskosität (0,1/s, Kat) | 8,5 kPa*s | 9,9 kPa*s | 8,9 kPa*s | 8,0 kPa*s | 10,5 kPa*s |
| Viskosität (10/s, Kat) | 56 Pa*s | 63 Pa*s | 60 Pa*s | 50 Pa*s | 65 Pa*s |
| Ausdrückbarkeit | gut | gut | gut | sehr gut | akzeptabel |
| Ausdrückkraft | 59 N | 68 N | 69 N | 43 N | 83 N |
| Spreitbarkeit (Basis) | 61 mm | 53 mm | 59 mm | 69 mm | 51 mm |
| Spreitbarkeit (Kat) | 59 mm | 51 mm | 50 mm | 67 mm | 50 mm |

Bestimmung der Eigenschaften:

Biegefestigkeit (BF): Die Biegefestigkeit wird entsprechend ISO 4049 bestimmt. Die fließfähigen Komposite (Beispiele 5) werden aus 1 ml-Spritzen (schwarz, 3-teilig, Luerlock, mit Silikon-O-Ring) der Firma Transcodent GmbH & Co. KG durch Kanülen (Typ 41, VOCO GmbH) in die Form appliziert. Die Stumpfaufbau- und Befestigungskomposite (Beispiele 6 und 7) werden aus 5 ml-Doppelkammerspritzen (SDL X05-01-52, PED X05-01-SI) der Firma Sulzer Mixpac AG (Haag, Schweiz) durch einen statischen Mischer (ML 2.5-08-D und IOT 212-20) in die Formen appliziert. Alle Materialien werden mit einer Celalux 2-Lampe (VOCO GmbH) abschnittsweise für 40 Sekunden lichtgehärtet. Die Bestimmung der Biegefestigkeit erfolgt bei einer Vorschubgeschwindigkeit von 0,75 mm/min an einer Zwick-Universalprüfmaschine (Zwick GmbH & Co. KG, Ulm).

Elastizitätsmodul (E-Modul): Der Elastizitätsmodul wird aus der Steigung im elastischen Bereich der Kraft-Weg-Kurven der Biegefestigkeitsmessungen ermittelt.

Schrumpfung: Die Schrumpfung wurde nach der Bonded-Disk-Methode von Watts et al. bestimmt (Watts DC, Cash AJ. Determination of polymerization kinetics in visible-light cured materials: Methods development. Dent Mater 1991; 7:281-287). Abweichend wurde mit einer Celalux 2-Lampe (VOCO GmbH) für 40 Sekunden pro Messung belichtet.

Viskosität: Die Bestimmung der Viskosität erfolgt mittels eines Rheometers (Physica MCR 301) der Firma Anton Paar (Graz, Österreich). Die Messung erfolgt bei 23 °C im Rotationsversuch mit Platte/Platte-Anordnung (Durchmesser 25 mm, Spaltabstand 1 mm) in einem Scherratenbereich von $10^{-2}$ bis 10 $s^{-1}$. Es werden pro Messung 16 Messwerte in einem Intervall von 30 Sekunden je Scherrate aufgenommen. In den Tabellen sind jeweils die Viskositäten für die Scherraten 0,01 und 10 $s^{-1}$ angegeben. Eine niedrige Scherrate entspricht annähernd der Ruheviskosität des Materials, während hohe Scherraten beim Applizieren des Materials durch eine enge Kanüle oder einen statischen Mischer beobachtet werden.

Ausdrückbarkeit: Die fließfähigen Komposite (Beispiele 5) wurden in 1 ml-Spritzen (schwarz, 3-teilig, Luerlock, mit Silikon-O-Ring) der Firma Transcodent GmbH & Co. KG abgefüllt und durch Kanülen (Typ 41, VOCO GmbH) ausgedrückt. Die Stumpfaufbau- und Befestigungskomposite (Beispiele 6 und 7) wurden in 5 ml-Doppelkammerspritzen (SDL X05-01-52, PED X05-01-SI) der Firma Sulzer Mixpac AG (Haag, Schweiz) abgefüllt und durch einen statischen Mischer (ML 2.5-08-D und IOT 212-20) ausgedrückt. Die Ausdrückbarkeit wurde in fünf Stufen bewertet: sehr gut, gut, akzeptabel, schwer, sehr schwer.

Ausdrückkraft: Die fließfähigen Komposite (Beispiele 5) wurden in 1 ml-Spritzen (schwarz, 3-teilig, Luerlock, mit Silikon-O-Ring) der Firma Transcodent GmbH & Co. KG abgefüllt und an einer Universalprüfmaschine (Zwick GmbH & Co. KG, Ulm) mit einer Vorschubgeschwindigkeit von 10 mm/min und einen Weg von 20 mm durch Kanülen (Typ 41, VOCO GmbH) ausgedrückt. Die Stumpfaufbau- und Befestigungskomposite (Beispiele 6 und 7) wurden in 5 ml-Doppelkammerspritzen (SDL X05-01-52, PED X05-01-SI) der Firma Sulzer Mixpac AG (Haag, Schweiz) abgefüllt und an einer Universalprüfmaschine (Zwick GmbH & Co. KG, Ulm) mit einer Vorschubgeschwindigkeit von 23 mm/min und einen Weg von 30 mm durch einen statischen Mischer (ML 2.5-08-D und IOT 212-20) ausgedrückt. Als Ausdrückkraft wurde jeweils die Maximalkraft ausgewertet.

Spreitbarkeit: Die Bestimmung der Spreitbarkeit erfolgt im Klimaraum bei einer Temperatur von 23 °C und einer relativen Luftfeuchtigkeit von 50% mittels einer Glasplattenanordnung. Die Dimensionen der Platten betragen 60 mm x

60 mm x 3 mm bei einem Plattengewicht von 25 g. Bei der Bestimmung werden auf die erste Platte 0,5 ml der jeweiligen Kompositpaste mittig appliziert. Anschließend wird die zweite Platte parallel zur ersten vorsichtig aufgelegt und mittig mit einem 100 g Gewicht. Nach 10 Minuten Belastung wird das Gewicht abgenommen und der größte und kleinste Durchmesser der komprimierten Scheibe aus dem Kompositpaste werden gemessen. Der Mittelwert der beiden Durchmesser wird als Spreitbarkeit angegeben.

## Patentansprüche

1. Radikalisch härtbare dentale Zusammensetzungen enthaltend

a.) mit radikalisch polymerisierbaren Gruppen substituierte kettenförmige und/oder zyklische und/oder in Käfigform vorliegende Polysiloxane mit mindestens 3 Siliziumatomen und/oder deren Mischformen,
b.) mit radikalisch polymerisierbaren Gruppen substituierte Disiloxane der folgenden Struktur

$$R^1_a\, R^2{}_{(3\text{-}a)}Si\text{-}O\text{-}SiR^2{}_{(3\text{-}b)}R^1_b$$

mit

$R^2$: Alkyl, Alkenyl, Aryl, Alkylaryl, Arylalkyl, wobei unterschiedliche $R^2$ gleich oder verschieden sein können,
$R^1$: YZ,
Z: radikalisch polymerisierbare Gruppe, ausgewählt aus den Strukturelementen $-O-(C=O)-CH=CH_2$, $-O-(C=O)-C(CH_3)=CH_2$, $-(C=O)-CH=CH_2$, $-(C=O)-C(CH_3)=CH_2$, $-CH=CH_2$, $-C(CH_3)=CH_2$, $-NH-(C=O)-CH=CH_2$ und $-NH-(C=O)-C(CH_3)=CH_2$,
wobei unterschiedliche Z gleich oder verschieden sein können,
a: 1 oder 2,
b: 1 oder 2,
Y: ein Verbindungselement, das das Siliziumatom mit der radikalisch polymerisierbaren Gruppe verknüpft und aus einer Alkylengruppe besteht,
wobei die Alkylenguppe eine unsubstituierte, durchgängige, geradkettige oder verzweigte Kohlenwasserstoffkette ist oder
wobei die Alkylengruppe eine unsubstituierte, durch eine Urethangruppe, Harnstoffgruppe, Estergruppe, Thiourethangruppe oder Amidgruppe unterbrochene Kohlenwasserstoffgruppe ist oder
wobei die Alkylengruppe eine durch eine Hydroxylgruppe substituierte Kohlenwasserstoffgruppe ist oder diese Hydroxylgruppe verestert oder verethert ist oder
wobei die Alkylengruppe eine durch ein Sauerstoffatom und/oder Stickstoffatom und/oder Schwefelatom und/oder Estergruppen und/oder Thioestergruppen unterbrochene und durch eine Hydroxylgruppe substituierte Kohlenwasserstoffgruppe ist oder diese Hydroxylgruppe verestert oder verethert ist und
wobei unterschiedliche Y gleich oder verschieden sein können und
wobei Y 20 oder weniger Kohlenstoffatome enthält und
wobei YZ so gewählt wird, dass Z immer möglichst viele Atome aufweist,

c.) optional ein, zwei, drei oder mehrere radikalisch härtbare Monomere ohne Siliziumatom,
d.) 85 Gewichtsprozent oder weniger Füllstoffe, umfassend:

d.)1. 1 - 20 Gew.-% organisch oberflächenmodifizierte anorganische Nanopartikel mit einer mittleren Teilchengröße kleiner 200 nm,
d.)2. 30 - 84 Gew.-% anorganische Mikropartikel mit einer mittleren Teilchengröße im Bereich von 0,4 µm und 10 µm sowie
d.)3. 0 - 30 Gew.-% weitere Füllstoffe, die nicht d.) 1. und d.) 2. entsprechen, wobei die Gewichtsangaben auf die Gesamtmasse der radikalisch härtbaren dentalen Zusammensetzung bezogen sind,

e.) Initiatoren und/oder Katalysatoren für die radikalische Polymerisation und
f.) weitere übliche Additive,
wobei die Zusammensetzung die Komponente

a.) zu 10 - 35 Gew.-%,
b.) zu 2 - 25 Gew.-%,

c.) zu 0 - 20 Gew.-%,
d.) zu 50 - 85 Gew.-%,
e.) zu 0,001 - 5 Gew.-%, und
f.) zu 0,001 - 20 Gew.-%
enthält und wobei die jeweiligen Gewichtsprozente auf die Gesamtmasse der Zusammensetzung bezogen sind.

**2.** Dentale radikalisch härtbare Zusammensetzung nach Anspruch 1 wobei die Polysiloxane a.) durch Hydrolyse oder Teilhydrolyse und anschließende Kondensation oder Cokondensation von ein, zwei, drei oder mehr Verbindungen $R^1_aR^2_bSiX_c$ mit

X: Halogen oder Alkoxy
$R^2$: Alkyl, Alkenyl, Aryl, Alkylaryl, Arylalkyl, wobei unterschiedliche $R^2$ gleich oder verschieden sein können,
$R^1$: YZ,
Z: radikalisch polymerisierbare Gruppe, ausgewählt aus den Strukturelementen $-O-(C=O)-CH=CH_2$, $-O-(C=O)-C(CH_3)=CH_2$, $-(C=O)-CH=CH_2$, $-(C=O)-C(CH_3)=CH_2$, $-CH=CH_2$, $-C(CH_3)=CH_2$, $-NH-(C=O)-CH=CH_2$ und $-NH-(C=O)-C(CH_3)=CH_2$,
wobei unterschiedliche Z gleich oder verschieden sein können,
a: 1 oder 2,
b: 0 oder 1,
c: 2 oder 3,
a + b + c = 4,
Y: ein Verbindungselement, das das Siliziumatom mit der radikalisch polymerisierbaren Gruppe verknüpft und aus einer Alkylengruppe besteht,
wobei die Alkylenguppe eine unsubstituierte, durchgängige, geradkettige oder verzweigte Kohlenwasserstoffkette ist oder
wobei die Alkylengruppe eine unsubstituierte, durch eine Urethangruppe, Harnstoffgruppe, Estergruppe, Thiourethangruppe oder Amidgruppe unterbrochene Kohlenwasserstoffgruppe ist oder
wobei die Alkylengruppe eine durch eine Hydroxylgruppe substituierte Kohlenwasserstoffgruppe ist oder diese Hydroxylgruppe verestert oder verethert ist oder
wobei die Alkylengruppe eine durch ein Sauerstoffatom und/oder Stickstoffatom und/oder Schwefelatom und/oder Estergruppen und/oder Thioestergruppen unterbrochene und durch eine Hydroxylgruppe substituierte Kohlenwasserstoffgruppe ist oder diese Hydroxylgruppe verestert oder verethert ist und
wobei unterschiedliche Y gleich oder verschieden sein können und
wobei Y 20 oder weniger Kohlenstoffatome enthält und
wobei YZ so gewählt wird, dass Z immer möglichst viele Atome aufweist.

erhalten werden.

**3.** Radikalisch härtbare dentale Zusammensetzung nach Anspruch 1 oder 2, wobei die Zusammensetzung die Komponente

a.) zu 15 - 25 Gew.-%,
b.) zu 5 - 15 Gew.-%,
c.) zu 0 - 10 Gew.-%,
d.) zu 60 - 78 Gew.-%,
e.) zu 0,1 - 2 Gew.-% und
f.) zu 0,001 - 10 Gew.-%

enthält und wobei die jeweiligen Gewichtsprozente auf die Gesamtmasse der Zusammensetzung bezogen sind.

**4.** Radikalisch härtbare dentale Zusammensetzung nach einem der Ansprüche 1, 2 oder 3, wobei die Zusammensetzung als Bestandteil b.) das 1,3-Bis (3-methacryloxypropyl)tetramethyldisiloxan) enthält.

**5.** Radikalisch härtbare dentale Zusammensetzung nach einem der Ansprüche 1, 2, 3 oder 4, wobei die Zusammensetzung keinen Bestandteil c.) enthält.

**6.** Radikalisch härtbare dentale Zusammensetzung nach einem der vorangehenden Ansprüche , wobei Komponente

d.) 1. zumindest teilweise nicht agglomeriert und/oder nicht aggregiert ist.

**7.** Radikalisch härtbare dentale Zusammensetzung nach einem der vorangehenden Ansprüche, wobei Komponente d.) 2. zwei oder mehr Mikrofraktionen enthält, wobei eine oder mehrere erste Mikrofraktionen jeweils eine mittlere Partikelgröße im Bereich von 1 bis 10 μm, vorzugsweise 1 bis 5 μm und wobei eine oder mehrere zweite Mikropartikelfraktionen jeweils eine mittlere Partikelgröße im Bereich von größer 0,4 μm bis kleiner 1 μm vorzugsweise 0,5 μm bis 0,8 μm besitzen.

**8.** Radikalisch härtbare dentale Zusammensetzung nach Anspruch 7, wobei das Verhältnis der Gesamtmasse der ersten Mikropartikelfraktion(en) zur Gesamtmasse der zweiten Mikropartikelfraktion(en) im Bereich von 1:1 bis 12:1 vorzugsweise im Bereich von 1,5:1 bis 8:1 liegt.

**9.** Radikalisch härtbare dentale Zusammensetzung nach einem der Ansprüche 7 oder 8, wobei das Verhältnis der mittleren Korngröße der oder einer ersten Mikropartikelfraktion zur mittleren Korngröße der oder einer zweiten Mikropartikelfraktion der Komponente d.) 2. im Bereich von 1,5:1 bis 10:1 vorzugsweise im Bereich von 2:1 bis 5:1 liegt.

**10.** Radikalisch härtbare dentale Zusammensetzung nach einem der vorangehenden Ansprüche, wobei zumindest ein Teil der Mikropartikel der Komponente d.) 2. organisch oberflächenmodifizierte Partikel vorzugsweise silanisierte Partikel sind und/oder zumindest ein Teil der Mikropartikel der Komponente d.) 2. Dentalglaspartikel sind, wobei zumindest ein Teil der Mikropartikel der Komponente d.) 2. organisch oberflächenmodifizierte Dentalglaspartikel vorzugsweise silanisierte Dentalglaspartikel sind.

**11.** Radikalisch härtbare dentale Zusammensetzung nach einem der vorangehenden Ansprüche, **dadurch gekennzeichnet, dass** die Komponente d.) röntgenopake Anteile enthält.

**12.** Radikalisch härtbare dentale Zusammensetzung nach Anspruch 11, wobei die röntgenopaken Anteile nanoskaliges $YbF_3$ und/oder $BaSO_4$ sind.

**13.** Radikalisch härtbare dentale Zusammensetzung nach einem der vorangehenden Ansprüche, wobei die radikalisch polymerisierbaren Gruppen Acrylat- oder Methacrylatgruppen sind.

**14.** Gehärtete dentale Zusammensetzung, erhältlich aus einer radikalisch härtbaren Zusammensetzung nach einem der vorangehenden Ansprüche 1 bis 13, **dadurch gekennzeichnet, dass** die dentale Zusammensetzung eine Schrumpfung von weniger als 3,5 Vol.-% vorzugsweise weniger als 3 Vol.-% aufweist.

**15.** Radikalisch härtbare dentale Zusammensetzung nach einem der Ansprüche 1 bis 13 zur Anwendung in einem therapeutischen Verfahren als fließfähiges dentales Füllungskompositmaterial (Flowmaterial) oder als dentaler Befestigungszement oder als dentales Stumpfaufbaumaterial.

**16.** Verfahren zur Herstellung einer radikalisch härtbaren Zusammensetzung nach einem der Ansprüche 1 bis 13 durch Mischen der Komponenten a.) bis f.).

**17.** Verfahren zur Herstellung einer gehärteten dentalen Zusammensetzung nach Anspruch 14 mit den folgenden Schritten:

- Bereitstellen einer radikalisch härtbaren dentalen Zusammensetzung nach einem der Ansprüche 1 bis 13,
- Polymerisation der radikalisch härtbaren dentalen Zusammensetzung.

**18.** Kit umfassend

- eine oder mehr als eine dentale Spritze und/oder eine oder mehr als eine dentale Compule und/oder eine oder mehr als eine Doppelkammerkartusche sowie

(i) ein, zwei oder mehr als zwei radikalisch härtbare dentale Zusammensetzungen nach einem der Ansprüche 1 bis 13 und/oder
(ii) eine, zwei oder mehr als zwei Basispasten und eine, zwei oder mehr als zwei Katalysatorpasten, wobei ein härtbares Dentalmaterial nach einem der Ansprüche 1 bis 13 durch Mischen einer Basispaste und der

dazugehörigen Katalysatorpaste erhältlich ist.

**19.** Extraorale Verwendung einer gehärteten dentalen Zusammensetzung nach Anspruch 14 zur Herstellung dentaler Bauteile oder Vorstufen dentaler Bauteile., vorzugsweise zur Herstellung dentaler Bauteile oder Vorstufen dentaler Bauteile mittels computergestützter Formgebung (computer-aided design) und/oder computergestützter Fertigung (computer-aided manufacturing, CAM).

## Claims

**1.** A free-radically curable dental composition comprising

a.) chain-like and/or cyclic and/or cage-type polysiloxanes substituted by free-radically polymerizable groups and having at least 3 silicon atoms and/or mixed forms thereof,
b.) disiloxanes substituted by free-radically polymerizable groups and having the following structure:

$$R^1{}_aR^2{}_{(3-a)}Si\text{-}O\text{-}SiR^2{}_{(3-b)}R^1b$$

with

$R^2$: alkyl, alkenyl, aryl, alkylaryl, arylalkyl, wherein different $R^2$ may be the same or different,
$R^1$: YZ,
Z: free-radically polymerizable group selected from the structural elements -O-(C=O)-CH=$CH_2$, -O-(C=O)-C($CH_3$)=$CH_2$, -(C=O)-CH=$CH_2$, -(C=O)-C($CH_3$)=$CH_2$, -CH=$CH_2$, -C($CH_3$)=$CH_2$, -NH-(C=O)-CH=$CH_2$ and -NH-(C=O)-C($CH_3$)=$CH_2$,
wherein different Z may be the same or different,
a: 1 or 2,
b: 1 or 2,
Y: a connecting element which links the silicon atom to the free-radically polymerizable group and consists of an alkylene group,
wherein the alkylene group is an unsubstituted, linear, straight-chain or branched hydrocarbyl chain or wherein the alkylene group is an unsubstituted hydrocarbyl group interrupted by a urethane group, urea group, ester group, thiourethane group or amide group or
wherein the alkylene group is a hydrocarbyl group substituted by a hydroxyl group or this hydroxyl group has been esterified or etherified or
wherein the alkylene group is a hydrocarbyl group interrupted by an oxygen atom and/or nitrogen atom and/or sulfur atom and/or ester groups and/or thioester groups and substituted by a hydroxyl group or this hydroxyl group has been esterified or etherified and
wherein different Y may be the same or different and
wherein Y contains 20 or fewer carbon atoms and wherein YZ is chosen such that Z always has a maximum number of atoms,

c.) optionally one, two, three or more free-radically curable monomers having no silicon atom,
d.) 85 percent by weight or less of fillers comprising:

d.)1. 1% - 20% by weight of organically surface-modified inorganic nanoparticles having an average particle size of less than 200 nm,
d.)2. 30% - 84% by weight of inorganic microparticles having an average particle size in the range from 0.4 μm to 10 μm and
d.)3. 0% - 30% by weight of further fillers which do not correspond to d.)1. and d.)2. wherein the respective percentages by weight are based on the total weight of the free-radically curable dental composition,

e.) initiators and/or catalysts for the free-radical polymerization and
f.) further customary additives,

wherein the composition contains:

a.) in an amount of 10%-35% by weight,

b.) in an amount of 2%-25% by weight,
c.) in an amount of 0%-20% by weight,
d.) in an amount of 50%-85% by weight,
e.) in an amount of 0.001%-5% by weight, and
f.) in an amount of 0.001%-20% by weight,

and wherein the respective percentages by weight are based on the total weight of the composition.

2. The dental free-radically curable composition as claimed in claim 1, wherein the polysiloxanes a.) are obtained by hydrolysis or partial hydrolysis and subsequent condensation or co-condensation of one, two, three or more compounds $R^1_aR^2_bSiX_c$ with

X: halogen or alkoxy,
$R^2$: alkyl, alkenyl, aryl, alkylaryl, arylalkyl, wherein different $R^2$ may be the same or different,
$R^1$: YZ,
Z: free-radically polymerizable group selected from the structural elements $-O-(C=O)-CH=CH_2$, $-O-(C=O)-C(CH_3)=CH_2$, $-(C=O)-CH=CH_2$, $-(C=O)-C(CH_3)=CH_2$, $-CH=CH_2$, $-C(CH_3)=CH_2$, $-NH-(C=O)-CH=CH_2$ and $-NH-(C=O)-C(CH_3)=CH_2$,
wherein different Z may be the same or different,
a: 1 or 2,
b: 0 or 1,
c: 2 or 3,
a + b + c = 4,
Y: a connecting element which links the silicon atom to the free-radically polymerizable group and consists of an alkylene group,
wherein the alkylene group is an unsubstituted, linear, straight-chain or branched hydrocarbyl chain or
wherein the alkylene group is an unsubstituted hydrocarbyl group interrupted by a urethane group, urea group, ester group, thiourethane group or amide group or
wherein the alkylene group is a hydrocarbyl group substituted by a hydroxyl group or this hydroxyl group has been esterified or etherified or
wherein the alkylene group is a hydrocarbyl group interrupted by an oxygen atom and/or nitrogen atom and/or sulfur atom and/or ester groups and/or thioester groups and substituted by a hydroxyl group or this hydroxyl group has been esterified or etherified and
wherein different Y may be the same or different and
wherein Y contains 20 or fewer carbon atoms and wherein YZ is chosen such that Z always has a maximum number of atoms.

3. The free-radically curable dental composition as claimed in claim 1 or 2, wherein the composition contains:

a.) in an amount of 15%-25% by weight,
b.) in an amount of 5%-15% by weight,
c.) in an amount of 0%-10% by weight,
d.) in an amount of 60%-78% by weight,
e.) in an amount of 0.1%-2% by weight and
f.) in an amount of 0.001%-10% by weight,

and wherein the respective percentages by weight are based on the total weight of the composition.

4. The free-radically curable dental composition as claimed in any of claims 1, 2 and 3, wherein the composition contains, as constituent b.), 1,3-bis(3-methacryloyloxypropyl)tetramethyldisiloxane.

5. The free-radically curable dental composition as claimed in any of claims 1, 2, 3 and 4, wherein the composition does not contain any constituent c.).

6. The free-radically curable dental composition as claimed in any of the preceding claims, wherein component d.)1. is at least partly in nonagglomerated and/or nonaggregated form.

7. The free-radically curable dental composition as claimed in any of the preceding claims, wherein component d.)2.

contains two or more micro fractions, wherein a first micro fraction or each of a plurality of first micro fractions has an average particle size in the range from 1 to 10 μm, preferably 1 to 5 μm, and wherein a second microparticle fraction or each of a plurality of second microparticle fractions has an average particle size in the range from greater than 0.4 μm to less than 1 μm, preferably 0.5 μm to 0.8 μm.

**8.** The free-radically curable dental composition as claimed in claim 7, wherein the ratio of the total mass of the first microparticle fraction(s) to the total mass of the second microparticle fraction (s) is in the range from 1:1 to 12:1, preferably in the range from 1.5:1 to 8:1.

**9.** The free-radically curable dental composition as claimed in either of claims 7 and 8, wherein the ratio of the average particle size of the first or a first microparticle fraction to the average particle size of the second or a second microparticle fraction of component d.)2. is in the range from 1.5:1 to 10:1, preferably in the range from 2:1 to 5:1.

**10.** The free-radically curable dental composition as claimed in any of the preceding claims, wherein at least some of the microparticles of component d.)2. are organically surface-modified particles, preferably silanized particles, and/or at least some of the microparticles of component d.)2. are dental glass particles, wherein at least some of the microparticles of component d.)2. are organically surface-modified dental glass particles, preferably silanized dental glass particles.

**11.** The free-radically curable dental composition as claimed in any of the preceding claims, wherein component d.) contains x-ray-opaque fractions.

**12.** The free-radically curable dental composition as claimed in claim 11, wherein the x-ray-opaque fractions are nanoscale $YbF_3$ and/or $BaSO_4$.

**13.** The free-radically curable dental composition as claimed in any of the preceding claims, wherein the free-radically polymerizable groups are acrylate or methacrylate groups.

**14.** A cured dental composition obtainable from the free-radically curable composition according to any of the preceding claims 1 to 13, **characterized in that** the dental composition has a shrinkage of less than 3.5% by volume, preferably less than 3% by volume.

**15.** The free-radically curable dental composition as claimed in any of claims 1 to 13 for use in a therapeutic method as a flowable dental filling composite material (flow material) or as a dental luting cement or as a dental core buildup material.

**16.** A method for producing a free-radically curable composition as claimed in any of claims 1 to 13 by mixing components a.) to f.).

**17.** A method for producing a cured dental composition as claimed in claim 14, comprising the following steps:

- providing a free-radically curable dental composition as claimed in any of claims 1 to 13,
- polymerizing the free-radically curable dental composition.

**18.** A kit comprising

- one or more than one dental syringe and/or one or more than one dental compule and/or one or more than one double-chamber cartridge, and also

(i) one, two or more than two free-radically curable dental compositions as claimed in any of claims 1 to 13 and/or
(ii) one, two or more than two base pastes and one, two or more than two catalyst pastes, wherein a curable dental material as claimed in any of claims 1 to 13 is obtainable by mixing a base paste and the corresponding catalyst paste.

**19.** The extraoral use of a cured dental composition as claimed in claim 14 for production of dental components or precursors of dental components, preferably for production of dental components or precursors of dental components by means of computer-aided design and/or computer-aided manufacturing (CAM).

**Revendications**

1. Compositions dentaires durcissables par voie radicalaire, contenant :

a.) des polysiloxanes linéaires et/ou cycliques et/ou sous la forme de cage substitués avec des groupes polymérisables par voie radicalaire, contenant au moins 3 atomes de silicium et/ou leurs formes mixtes,
b.) des disiloxanes substitués avec des groupes polymérisables par voie radicalaire de la structure suivante :

$$R^1{}_aR^2{}_{(3-a)}Si\text{-}O\text{-}SiR^2{}_{(3-b)}R^1{}_b$$

avec

$R^2$ : alkyle, alcényle, aryle, alkylaryle, arylalkyle, différents $R^2$ pouvant être identiques ou différents,
$R^1$ : YZ,
Z : groupe polymérisable par voie radicalaire, choisi parmi les éléments structuraux $-O-(C=O)-CH=CH_2$, $-O-(C=O)-C(CH_3)=CH_2$, $-(C=O)-CH=CH_2$, $-(C=O)-C(CH_3)=CH_2$, - $CH=CH_2$, $-C(CH_3)=CH_2$, $-NH-(C=O)-CH=CH_2$ et $-NH-(C=O)-C(CH_3)=CH_2$, différents Z pouvant être identiques ou différents,
a : 1 ou 2,
b : 1 ou 2,
Y : un élément de liaison, qui relie l'atome de silicium avec le groupe polymérisable par voie radicalaire et est constitué par un groupe alkylène,
le groupe alkylène étant une chaîne hydrocarbonée non substituée, continue, linéaire ou ramifiée, ou
le groupe alkylène étant un groupe hydrocarboné non substitué, interrompu par un groupe uréthane, un groupe urée, un groupe ester, un groupe thiouréthane ou un groupe amide, ou
le groupe alkylène étant un groupe hydrocarboné substitué par un groupe hydroxyle, ou ce groupe hydroxyle étant estérifié ou éthérifié, ou
le groupe alkylène étant un groupe hydrocarboné interrompu par un atome d'oxygène et/ou un atome d'azote et/ou un atome de soufre et/ou des groupes esters et/ou des groupes thioester et substitué par un groupe hydroxyle, ou ce groupe hydroxyle étant estérifié ou éthérifié, et
différents Y pouvant être identiques ou différents, et
Y contenant 20 atomes de carbone ou moins, et
YZ étant choisi de telle sorte que Z comprenne toujours autant d'atomes que possible,

c.) éventuellement un, deux, trois ou davantage de monomères durcissables par voie radicalaire sans atome de silicium,
d.) 85 pour cent en poids ou moins de charges, comprenant :

d.)1. 1 à 20 % en poids de nanoparticules inorganiques modifiées organiquement en surface ayant une taille de particule moyenne inférieure à 200 nm,
d.)2. 30 à 84 % en poids de microparticules inorganiques ayant une taille de particule moyenne dans la plage allant de 0,4 μm à 10 μm, et
d.)3. 0 à 30 % en poids d'autres charges, qui ne correspondent ni à d.)1., ni à d.)2., les données de poids se rapportant à la masse totale de la composition dentaire durcissable par voie radicalaire,

e.) des initiateurs et/ou des catalyseurs pour la polymérisation radicalaire, et
f.) d'autres additifs usuels,

la composition contenant le composant

a.) à hauteur de 10 à 35 % en poids,
b.) à hauteur de 2 à 25 % en poids,
c.) à hauteur de 0 à 20 % en poids,
d.) à hauteur de 50 à 85 % en poids,
e.) à hauteur de 0,001 à 5 % en poids, et
f.) à hauteur de 0,001 à 20 % en poids,

les pourcentages en poids respectifs se rapportant à la masse totale de la composition.

**2.** Composition dentaire durcissable par voie radicalaire selon la revendication 1, dans laquelle les polysiloxanes a.) sont obtenus par hydrolyse ou hydrolyse partielle, puis condensation ou co-condensation d'un, de deux, de trois ou de davantage de composés $R^1_aR^2_bSiX_c$, avec

X : halogène ou alcoxy,
$R^2$ : alkyle, alcényle, aryle, alkylaryle, arylalkyle, différents $R^2$ pouvant être identiques ou différents,
$R^1$ : YZ,
Z : groupe polymérisable par voie radicalaire, choisi parmi les éléments structuraux $-O-(C=O)-CH=CH_2$, $-O-(C=O)-C(CH_3)=CH_2$, $-(C=O)-CH=CH_2$, $-(C=O)-C(CH_3)=CH_2$, $-CH=CH_2$, $-C(CH_3)=CH_2$, $-NH-(C=O)-CH=CH_2$ et $-NH-(C=O)-C(CH_3)=CH_2$, différents Z pouvant être identiques ou différents,
a : 1 ou 2,
b : 0 ou 1,
c : 2 ou 3,

$$a+b+c = 4,$$

Y: un élément de liaison, qui relie l'atome de silicium avec le groupe polymérisable par voie radicalaire et est constitué par un groupe alkylène,
le groupe alkylène étant une chaîne hydrocarbonée non substituée, continue, linéaire ou ramifiée, ou
le groupe alkylène étant un groupe hydrocarboné non substitué, interrompu par un groupe uréthane, un groupe urée, un groupe ester, un groupe thiouréthane ou un groupe amide, ou
le groupe alkylène étant un groupe hydrocarboné substitué par un groupe hydroxyle, ou ce groupe hydroxyle étant estérifié ou éthérifié, ou
le groupe alkylène étant un groupe hydrocarboné interrompu par un atome d'oxygène et/ou un atome d'azote et/ou un atome de soufre et/ou des groupes esters et/ou des groupes thioester et substitué par un groupe hydroxyle, ou ce groupe hydroxyle étant estérifié ou éthérifié, et
différents Y pouvant être identiques ou différents, et
Y contenant 20 atomes de carbone ou moins, et
YZ étant choisi de telle sorte que Z comprenne toujours autant d'atomes que possible.

**3.** Composition dentaire durcissable par voie radicalaire selon la revendication 1 ou 2, dans laquelle la composition contient le composant

a.) à hauteur de 15 à 25 % en poids,
b.) à hauteur de 5 à 15 % en poids,
c.) à hauteur de 0 à 10 % en poids,
d.) à hauteur de 60 à 78 % en poids,
e.) à hauteur de 0,1 à 2 % en poids, et
f.) à hauteur de 0,001 à 10 % en poids,

les pourcentages en poids respectifs se rapportant à la masse totale de la composition.

**4.** Composition dentaire durcissable par voie radicalaire selon l'une quelconque des revendications 1, 2 ou 3, dans laquelle la composition contient en tant que constituant b.) le 1,3-bis(3-méthacryloxypropyl)tétraméthyldisiloxane.

**5.** Composition dentaire durcissable par voie radicalaire selon l'une quelconque des revendications 1, 2, 3 ou 4, dans laquelle la composition ne contient pas de constituant c.).

**6.** Composition dentaire durcissable par voie radicalaire selon l'une quelconque des revendications précédentes, dans laquelle le composant d.)1. est au moins partiellement non aggloméré et/ou non agrégé.

**7.** Composition dentaire durcissable par voie radicalaire selon l'une quelconque des revendications précédentes, dans laquelle le composant d.)2. contient deux microfractions ou plus, une ou plusieurs premières microfractions présentant chacune une taille de particule moyenne dans la plage allant de 1 à 10 µm, de préférence de 1 à 5 µm, et une ou plusieurs deuxièmes fractions de microparticules présentant chacune une taille de particule moyenne dans la plage allant de plus de 0,4 µm à moins de 1 µm, de préférence de 0,5 µm à 0,8 µm.

8. Composition dentaire durcissable par voie radicalaire selon la revendication 7, dans laquelle le rapport entre la masse totale de la ou des premières fractions de microparticules et la masse totale de la ou des deuxièmes fractions de microparticules se situe dans la plage allant de 1:1 à 12:1, de préférence dans la plage allant de 1,5:1 à 8:1.

9. Composition dentaire durcissable par voie radicalaire selon l'une quelconque des revendications 7 ou 8, dans laquelle le rapport entre la taille de particule moyenne de la ou d'une première fraction de microparticules et la taille de particule moyenne de la ou d'une deuxième fraction de microparticules du composant d.)2. se situe dans la plage allant de 1,5:1 à 10:1, de préférence dans la plage allant de 2:1 à 5:1.

10. Composition dentaire durcissable par voie radicalaire selon l'une quelconque des revendications précédentes, dans laquelle au moins une partie des microparticules du composant d.)2. sont des particules modifiées organiquement en surface, de préférence des particules silanisées, et/ou au moins une partie des microparticules du composant d.)2. sont des particules de verre dentaire, au moins une partie des microparticules du composant d.)2. étant des particules de verre dentaire modifiées organiquement en surface, de préférence des particules de verre dentaire silanisées.

11. Composition dentaire durcissable par voie radicalaire selon l'une quelconque des revendications précédentes, **caractérisée en ce que** le composant d.) contient des fractions opaques aux rayons X.

12. Composition dentaire durcissable par voie radicalaire selon la revendication 11, dans laquelle les fractions opaques aux rayons X sont du $YbF_3$ et/ou $BaSO_4$ nanométrique.

13. Composition dentaire durcissable par voie radicalaire selon l'une quelconque des revendications précédentes, dans laquelle les groupes polymérisables par voie radicalaire sont des groupes acrylate ou méthacrylate.

14. Composition dentaire durcie, pouvant être obtenue à partir d'une composition durcissable par voie radicalaire selon l'une quelconque des revendications 1 à 13 précédentes, **caractérisée en ce que** la composition dentaire présente un retrait inférieur à 3,5 % en volume, de préférence inférieur à 3 % en volume.

15. Composition dentaire durcissable par voie radicalaire selon l'une quelconque des revendications 1 à 13, destinée à une utilisation dans un procédé thérapeutique en tant que matériau composite de remplissage dentaire fluide (flow material) ou en tant que ciment de fixation dentaire ou en tant que matériau de reconstruction de moignon dentaire.

16. Procédé de fabrication d'une composition durcissable par voie radicalaire selon l'une quelconque des revendications 1 à 13 par mélange des composants a.) à f.).

17. Procédé de fabrication d'une composition dentaire durcie selon la revendication 14, comprenant les étapes suivantes :

   - la préparation d'une composition dentaire durcissable par voie radicalaire selon l'une quelconque des revendications 1 à 13,
   - la polymérisation de la composition dentaire durcissable par voie radicalaire.

18. Kit comprenant :

   - une ou plus d'une seringue dentaire et/ou une ou plus d'une compule dentaire et/ou une ou plus d'une cartouche à chambre double, ainsi que

     (i) une, deux ou plus de deux compositions dentaires durcissables par voie radicalaire selon l'une quelconque des revendications 1 à 13, et/ou
     (ii) une, deux ou plus de deux pâtes de base et une, deux ou plus de deux pâtes de catalyseur, un matériau dentaire durcissable selon l'une quelconque des revendications 1 à 13 pouvant être obtenu par mélange d'une pâte de base et de la pâte de catlayseur correspondante.

19. Utilisation extra-orale d'une composition dentaire durcie selon la revendication 14 pour la fabrication de composants dentaires ou de précurseurs de composants dentaires, de préférence pour la fabrication de composants dentaires ou de précurseurs de composants dentaires par conception assistée par ordinateur (computer-aided design) et/ou

fabrication assitée par ordinateur (computer-aided manufacturing, CAM).

Abbildung 1: E-Modul der Beispiele 1-4

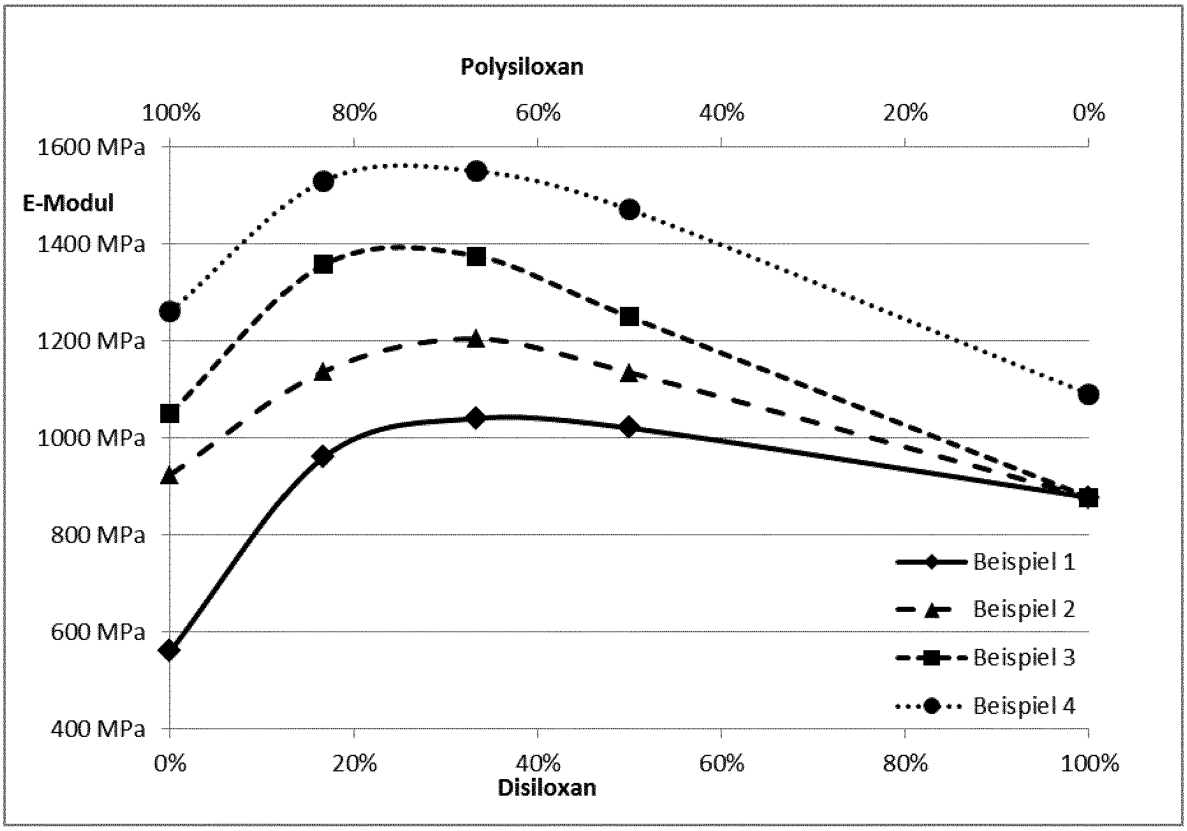


Abbildung 2: Viskositätskurven der Flows 5-A, B und F

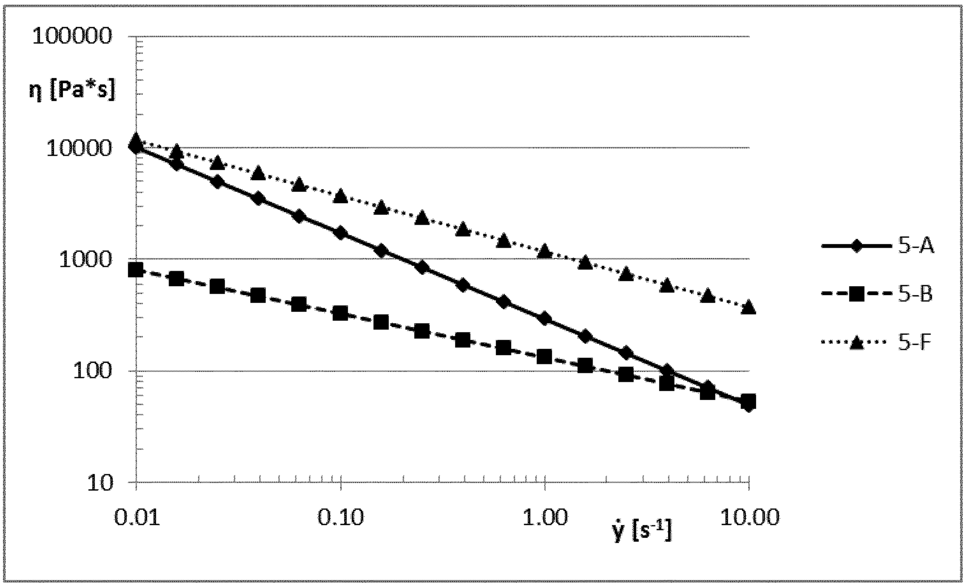

Abbildung 3: Viskositätskurven der Flows 5-A, B, D, E

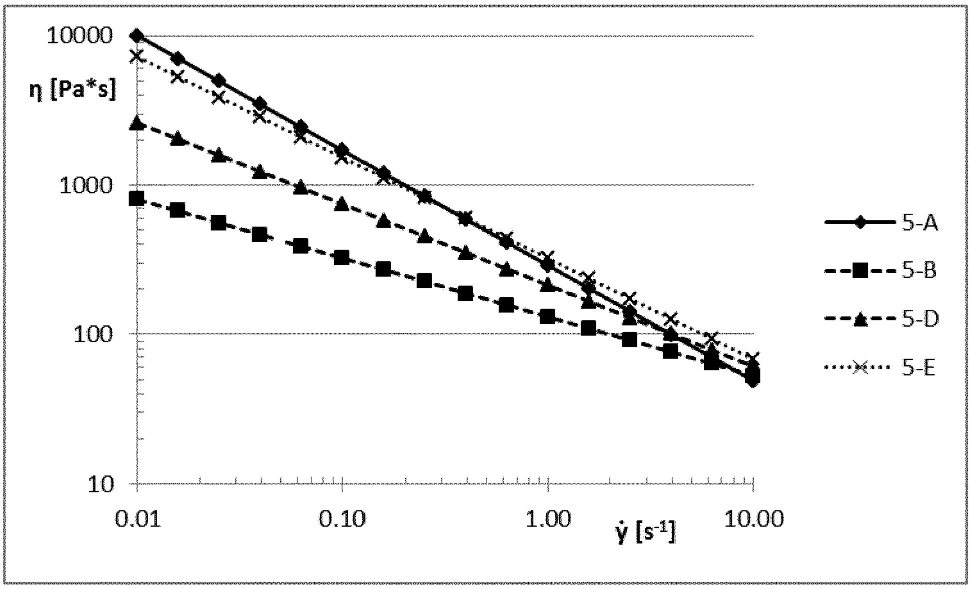

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 2758414 A1
- DE 4416857 C1
- DE 19860364 C2
- EP 1874847 B1
- EP 1685182 B1
- WO 2013041723 A1
- WO 2013053693 A1
- DE 102014210432
- DE 3941629 A1
- EP 11183333 A
- EP 11183328 A
- EP 11183345 A
- EP 11183338 A
- EP 11183342 A
- EP 11188086 A
- WO 2005011621 A1
- DE 102006019092 A1
- DE 3941629 C2
- DE 102006019092
- DE 60116142
- DE 3801511 C2
- DE 102006050153 A1
- EP 0184095 B1
- DE 4231579 C2
- EP 0366977 B1
- US 7081485 B2
- DE 3236026 A1
- US 20070027229 A1
- EP 0262629 B1
- EP 0073413 A
- US 7148382 B2
- US 5761169 A
- DE 19708294 A1
- EP 0057474 A
- EP 0047902 A
- EP 0007508 A
- DE 60029481 T2
- EP 0980682 B1
- EP 0948955 B1
- EP 1236459 B1
- EP 0173567 A2
- EP 1905415 A
- US 4772530 A
- US 4954414 A
- US 4874450 A
- US 5055372 A
- US 5057393 A
- EP 1720506 A
- DE 3941629
- EP 1839640 A
- DE 1495520
- WO 02092021 A
- WO 02092023 A
- EP 1872767 A
- EP 2070506 A
- EP 1881010 A
- DE 102007050763
- US 6288138 B
- DE 112006001049
- US 7214726 B
- EP 2070935 A
- EP 0059451 A
- EP 0783880 B1
- DE 10119831 A1
- EP 1563821 A1


### In der Beschreibung aufgeführte Nicht-Patentliteratur


- **J.M. ANTONUCI ; J.W. STANSBURY ; S. VENZ.** Synthesis and properties of a polyfluorinated prepolymer multifunctional urethan methacrylate. *Polymeric Materials Science and Engineering,* 1988, vol. 59, 388-396
- **J.S. KUO ; J.M. ANTONUCCI ; W. WU.** Evaluation of siloxane containing dental composites. *Journal of Dental Research Abstracts,* 1985
- **X. LIANG ; F. LIU ; J. HE.** Synthesis of none Bisphenol A structure dimethacrylate monomer and characterization for dental composite applications. *Dental Materials,* 2014, vol. 30, 917-925
- **DEBORAH HUCK-JONES ; RENATE HESSEMANN.** Chemische Identität einzelner Partikel. *Nachrichten aus der Chemie,* September 2014, vol. 62, 886-887
- **J.-P. FOUASSIER.** Photoinitiation, Photopolymerization and Photocuring. Hanser Publishers, 1995
- Radiation Curing in Polymer Science and Technology. Elsevier Applied Science, 1993, vol. II

- **WATTS DC ; CASH AJ.** Determination of polymerization kinetics in visible-light cured materials: Methods development. *Dent Mater,* 1991, vol. 7, 281-287